(19) Europäisches Patentamt
European Patent Office
Office européen des brevets

(11) EP 3 831 935 A1

(12) EUROPEAN PATENT APPLICATION
published in accordance with Art. 153(4) EPC

(43) Date of publication:
**09.06.2021 Bulletin 2021/23**

(21) Application number: **19844983.7**

(22) Date of filing: **30.07.2019**

(51) Int Cl.:
*C12N 5/0775 (2010.01)* *A61K 35/32 (2015.01)*
*A61K 35/545 (2015.01)* *A61P 19/02 (2006.01)*
*A61P 19/04 (2006.01)* *A61P 29/00 (2006.01)*
*A61P 37/06 (2006.01)*

(86) International application number:
**PCT/JP2019/029913**

(87) International publication number:
**WO 2020/027163 (06.02.2020 Gazette 2020/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **31.07.2018 JP 2018143793**

(71) Applicants:
- **JCR Pharmaceuticals Co., Ltd.**
**Ashiya-shi, Hyogo 659-0021 (JP)**
- **Teijin Limited**
**Osaka 530-0005 (JP)**

(72) Inventors:
- **IMAGAWA, Kiwamu**
**Kobe-shi, Hyogo 651-2241 (JP)**
- **MINAMI, Kotaro**
**Kobe-shi, Hyogo 651-2241 (JP)**
- **MAEDA, Kenichi**
**Kobe-shi, Hyogo 651-2241 (JP)**
- **HOSODA, Yuki**
**Kobe-shi, Hyogo 651-2241 (JP)**
- **WATANABE, Shunsuke**
**Kobe-shi, Hyogo 651-2241 (JP)**
- **SATO, Kazutoshi**
**Kobe-shi, Hyogo 651-2241 (JP)**
- **HIGASHIGUCHI, Yasuna**
**Kobe-shi, Hyogo 651-2241 (JP)**
- **KUSHIDA, Takashi**
**Osaka-shi, Osaka 530-0005 (JP)**
- **ISHIWARI, Ayumi**
**Osaka-shi, Osaka 530-0005 (JP)**

(74) Representative: **Boult Wade Tennant LLP**
**Salisbury Square House**
**8 Salisbury Square**
**London EC4Y 8AP (GB)**

# (54) METHOD FOR PRODUCING DENTAL PULP-DERIVED CELLS

(57) Pluripotent stem cell-enriched dental pulp-derived cells that can be administered to humans as a medicine, and a production method thereof are disclosed. The production method is a method for producing dental pulp-derived cells enriched with pluripotent stem cells including: a step (a) of digesting dental pulp with a protease to prepare a dental pulp suspension; a step (b) of culturing the suspension to proliferate pluripotent stem cells contained in the suspension; a step (c) of freezing the proliferated pluripotent stem cells in a state in which the pluripotent stem cells are suspended in a first cryopreservation liquid; a step (d) of thawing the frozen pluripotent stem cells; a step (e) of culturing the thawed pluripotent stem cells in a state in which the pluripotent stem cells are adhered to surfaces of particles to proliferate the pluripotent stem cells on the surfaces of the particles; and a step (f) of bringing the particles into contact with a protease to separate the pluripotent stem cells adhered to the surfaces of the particles from the particles.

EP 3 831 935 A1

## Description

### Technical field

The present invention relates to pluripotent stem cells which are obtained from dental pulp and have abilities to differentiate into chondrocytes and osteocytes, and a method for producing such pluripotent stem cells, for example.

### Background Art

It is known that stem cells (pluripotent stem cells) having an ability to differentiate into cells of a plurality of systems can be obtained from various tissues. Mesenchymal stem cells isolated from bone marrow are one of these stem cells and have an ability to differentiate into various cells such as osteocytes, cardiomyocytes, chondrocytes, and adipocytes (Patent Literature 1 to 4). Attempts have been made to apply mesenchymal stem cells to regenerative medicine in various tissues utilizing this differentiation ability. For example, it has been reported that attempts have been made to administer mesenchymal stem cells to patients with myocardial infarction to regenerate myocardium which has necrosed due to myocardial infarction, thereby improving cardiac functions of the patients (Non Patent Literature 1).

It is known that mesenchymal stem cells can be obtained not only from bone marrow as described above but also from various tissues such as adipose tissue (Patent Literature 5), placental tissue and umbilical cord tissue (Patent Literature 6).

Regarding the fact that pluripotent stem cells can be obtained also from dental tissue, it has been reported that pluripotent stem cells can be obtained from, for example, dental pulp (Patent Literature 7 to 11), dental follicle (Patent Literature 12), dental sac (Patent Literature 11 and 13), dental papilla (Patent Literature 14), and periodontal membrane (Patent Literature 15). The pluripotent stem cells derived from dental tissue usually have an ability to differentiate into adipocytes (Non Patent Literature 2 to 4).

Acquisition of pluripotent stem cells from dental pulp is roughly performed according to the following procedure (Patent Literature 16). Tissue obtained by crushing an extracted tooth is treated with type I collagenase and Dispase (registered trademark), and cell aggregations are removed by passing through a filter to obtain a cell suspension. Subsequently, the cells are proliferated in a cell culture plate using a DMEM medium containing 20% FBS. The cells which have been adhered to the inner surface of the cell culture plate and proliferated are treated with trypsin and peeled off, and the peeled off cells are collected. The cells thus collected are dental pulp-derived pluripotent stem cells. Dispase is a neutral protease derived from Bacillus polymyxa.

Dental pulp is a loose fibrous connective tissue that fills the pulp cavity of the tooth, and is divided into crown pulp and root pulp according to its location. In addition, it has been reported that pluripotent stem cells derived from tooth tissue usually have an ability to differentiate into adipocytes (Non Patent Literature 2 to 4), whereas stem cells derived from dental pulp do not differentiate into adipocytes (Patent Literature 8). That is, there is a possibility that there are at least two types of stem cells obtained from dental pulp distinguished by the presence or absence of the ability to differentiate into adipocytes. In addition, it has been reported that stem cells obtained from dental pulp of deciduous teeth have properties different from those of stem cells existing in dental pulp of permanent teeth as these have a high proliferation ability and highly express FGF2, TGF-$\beta$, collagen I, and collagen III compared to stem cells obtained from dental pulp of permanent teeth (Patent Literature 16). In addition, it has also been reported that pluripotent stem cells derived from dental pulp have properties different from those of mesenchymal stem cells derived from bone marrow in terms of inducing differentiation into osteoblasts (Patent Literature 11).

It is expected that pluripotent stem cells derived from dental pulp will be clinically applicable as these can be used as a therapeutic agent for neurological diseases (Patent Literature 17).

### Citation List

### Patent Literature

Patent Literature 1: United States Patent No. 5486359
Patent Literature 2: United States Patent No. 5827740
Patent Literature 3: United States Patent No. 6835377
Patent Literature 4: United States Patent No. 6387369
Patent Literature 5: Japanese Unexamined Patent Publication No. 2004-129549
Patent Literature 6: Japanese Unexamined Patent Publication No. 2004-210713
Patent Literature 7: Japanese Unexamined Patent Publication No. 2010-252778

Patent Literature 8: WO2002/007679
Patent Literature 9: Japanese Unexamined Patent Publication No. 2008-507962
Patent Literature 10: WO2009/072527
Patent Literature 11: Japanese Unexamined Patent Publication No. 2004-201612
Patent Literature 12: Japanese Unexamined Patent Publication No. 2009-527223
Patent Literature 13: Japanese Unexamined Patent Publication No. 2005-516616
Patent Literature 14: Japanese Unexamined Patent Publication No. 2006-238875
Patent Literature 15: Japanese Unexamined Patent Publication No. 2008-295420
Patent Literature 16: Japanese Unexamined Patent Publication No. 2010-268715
Patent Literature 17: Japanese Unexamined Patent Publication No. 2011-219432

**Non Patent Literature**

Non Patent Literature 1: Katritsis DG. et. al., Catheter Cardiovasc Interv. 65(3): 321-9 (2005)
Non Patent Literature 2: Gronthos S. et. al., J Dent Res. 81(8): 531-5 (2002)
Non Patent Literature 3: Tirino V. et. al., Stem Cell Rev. 7(3): 608-15 (2011)
Non Patent Literature 4: Vishwanath VR et. al., J Conserv Dent. 16(5): 423-8 (2013)

**Summary of Invention**

**Technical Problem**

An object of the present invention is to provide novel pluripotent stem cells preparation derived from dental pulp which are useful for treating cerebral infarction or the like and can be administered to humans. Another object of the present invention is to provide a method for efficiently producing pluripotent stem cells derived from dental pulp so that the pluripotent stem cells can be stably supplied to the market in a sufficient amount. In addition, still another object of the present invention is to produce the cells in a form in which stability suitable for distribution or storage can be maintained.

**Solution to Problem**

In the research for the above-described objects, the present inventors have conducted extensive studies. As a result, they have found a method for efficiently producing dental pulp-derived cells enriched with pluripotent stem cells from dental pulp tissue obtained from human extracted teeth, and have completed the present invention. That is, the present invention includes the following.

1. A method for producing dental pulp-derived cells enriched with pluripotent stem cells, the method comprising: a step (a) of digesting dental pulp with a protease to prepare a dental pulp suspension; a step (b) of culturing the suspension to proliferate pluripotent stem cells contained in the suspension; a step (c) of freezing the proliferated pluripotent stem cells in a state in which the pluripotent stem cells are suspended in a first cryopreservation liquid; a step (d) of thawing the frozen pluripotent stem cells; a step (e) of culturing the thawed pluripotent stem cells in a state in which the pluripotent stem cells are adhered to surfaces of carrier particles to proliferate the pluripotent stem cells on the surfaces of the carrier particles; a step (f) of bringing the carrier particles into contact with a protease to separate the pluripotent stem cells adhered to the surfaces of the carrier particles from the carrier particles; and a step (g) of preparing a suspension of the separated pluripotent stem cells.
2. The production method according to the above-described 1, wherein quality tests of the pluripotent stem cells are performed in the step (c) or (d).
3. The production method according to the above-described 2, wherein the quality tests are performed on cells fractionated from cells before suspension in the first cryopreservation liquid, subcultured cells which have been fractionated from the cells before suspension in the first cryopreservation liquid, pre-freezing cells which have been suspended in the first cryopreservation liquid and fractionated, cells immediately after thawing, which have been fractionated and frozen, and/or cells obtained by fractionation, freezing, thawing, and culturing.
4. The production method according to the above-described 3, wherein the quality tests are performed for one or more of the following Quality Tests a to j.

Quality Test a which verifies that a proportion of the number of cells showing a substantially spindle-shaped form in all cells when observed under an optical microscope is greater than or equal to 99%, greater than or equal to 99.5%, greater than or equal to 99.9%, or greater than or equal to 99.95%,

Quality Test b which verifies that a cell viability is greater than or equal to 50%, greater than or equal to 60%, greater than or equal to 70%, greater than or equal to 80%, greater than or equal to 90%, or greater than or equal to 95%,

Quality Test c which verifies that cells are positive for CD73, CD90, CD105, and CD166 and negative for CD34 and CD45 in expression patterns of the cell surface antigen markers, or verifies that cells are positive for at least one of CD73 and CD90 and negative for CD34 in expression patterns of the cell surface antigen markers,

Quality Test d which verifies that cells are negative for CD40, CD80, CD86, and MHC-class II antigen in expression patterns of the cell surface antigen markers, or verifies that cells are negative for at least one of CD40, CD80, CD86, and MHC-class II antigen in expression patterns of the cell surface antigen markers,

Quality test e which verifies that cells remain negative for CD40, CD80, and CD86 and become positive for MHC-class II antigen in expression patterns of the cell surface antigen markers when the cells are stimulated with interferon-γ, or verifies at least any one of cells being negative for CD40, negative for CD80, negative for CD86, or positive for MHC-class II antigen in expression patterns of the cell surface antigen markers when the cells are stimulated with interferon-y,

Quality Test f which verifies that cells express prostaglandin $E_2$ and/or vascular endothelial growth factor (VEGF) and an expression level of prostaglandin $E_2$ is increased by stimulating the cells with TNF-α,

Quality Test g which verifies that an expression level of aggrecan increases when cells are cultured in a medium containing a substance that induces differentiation into chondrocytes,

Quality Test h which verifies that an amount of calcium accumulated in cells increases when the cells are cultured in a medium containing a substance that induces differentiation into osteocytes,

Quality Test i which verifies that cells have an ability of dividing at least 10, 14, or 15 times on a cell culture plate, and

Quality Test j which verifies that an average doubling time of cells on a cell culture plate is within 96 hours, 84 hours, 72 hours, 48 hours, or 36 hours during a period of Quality Test i.

5. The production method according to any one of the above-described 1 to 4, further comprising: loading the suspension on a filtration membrane to collect cells which have been passed through in the step (g).

6. The production method according to the above-described 5, wherein the filtration membrane has a pore diameter of 20 μm to 80 μm.

7. The production method according to any one of the above-described 1 to 6, wherein the dental pulp is obtained from human permanent teeth or deciduous teeth.

8. The production method according to any one of the above-described 1 to 7, wherein the protease used in the step (a) comprises a serine protease, a metalloprotease, or a mixture thereof.

9. The production method according to any one of the above-described 1 to 7, wherein the protease used in the step (a) comprises a metalloprotease.

10. The production method according to any one of the above-described 1 to 7, wherein the protease used in the step (a) comprises a matrix metalloprotease, a neutral metalloprotease, or a mixture thereof.

11. The production method according to any one of the above-described 1 to 7, wherein the protease used in the step (a) comprises a collagenase and a neutral metalloprotease.

12. The production method according to the above-described 11, wherein the collagenase comprises collagenase I, collagenase II, or a mixture thereof.

13. The production method according to the above-described 10 or 11, wherein the neutral metalloprotease comprises thermolysin, Dispase, or a mixture thereof.

14. The production method according to any one of the above-described 1 to 13, wherein the first cryopreservation liquid used in the step (c) includes a bicarbonate Ringer's solution, human serum albumin, and DMSO.

15. The production method according to any one of the above-described 1 to 14, wherein the carrier particles used in the step (e) have a substantially spherical shape having a diameter of 80 to 300 μm in a swollen state.

16. The production method according to the above-described 15, wherein the carrier particles are porous carrier particles having pores of 3 to 40 μm in diameter which open to the surfaces of the carrier particles in the swollen state.

17. The production method according to any one of the above-described 1 to 16, wherein the carrier particles contain gelatin.

18. The production method according to any one of the above-described 1 to 17, wherein the protease used in the step (f) comprises a serine protease, a metalloprotease, or a mixture thereof.

19. The production method according to any one of the above-described 1 to 17, wherein the protease used in the step (f) comprises trypsin.

20. The production method according to any one of the above-described 1 to 19, further comprising: a step (h) of freezing the suspension obtained in the step (g) in a state in which the suspension is suspended in a second cryopreservation liquid.

21. The production method according to the above-described 20, wherein the second cryopreservation liquid used in the step (h) includes a bicarbonate Ringer's solution, human serum albumin, and DMSO.

22. The production method according to any one of the above-described 1 to 19, wherein quality tests of the pluripotent stem cells are performed in the step (g).

23. The production method according to the above-described 20 or 21, wherein quality tests of the pluripotent stem cells are performed in the step (g) or (h).

24. The production method according to the above-described 22 or 23, wherein the quality tests are performed on cells fractionated from cells before suspension in the second cryopreservation liquid, subcultured cells which have been fractionated from the cells before suspension in the second cryopreservation liquid, pre-freezing cells which have been suspended in the second cryopreservation liquid and fractionated, cells immediately after thawing, which have been fractionated and frozen, and/or cells obtained by fractionation, freezing, thawing, and culturing.

25. The production method according to the above-described 22 or 24, wherein the quality tests are performed for one or more of the following Quality Tests a' to j'.

Quality Test a' which verifies that a proportion of the number of cells showing a substantially spindle-shaped form in all cells when observed under an optical microscope is greater than or equal to 99%, greater than or equal to 99.5%, greater than or equal to 99.9%, or greater than or equal to 99.95%,

Quality Test b' which verifies that a cell viability is greater than or equal to 50%, greater than or equal to 60%, greater than or equal to 70%, greater than or equal to 80%, greater than or equal to 90%, or greater than or equal to 95%,

Quality Test c' which verifies that cells are positive for CD73, CD90, CD105, and CD166 and negative for CD34 and CD45 in expression patterns of the cell surface antigen markers, or verifies that cells are positive for at least one of CD73 and CD90 and negative for CD34 in expression patterns of the cell surface antigen markers,

Quality Test d' which verifies that cells are negative for CD40, CD80, CD86, and MHC-class II antigen in expression patterns of the cell surface antigen markers, or verifies that cells are negative for at least CD40, CD80, CD86, and MHC-class II antigen in expression patterns of the cell surface antigen markers,

Quality Test e' which verifies that cells remain negative for CD40, CD80, and CD86 and become positive for MHC-class II antigen in expression patterns of the cell surface antigen markers when the cells are stimulated with interferon-y, or verifies at least any one of cells being negative for CD40, negative for CD80, negative for CD86, or positive for MHC-class II antigen in expression patterns of the cell surface antigen markers when the cells are stimulated with interferon-y,

Quality Test f' which verifies that cells express prostaglandin $E_2$ and/or vascular endothelial growth factor (VEGF) and an expression level of prostaglandin $E_2$ is increased by stimulating the cells with TNF-$\alpha$,

Quality Test g' which verifies that an expression level of aggrecan increases when cells are cultured in a medium containing a substance that induces differentiation into chondrocytes,

Quality Test h' which verifies that an amount of calcium accumulated in cells increases when the cells are cultured in a medium containing a substance that induces differentiation into osteocytes,

Quality Test i' which verifies that cells have an ability of dividing at least 3, 4, or 5 times on a cell culture plate, and

Quality Test j' which verifies that an average doubling time of cells on a cell culture plate is within 96 hours, 84 hours, 72 hours, 48 hours, or 36 hours during a period of Quality Test i'.

26. The production method according to any one of the above-described 22 to 25, wherein a positive rate of CD107b in the pluripotent stem cells used in the quality tests in the step (g) or (h) is higher than the corresponding positive rate of the pluripotent stem cells used in the quality tests in the step (c) or (d).

27. The production method according to any one of the above-described 22 to 26, wherein a positive rate of at least one of CD39, CD49a, CD61, CD107a, CD107b, and CD143 in the pluripotent stem cells used in the quality tests in the step (g) or (h) is higher than the corresponding positive rate of the pluripotent stem cells used in the quality tests in the step (c) or (d), and a positive rate of CD146 in the pluripotent stem cells used in the quality tests in the step (g) or (h) is lower than the corresponding positive rate of the pluripotent stem cells used in the quality tests in the step (c) or (d).

28. The production method according to any one of the above-described 22 to 27, wherein an expression level of at least one of IL-6, HGF, IGFBP-4, IL-11, TIMP-3, and TIMP-2 in the pluripotent stem cells used in the quality tests in the step (g) or (h) is higher than the corresponding expression level in the pluripotent stem cells used in the quality tests in the step (c) or (d).

29. Ccells obtained through the production method according to any one of the above-described 1 to 28.

30. The cells according to the above-described 29, which are positive for CD73, CD90, CD105, and CD166 and negative for CD34 and CD45.

31. The cells according to the above-described 30, which are negative for CD40, CD80, CD86, and MHC-class II antigen.

32. The cells according to the above-described 31, which become positive for the MHC-class II antigen when the cells are stimulated with interferon-$\gamma$.

33. Dental pulp-derived pluripotent stem cells having at least one characteristic shown in (1) to (4) below.

(1) the cells are positive for CD73, CD90, CD105, and CD166 and negative for CD34, CD40, CD45, CD80, CD86, and MHC-class II antigen, become positive for the MHC-class II antigen when the cells are stimulated with interferon-γ, and express prostaglandin $E_2$ and/or vascular endothelial growth factor, and an expression level of prostaglandin $E_2$ increases when the cells are stimulated with TNF-α,
(2) the cells are positive for at least one of CD73, CD90, CD105, and CD166 and negative for CD34 and CD45,
(3) the cells are positive for at least one of CD47, CD81, and CD147 and negative for at least one of CD19, CD34, and CD206, and
(4) the cells are positive for at least one of CD47, CD81, and CD147 and negative for at least one of CD19, CD31, CD33, CD34, CD38, CD45, CD206, CD235a, and SSEA-1.

34. The cells according to the above-described 33, which have an ability to differentiate into osteocyte and chondrocyte.
35. The cells according to the above-described 33 or 34, wherein an average diameter of the cells in a state in which the cells are made to float in a medium is 16 to 20 μm.
36. The cells according to any one of the above-described 33 to 35, which have an ability of dividing at least 3 times in an in vitro environment, and wherein an average doubling time is within 96 hours.
37. The dental pulp-derived pluripotent stem cells according to any one of the above-described 33 to 35, which have the following characteristics that the cells have divided at least 10, 15, 16, or 17 times in an in vitro environment, and an average time of the cell divisions is within 48 hours, and the cells have an ability of dividing at least 10, 14, or 15 times in an in vitro environment, and an average time of the cell divisions is within 96 hours, 84 hours, 72 hours, 48 hours, or 36 hours.
38. The dental pulp-derived pluripotent stem cells according to any one of the above-described 33 to 35, which have the following characteristics that the cells have divided at least 16, 21, 22, or 23 times in an in vitro environment, and the cells have an ability of dividing at least 3, 4, or 5 times in an in vitro environment, and an average time of the cell divisions is within 96 hours, 84 hours, 72 hours, 48 hours, or 36 hours.
39. The dental pulp-derived pluripotent stem cells according to any one of the above-described 33 to 38, which are negative for at least one of CD19, CD26, CD106, CD117, and CD271.
40. The dental pulp-derived pluripotent stems cell according to any one of the above-described 33 to 39, which are positive for at least one of CD140b and HLA-A, -B, and -C.
41. The dental pulp-derived pluripotent stem cells according to any one of the above-described 33 to 40, which are negative for at least one of CD56 and CD146.
42. The dental pulp-derived pluripotent stem cells according to any one of the above-described 33 to 41, which are positive for at least one of CD49e and CD95.
43. The dental pulp-derived pluripotent stem cells according to any one of the above-described 33 to 42 which are positive for at least one of CD10, CD46, CD47, CD55, CD58, and CD59.
44. The dental pulp-derived pluripotent stem cells according to any one of the above-described 33 to 43, which express at least one of MMP-2, IGFBP-4, cystatin C, IL-6, IL-11, MCP-1, IL-8, HGF, VEGF, TIMP-1, TIMP-2, and TIMP-3.
45. The dental pulp-derived pluripotent stem cells according to any one of the above-described 33 to 44, which express at least one of GROα, VCAM-I, and IP-10.
46. The dental pulp-derived pluripotent stem cells according to any one of the above-described 33 to 45, which express IL-6 and in which an expression level thereof is increased by TNF-α stimulation and interferon-γ stimulation.
47. The dental pulp-derived pluripotent stem cells according to any one of the above-described 33 to 46, which express IL-11 and in which an expression level thereof is increased by TNF-α stimulation and interferon-γ stimulation.
48. The dental pulp-derived pluripotent stem cells according to any one of the above-described 33 to 47, which express IP-10 and in which an expression level thereof is increased by TNF-α stimulation and interferon-γ stimulation.
49. The dental pulp-derived pluripotent stem cells according to any one of the above-described 33 to 48, which express MCP-1 and in which an expression level thereof is increased by TNF-α stimulation and interferon-γ stimulation.
50. The dental pulp-derived pluripotent stem cells according to any one of the above-described 33 to 49, in which expression of GM-CSF is induced by TNF-α stimulation.
51. The dental pulp-derived pluripotent stem cells according to any one of the above-described 33 to 50, which express HGF and in which an expression level thereof is decreased by TNF-α stimulation and increases by interferon-γ stimulation.
52. The dental pulp-derived pluripotent stem cells according to any one of the above-described 33 to 51, which express IL-8 and in which an expression level thereof is increased by TNF-α stimulation.
53. The dental pulp-derived pluripotent stem cells according to any one of the above-described 33 to 52, wherein the dental pulp is obtained from human permanent teeth or deciduous teeth.

54. A composition comprising: the dental pulp-derived pluripotent stem cells according to any one of the above-described 33 to 53 suspended in a bicarbonate Ringer's solution containing human serum albumin and dimethyl sulfoxide.
55. A composition comprising: the dental pulp-derived pluripotent stem cells according to any one of the above-described 33 to 53 suspended in a solution containing sodium ions, potassium ions, calcium ions, magnesium ions, hydrogen carbonate ions, citrate ions, human serum albumin, and dimethyl sulfoxide.
56. The composition according to the above-described 55, wherein sodium ions, potassium ions, calcium ions, magnesium ions, hydrogen carbonate ions, citrate ions, human serum albumin, and dimethyl sulfoxide are respectively contained at concentrations of 91 to 113 mM, 2.52 to 3.08 mM, 0.95 to 1.16 mM, 0.315 to 0.385 mM, 15.6 to 19.2 mM, 1.04 to 1.28 mM, 46 to 56 g/L, and 9% to 11% (v/v).
57. The composition according to any one of the above-described 54 to 56, further comprising: acetyltryptophan or salts thereof; and caprylic acid or salts thereof.
58. The composition according to any one of the above-described 54 to 57, wherein the dental pulp-derived pluripotent stem cells are contained at a density of $5 \times 10^6$ to $8 \times 10^7$ cells/mL.
59. The composition according to any one of the above-described 54 to 58, which are encapsulated in a container in an amount of 1 to 20 mL.
60. The composition according to the above-described 59, wherein the container is made of glass or plastic.
61. The composition according to any one of the above-described 54 to 60, which is in a frozen state.
62. A pharmaceutical composition comprising: the composition according to any one of the above-described 54 to 61.
63. The pharmaceutical composition according to the above-described 62, which is a therapeutic agent for a disease selected from the group consisting of an autoimmune disease, an inflammatory disease, rheumatoid arthritis, Crohn's disease, chronic inflammatory bowel disease, myocardial infarction, cerebral infarction (including chronic cerebral infarction and acute cerebral infarction), chronic inflammatory demyelinating polyneuritis, multiple sclerosis, systemic lupus erythematosus, liver cirrhosis (including decompensated liver cirrhosis), sepsis, osteoarthritis, psoriasis, and organ graft rejection.
64. The pharmaceutical composition according to the above-described 62, for use in treating a disease selected from the group consisting of an autoimmune disease, an inflammatory disease, rheumatoid arthritis, Crohn's disease, chronic inflammatory bowel disease, myocardial infarction, cerebral infarction (including chronic cerebral infarction and acute cerebral infarction), chronic inflammatory demyelinating polyneuritis, multiple sclerosis, systemic lupus erythematosus, liver cirrhosis (including decompensated liver cirrhosis), sepsis, osteoarthritis, psoriasis, and organ graft rejection.

**Advantageous Effects of Invention**

According to the present invention, a novel pluripotent stem cell preparation derived from dental pulp which is useful for treating cerebral infarction or the like and can be administered to humans is provided. In addition, according to the present invention, it is possible to efficiently produce such dental pulp-derived cells enriched with pluripotent stem cells (pluripotent stem cell-enriched dental pulp-derived cells) which can be administered to humans, and therefore, it is possible to stably supply pharmaceutical products containing these dental pulp-derived cells as active components according to demand. In addition, since the pluripotent stem cell-enriched dental pulp-derived cells can be provided in a stable form, a possibility of quality deterioration during distribution or storage can also be avoided.

**Brief Description of Drawings**

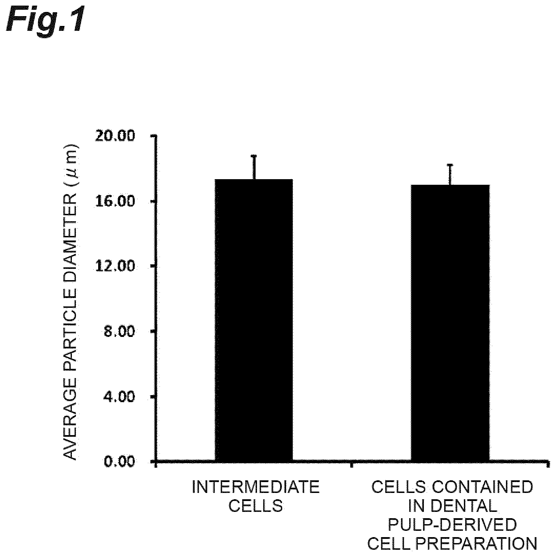

Fig. 1 is a graph showing an average of measurement results of cell particle diameters of dental pulp-derived cells (intermediate cells, and cells contained in dental pulp-derived cell preparations) which are respectively contained in intermediates and the dental pulp-derived cell preparations respectively obtained in Examples 7 and 16. The vertical axis indicates an average particle diameter (μm). Average particle diameters of the intermediate cells and the cells contained in the dental pulp-derived cell preparations are shown in order from left to right. Error bars indicate a standard deviation (n=3).

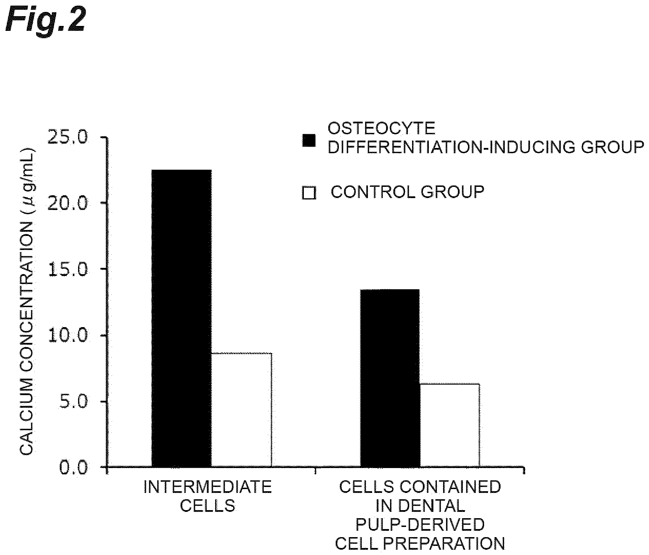

Fig. 2 is a graph showing test results of abilities to differentiate into osteocytes for the dental pulp-derived cells obtained in Examples 7 and 16. The vertical axis indicates a calcium concentration (μg/mL). Regarding the intermediate cells (Example 7) and the cells in the dental pulp-derived cell preparations (Example 16), black bars indicate concentrations of calcium released from cells of osteocyte differentiation-inducing groups and white bars indicate concentrations of calcium released from cells of control groups.

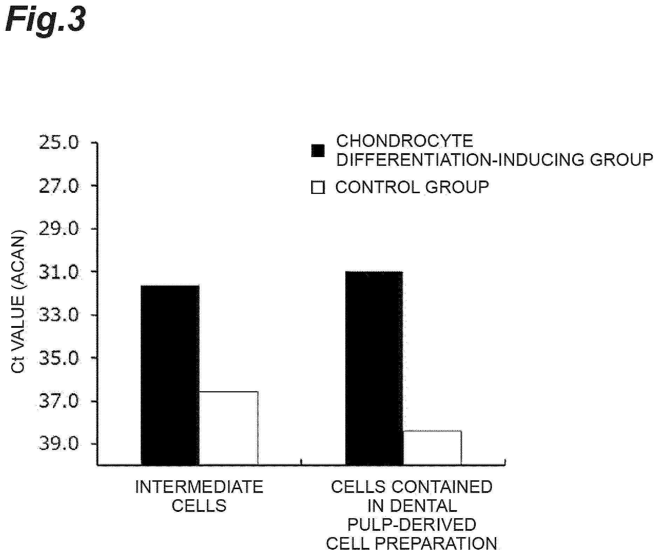

Fig. 3 is a graph showing test results of abilities to differentiate into chondrocytes for the dental pulp-derived cells obtained in Examples 7 and 16. The vertical axis indicates a Ct value of aggrecan (ACAN). Regarding the intermediate cells and the cells in the dental pulp-derived cell preparations, black bars indicate Ct values of aggrecan (ACAN) of cells of chondrocyte differentiation-inducing groups and white bars indicate Ct values of aggrecan (ACAN) of cells of control groups.

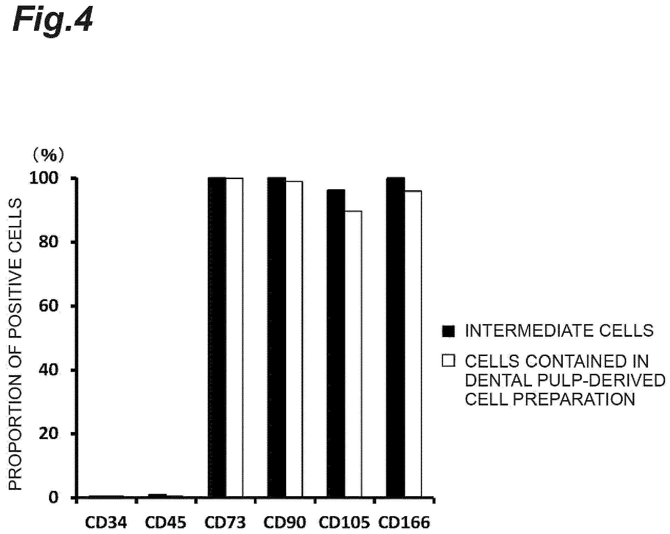

Fig. 4 is a graph showing measurement results of surface antigens of the dental pulp-derived cells obtained in Examples 7 and 16. The vertical axis indicates a proportion (%) of positive cells. Regarding the intermediate cells (black bars) and the cells (white bars) in the dental pulp-derived cell preparations, proportions of positive cells for CD34, CD45, CD73, CD90, CD105, and CD166 are shown in order from left to right.

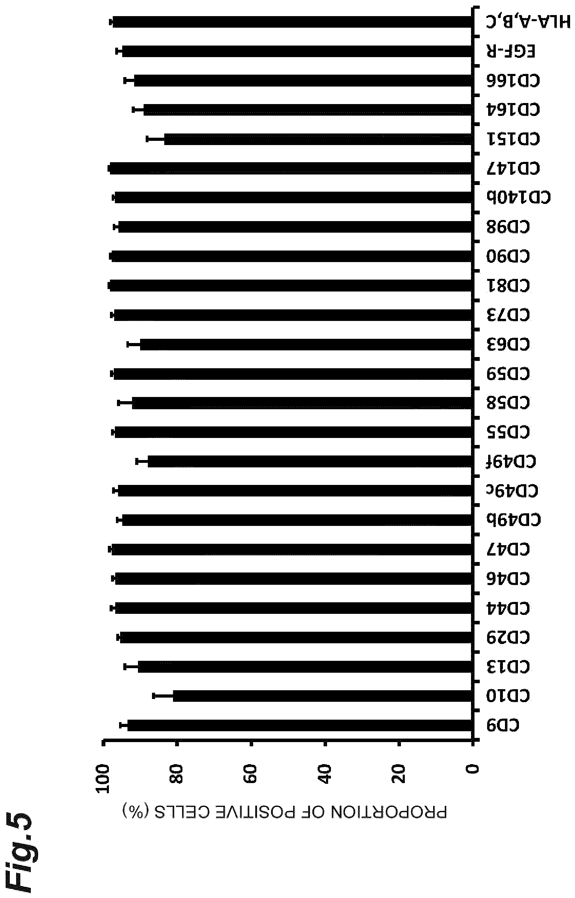

Fig. 5 is a graph showing measurement results of surface antigens of the dental pulp-derived cells (intermediate cells) which are contained in the intermediates obtained in Example 7. The vertical axis indicates a proportion (%) of positive cells. Proportions of positive cells for CD9, CD10, CD13, CD29, CD44, CD46, CD47, CD49b, CD49c, CD49f, CD55, CD58, CD59, CD63, CD73, CD81, CD90, CD98, CD140b, CD147, CD151, CD164, CD166, epidermal growth factor receptor (EGF-R), and human leukocyte antigen-A, -B, and -C (HLA-A, -B, and -C) are shown in order from left to right. The values are average values, and the error bars indicate standard errors (n=9).

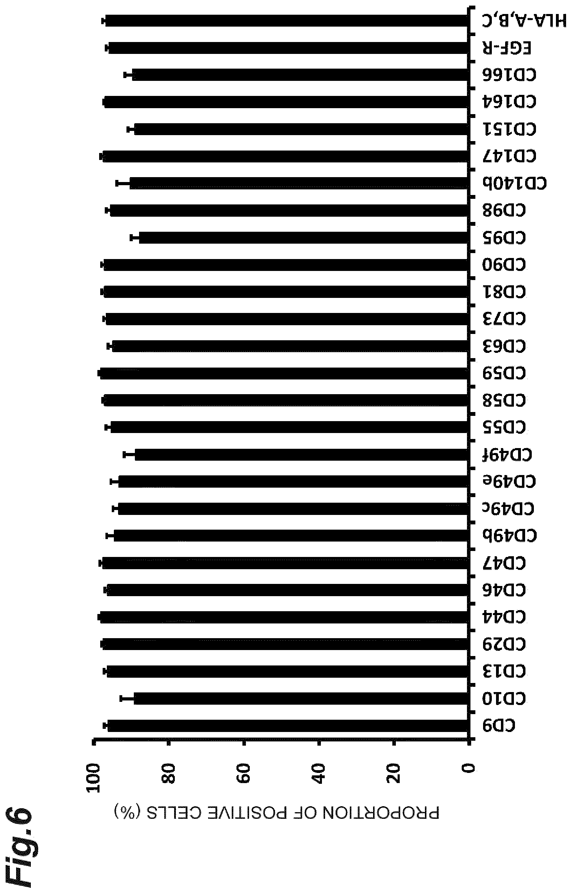

Fig. 6 is a graph showing measurement results of surface antigens of the cells contained in the dental pulp-derived cell preparations obtained in Example 16. The vertical axis indicates a proportion (%) of positive cells. Proportions of positive cells for CD9, CD10, CD13, CD29, CD44, CD46, CD47, CD49b, CD49c, CD49e, CD49f, CD55, CD58, CD59, CD63, CD73, CD81, CD90, CD95, CD98, CD140b, CD147, CD151, CD164, CD166, EGF-R, and HLA-A, -B, and -C are shown in order from left to right. The values are average values, and the error bars indicate standard errors (n=7).

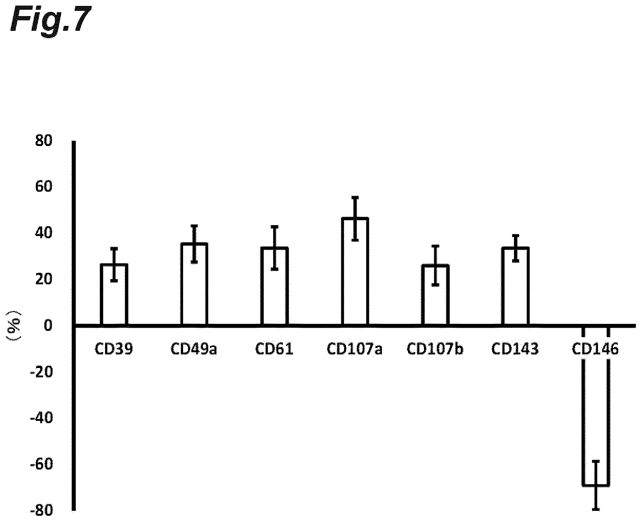

Fig. 7 is a graph showing differences (positive rate (%) in preparations - positive rate (%) in intermediates) between the intermediates and the preparations respectively obtained in Examples 7 and 16 for surface antigens having a positive rate difference of at least 10% in the dental pulp-derived cells contained in the intermediates and the preparations. Results for CD39, CD49a, CD61, CD107a, CD107b, CD143, and CD146 are shown in order from left to right. The values are average values, and the error bars indicate standard errors (n=7).

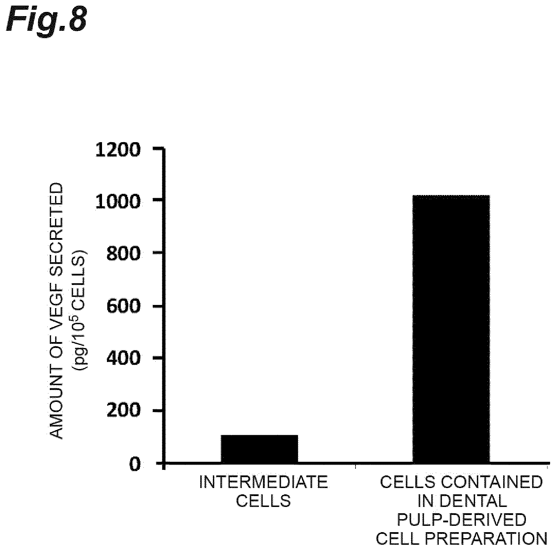

Fig. 8 is a graph showing test results of VEGF secretory abilities of the dental pulp-derived cells in the intermediates and the preparations respectively obtained in Examples 7 and 16. The vertical axis indicates the amount (pg/1 $\times$ $10^5$ cells) of vascular endothelial growth factor (VEGF) secreted.

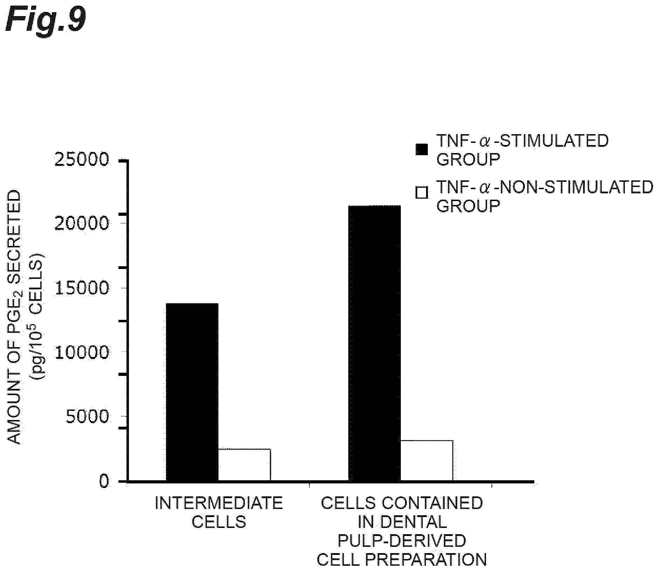

Fig. 9 is a graph showing test results of prostaglandin $E_2$ ($PGE_2$) secretory abilities of the dental pulp-derived cells in the intermediates and the preparations respectively obtained in Examples 7 and 16. The vertical axis indicates the amount (pg/1 $\times$ $10^5$ cells) of $PGE_2$ secreted. The black bars indicate results in tumor necrosis factor-a (TNF-$\alpha$)-stimulated groups, and the white bars indicate results in TNF-$\alpha$-non-stimulated groups.

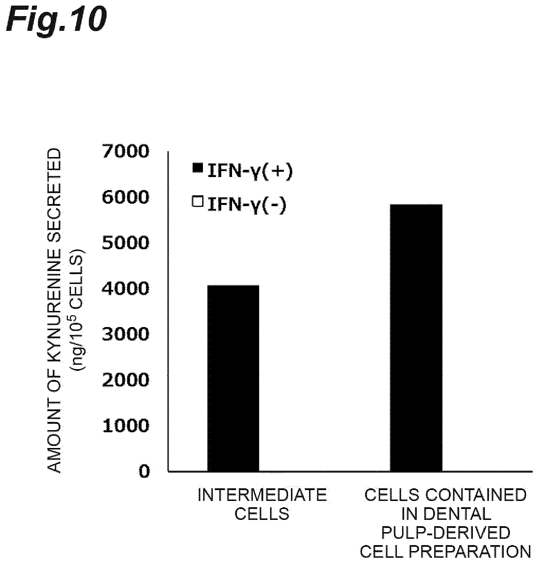

Fig. 10 is a graph showing test results of kynurenine secretory abilities of the dental pulp-derived cells obtained in the Examples 7 and 16. The vertical axis indicates the amount (ng/1 $\times$ $10^5$ cells) of kynurenine secreted for the intermediate cells and the cells in the dental pulp-derived cell preparations.

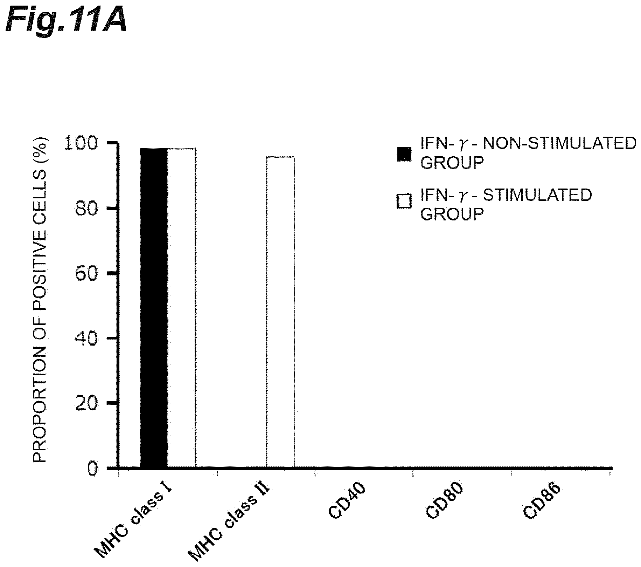

Fig. 11A is a graph showing test results of low immunogenicity of the dental pulp-derived cells in the intermediates obtained in Example 7. The vertical axis indicates a proportion (%) of positive cells. The black bars indicate results in interferon-y (IFN-y)-non-stimulated groups, and the white bars indicate results in IFN-$\gamma$-stimulated groups. Proportions of positive cells for major histocompatibility (MHC) class I antigen, major histocompatibility (MHC) class II antigen, CD40, CD80, and CD86 are shown in order from left to right.

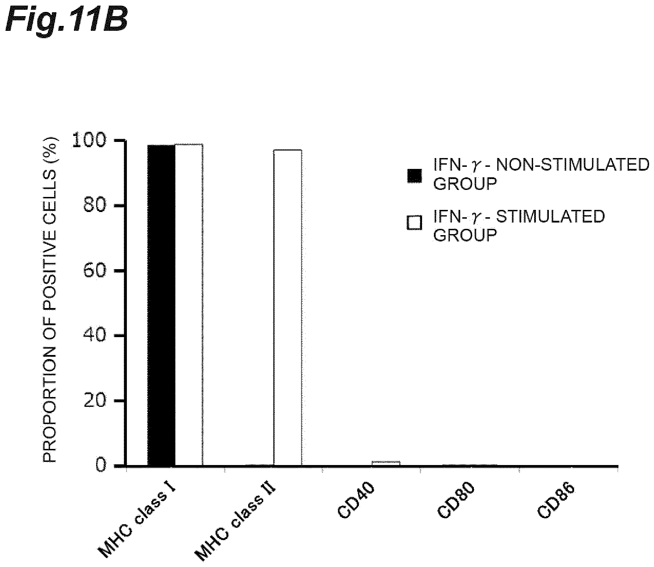

Fig. 11B is a graph showing test results of low immunogenicity of the dental pulp-derived cells in the preparations obtained in Example 16. The vertical axis indicates a proportion (%) of positive cells. The black bars indicate results in IFN-$\gamma$-non-stimulated groups, and the white bars indicate results in IFN-$\gamma$-stimulated groups. Proportions of positive cells for MHC-class I antigen, MHC-class II antigen, CD40, CD80, and CD86 in order from left to right.

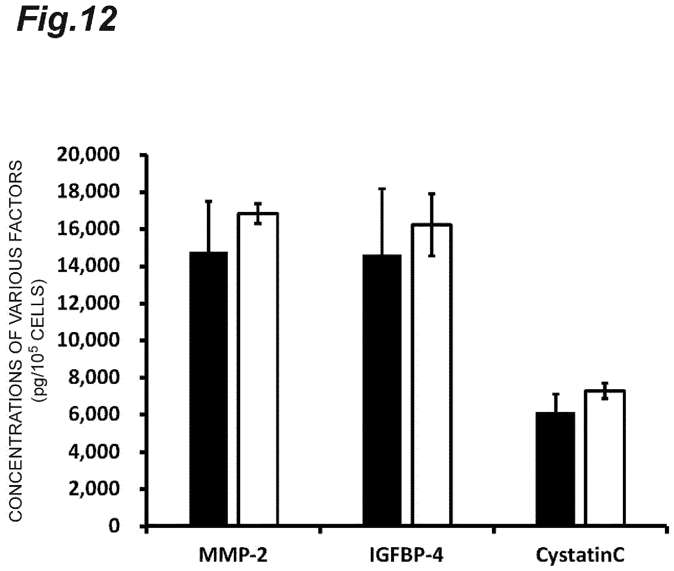

Fig. 12 is a graph showing measurement results of the concentrations of various factors such as a cytokine in culture supernatants when the dental pulp-derived cells contained in the intermediates and the preparations respectively obtained in Examples 7 and 16 are not stimulated. The vertical axis indicates the concentration (pg/1 $\times$ $10^5$ cells) of the various factors. Concentrations of matrix metalloprotease-2 (MMP-2), insulin-like growth factor-binding protein-4 (IGFBP-4), and cystatin C for the intermediate cells (black bars) and the cells (white bars) in the dental pulp-derived cell preparations are shown in order from left to right. The values are average values, and the error bars indicate standard errors (n=3).

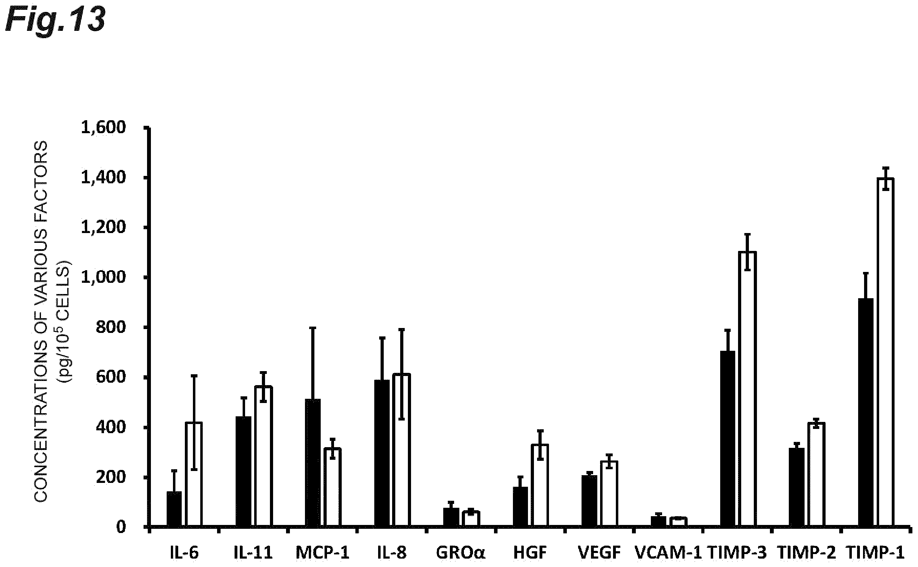

Fig. 13 is a graph showing measurement results of the concentrations of various factors such as a cytokine in the culture supernatants when the dental pulp-derived cells contained in the intermediates and the preparations respectively obtained in Examples 7 and 16 are not stimulated. The vertical axis indicates the concentration (pg/1 $\times$ $10^5$ cells) of the various factors. Concentrations of interleukin-6 (IL-6), interleukin-11 (IL-11), monocyte chemotactic protein-1 (MCP-1), interleukin-8 (IL-8), human growth regulated protein alpha (GRO$\alpha$), hepatocyte growth factor (HGF), vascular endothelial growth factor (VEGF), vascular cell adhesion molecule-1 (VCAM-1), tissue inhibitor of metalloproteinase-3 (TIMP-3), tissue inhibitor of metalloproteinase-2 (TIMP-2), and tissue inhibitor of metalloproteinase-1 (TIMP-1) for the intermediate cells (black bars) and the cells (white bars) in the dental pulp-derived cell preparations are shown in order from left to right. The values are average values, and the error bars indicate standard errors (n=3).

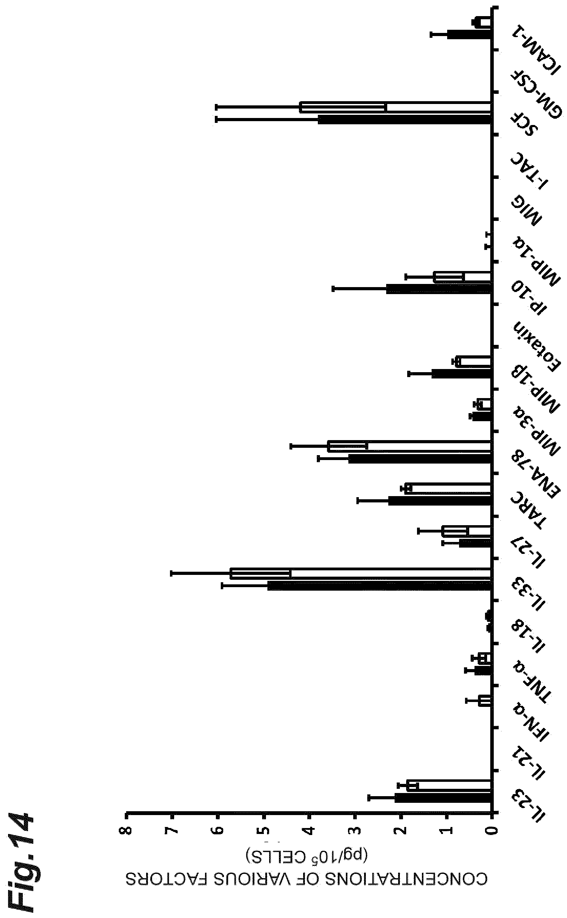

Fig. 14 is a view showing measurement results of the concentrations of various factors such as a cytokine in the culture supernatants when the dental pulp-derived cells contained in the intermediates and the preparations respectively obtained in Examples 7 and 16 are not stimulated. The vertical axis indicates the concentration (pg/1 $\times$ $10^5$ cells) of the various factors. Concentrations of interleukin-23 (IL-23), interleukin-21 (IL-21), interferon-a (IFN-$\alpha$), TNF-$\alpha$, interleukin-18 (IL-18), interleukin-33 (IL-33), interleukin-27 (IL-27), thymus and activation-regulated chemokine (TARC), epithelial neutrophil-activating protein-78 (ENA-78), macrophage inflammatory protein-3$\alpha$ (MIP-3$\alpha$), macrophage inflammatory protein-1$\beta$ (MIP-1$\beta$), eotaxin, interferon-$\gamma$-inducible protein-10 (IP-10), macrophage inflammatory protein-la (MIP-1$\alpha$), monokine induced by gamma interferon (MIG), interferon-inducible T-cell alpha chemoattractant (I-TAC), stem cell factor (SCF), granulocyte-monocyte colony-stimulating factor (GM-CSF), and intercellular adhesion moleculue-1 (ICAM-1) for the intermediates (black bars) and the preparations (white bars) are shown in order from left to right. The values are average values, and the error bars indicate standard errors (n=3).

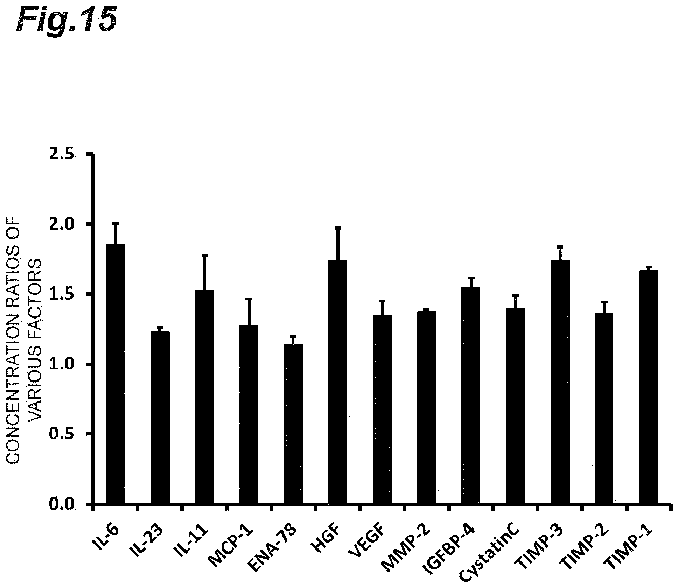

Fig. 15 is a graph showing ratios (concentration in preparation / concentration in intermediate) of factors of which the concentrations in a preparation are higher than those in an intermediate among various factors such as a cytokine in the culture supernatants when the dental pulp-derived cells contained in the intermediates and the preparations respectively obtained in Examples 7 and 16 are not stimulated. Concentration ratios for IL-6, IL-23, IL-11, MCP-1, ENA-78, HGF, VEGF, MMP-2, IGFBP-4, cystatin C, TIMP-3, TIMP-2, and TIMP-1 are shown in order from left to right. The values are average values, and the error bars indicate standard errors (n=3).

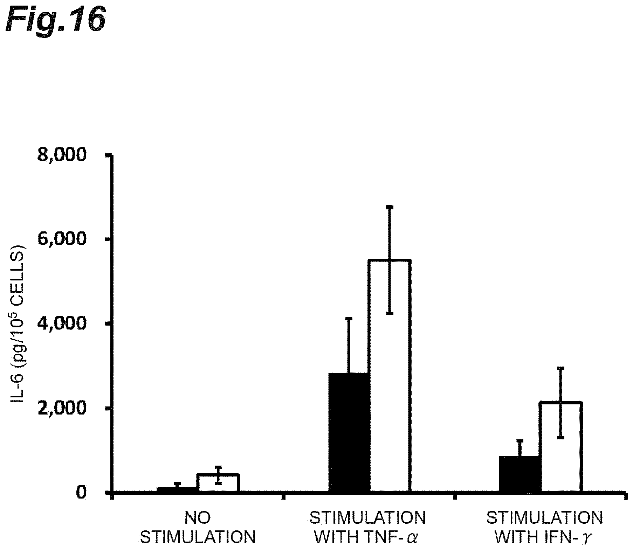

Fig. 16 is a graph showing concentrations of IL-6 in the culture supernatants in groups of non-stimulation, TNF-$\alpha$ stimulation, and IFN-$\gamma$ stimulation for the dental pulp-derived cells contained in the intermediates and the preparations respectively obtained in Examples 7 and 16. The vertical axis indicates a concentration (pg/1 $\times$ $10^5$ cells). Concentrations in the non-stimulated groups, TNF-$\alpha$-stimulated groups, and the IFN-$\gamma$-stimulated groups for the intermediates (black bars) and the preparations (white bars) are shown in order from left to right. The values are average values, and the error bars indicate standard errors (n=3).

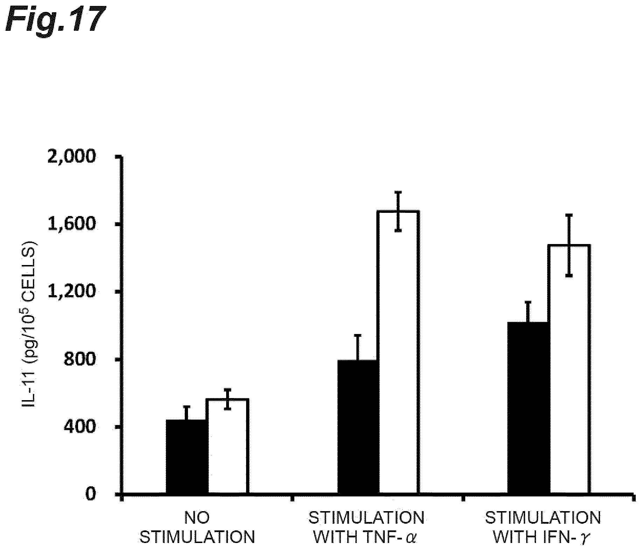

Fig. 17 is a graph showing concentrations of IL-11 in the culture supernatants in groups of non-stimulation, TNF-$\alpha$ stimulation, and IFN-$\gamma$ stimulation for the dental pulp-derived cells contained in the intermediates and the preparations respectively obtained in Examples 7 and 16. The vertical axis indicates a concentration (pg/1 $\times$ $10^5$ cells). Results of the non-stimulated groups, TNF-$\alpha$-stimulated groups, and the IFN-$\gamma$-stimulated groups for the intermediates (black bars) and the preparations (white bars) are shown in order from left to right. The values are average values, and the error bars indicate standard errors (n=3).

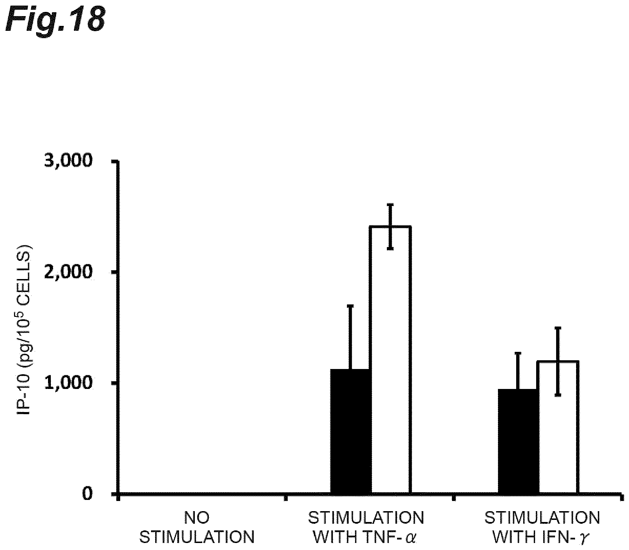

Fig. 18 is a graph showing concentrations of IP-10 in the culture supernatants in groups of non-stimulation, TNF-$\alpha$ stimulation, and IFN-$\gamma$ stimulation for the dental pulp-derived cells contained in the intermediates and the preparations respectively obtained in Examples 7 and 16. The vertical axis indicates a concentration (pg/1 $\times$ $10^5$ cells). Results of the non-stimulated groups, TNF-$\alpha$-stimulated groups, and the IFN-$\gamma$-stimulated groups for the intermediates (black bars) and the preparations (white bars) are shown in order from left to right. The values are average values, and the error bars indicate standard errors (n=3).

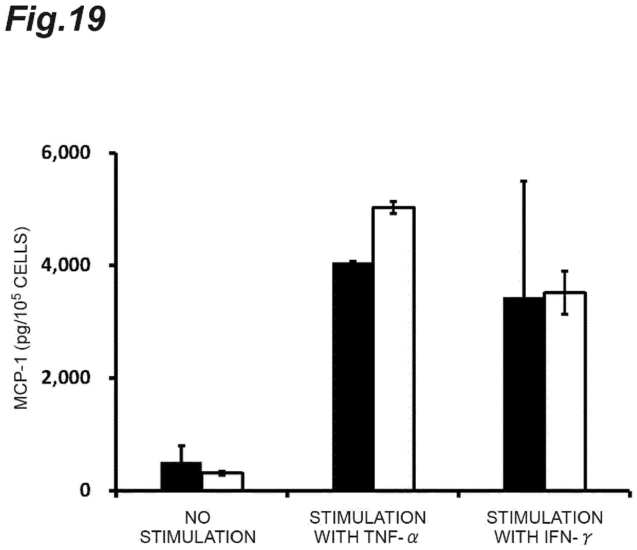

Fig. 19 is a graph showing concentrations of MCP-1 in the culture supernatants in groups of non-stimulation, TNF-$\alpha$ stimulation, and IFN-$\gamma$ stimulation for the dental pulp-derived cells contained in the intermediates and the preparations respectively obtained in Examples 7 and 16. The vertical axis indicates a concentration (pg/1 $\times$ $10^5$ cells). Results of the non-stimulated groups, TNF-$\alpha$-stimulated groups, and the IFN-$\gamma$-stimulated groups for the intermediates (black bars) and the preparations (white bars) are shown in order from left to right. The values are average values, and the error bars indicate standard errors (n=3).

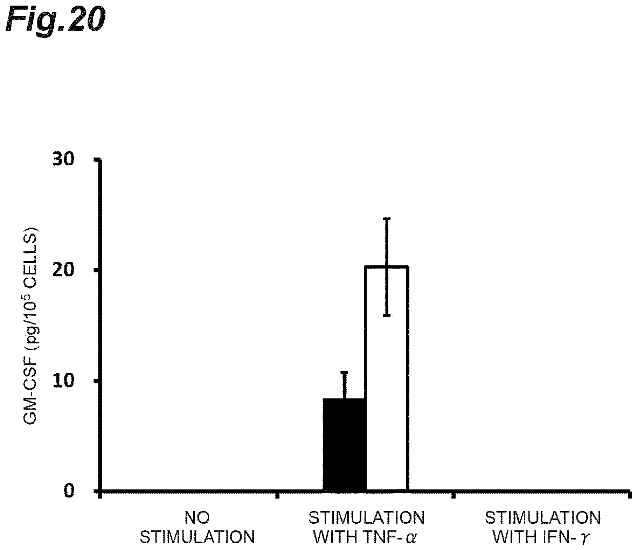

Fig. 20 is a graph showing concentrations of GM-CSF in the culture supernatants in groups of non-stimulation, TNF-$\alpha$ stimulation, and IFN-$\gamma$ stimulation for the dental pulp-derived cells contained in the intermediates and the preparations respectively obtained in Examples 7 and 16. The vertical axis indicates a concentration (pg/1 $\times$ $10^5$ cells). Results in the non-stimulated groups, TNF-$\alpha$-stimulated groups, and the IFN-$\gamma$-stimulated groups for the intermediates (black bars) and the preparations (white bars) are shown in order from left to right. The values are average values, and the error bars indicate standard errors (n=3).

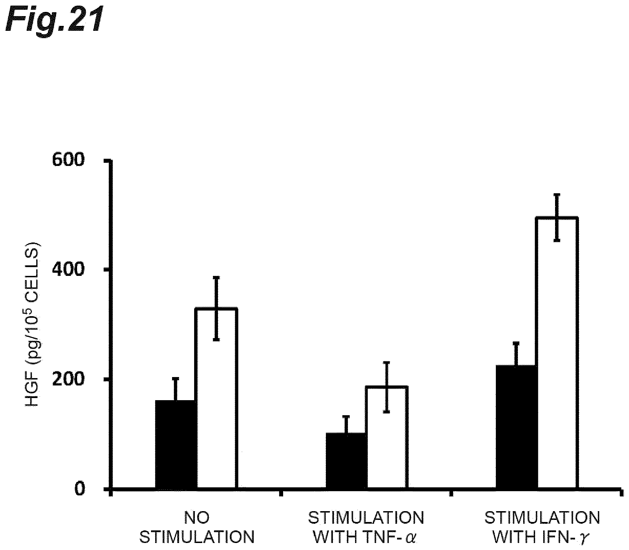

Fig. 21 is a graph showing concentrations of HGF in the culture supernatants in groups of non-stimulation, TNF-$\alpha$ stimulation, and IFN-$\gamma$ stimulation for the dental pulp-derived cells contained in the intermediates and the preparations respectively obtained in Examples 7 and 16. The vertical axis indicates a concentration (pg/1 $\times$ $10^5$ cells). Results in the non-stimulated groups, TNF-$\alpha$-stimulated groups, and the IFN-$\gamma$-stimulated groups for the intermediates (black bars) and the preparations (white bars) are shown in order from left to right. The values are average values, and the error bars indicate standard errors (n=3).

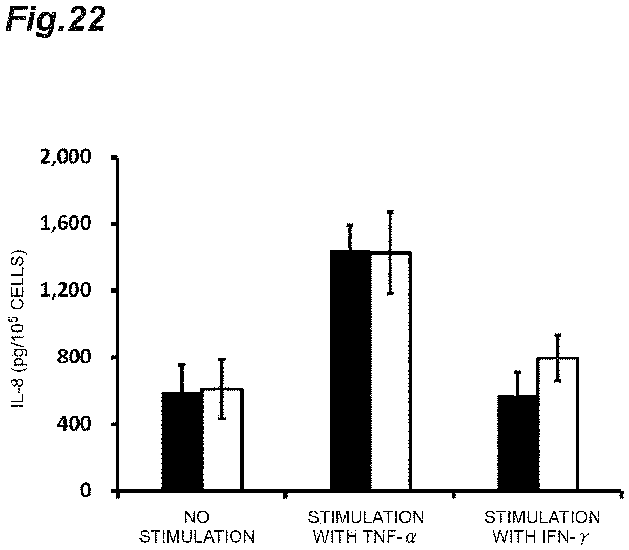

Fig. 22 is a graph showing concentrations of IL-8 in the culture supernatants in groups of non-stimulation, TNF-$\alpha$ stimulation, and IFN-$\gamma$ stimulation for the dental pulp-derived cells contained in the intermediates and the preparations respectively obtained in Examples 7 and 16. The vertical axis indicates a concentration (pg/1 $\times$ $10^5$ cells). Results in the non-stimulated groups, TNF-$\alpha$-stimulated groups, and the IFN-$\gamma$-stimulated groups for the intermediates (black bars) and the preparations (white bars) are shown in order from left to right. The values are average values, and the error bars indicate standard errors (n=3).

**Description of Embodiments**

Pluripotent stem cell is a cell having a self-replication ability and an ability to differentiate into two or more cells. Human dental pulp-derived pluripotent stem cell that can be obtained by the present invention preferably has abilities to differentiate into chondrocyte and osteocyte, but the present invention is not particularly limited thereto.

The fact that the pluripotent stem cell that can be obtained by the present invention has an ability to differentiate into chondrocyte can be confirmed by, for example, quantitatively determining an expression level of the aggrecan gene when the cell is cultured in a medium containing a substance known to induce differentiation of the cell into chondrocyte. Aggrecan is a main molecule constituting the extracellular matrix of cartilage, and its expression level increases when differentiation into chondrocyte of cell is induced. In this measurement method, TGF-b3 can be used as the substance (chondrocyte differentiation inducer) which induces differentiation into chondrocyte of cell, for example. A measurement method described in Example 20 is a suitable example of the measurement method for the ability to differentiate into chondrocyte.

The fact that the pluripotent stem cell that can be obtained by the present invention has an ability to differentiate into osteocyte can be confirmed by, for example, quantitatively determining the amount of calcium accumulated in cell when the cell is cultured in a medium containing a substance known to induce differentiation of the cell into osteocyte. When differentiation into osteocyte is induced, calcium accumulates in cell. In this measurement method, dexamethasone, a $\beta$-glycerophosphate, glycosaminoglycan, ascorbic acid, osteogenic factor 2 (BMP-2), and salts thereof can be used singly or in combination of two or more thereof as the substance (osteocyte differentiation inducer) which induces differentiation into osteocyte, for example. For example, it is possible to suitably use a combination of dexamethasone, an ascorbate, and a $\beta$-glycerophosphate as an osteocyte differentiation-inducing agent. A measurement method described in Example 19 is a suitable example of the measurement method for the ability to differentiate into osteocyte.

"Dental pulp-derived cells enriched with pluripotent stem cells" or "pluripotent stem cell-enriched dental pulp-derived cells" in the present invention are derived from dental pulp, and an aggregate of cells in which a proportion (number proportion) of pluripotent stem cells is higher than that in cells directly collected from dental pulp, and includes pluripotent stem cells finally selectively isolated from dental pulp through culture or the like. The cells directly collected from dental pulp are cells in which pluripotent stem cells are mixed with other cells. When these cells are cultured, the proportion of pluripotent stem cells at the completion of the culture is higher compared to that at the start of the culture because pluripotent stem cells have a higher proliferation ability than other cells. Since the proportion of pluripotent stem cells is further increased by repeating culturing, cells containing almost only pluripotent stem cells can be obtained. That is, "pluripotent stem cell-enriched dental pulp-derived cells" include cells in which the proportion of pluripotent stem cells in all cells is higher than in cells immediately after being collected from dental pulp due to proliferation of the pluripotent stem cells in the process of culture or the like. Cells derived from human dental pulp are particularly called "pluripotent stem cell-enriched human dental pulp-derived cells."

In the present invention, pluripotent stem cells directly isolated from dental pulp and pluripotent stem cells contained in pluripotent stem cell-enriched dental pulp-derived cells obtained from dental pulp through culture are collectively called "dental pulp-derived pluripotent stem cells", and sometimes simply called "dental pulp-derived stem cells" or "dental pulp stem cells." Pluripotent stem cells derived from human dental pulp are particularly called "human dental pulp-derived pluripotent stem cells," or sometimes simply called "human dental pulp-derived stem cells" or "human dental pulp stem cells." "Cells contained in intermediates (intermediate cells)" and "cells contained in dental pulp-derived cell preparations" which are one embodiment described in the present specification substantially contain only pluripotent stem cells, and both are dental pulp-derived pluripotent stem cells.

In the present invention, in a case where a cell population to be tested is positive for certain antigens in expression patterns of the surface antigen markers, this means that preferably at least 30%, more preferably at least 40%, still more preferably at least 50%, still more preferably at least 60%, still more preferably at least 70%, still more preferably at least 80%, still more preferably at least 90%, and particularly preferably at least 95% of the observed cells are positive for the antigens when the cell population is observed as a whole. Expression patterns of surface antigens can be examined through, for example, a method described in Example 21 or 22, but the present invention is not limited thereto.

In the present invention, in a case where a cell population to be tested is negative for certain antigens in expression patterns of the surface antigen markers, this means that preferably less than 20%, more preferably less than 10%, still more preferably less than 5%, still more preferably less than 2%, and particularly preferably less than 1% of the observed cells are positive for the antigens when the cell population is observed as a whole. Expression patterns of surface antigens can be examined through, for example, a method described in Example 21 or 22, but the present invention is not limited thereto.

When the pluripotent stem cell-enriched dental pulp-derived cells of the present invention are cultured in a medium containing a substance known to induce differentiation into chondrocytes, an increase in expression level of aggrecan is observed as a whole. In addition, when the pluripotent stem cell-enriched dental pulp-derived cells of the present invention are cultured in a medium containing a substance known to induce differentiation into osteocytes, an increase in the amount of calcium accumulated in the cells is observed as a whole. That is, the pluripotent stem cell-enriched dental pulp-derived cells of the present invention have abilities to differentiate into chondrocytes and osteocytes when observed as a whole.

In one embodiment, when the pluripotent stem cell-enriched dental pulp-derived cells of the present invention are observed as a whole (population), the cells are positive for at least one of CD73, CD90, CD105, and CD166 in expression patterns of the surface antigen markers. Similarly, the pluripotent stem cell-enriched dental pulp-derived cells of the present invention are negative for at least one of CD34 and CD45. For example, when the pluripotent stem cell-enriched dental pulp-derived cells of the present invention are observed as a whole, the cells are, for example, positive for CD73 and CD90 and negative for CD34 in the expression patterns of the surface antigen markers. In addition, when the pluripotent stem cell-enriched dental pulp-derived cells of the present invention are observed as a whole, the cells are positive for CD73, CD90, CD105, and CD166 and negative for CD34 and CD45 in the expression patterns of the surface antigen markers. These expression patterns are common to mesenchymal stem cells.

In addition, in one embodiment, when the pluripotent stem cell-enriched dental pulp-derived cells of the present invention are observed as a whole, the cells are negative for at least one of CD40, CD80, CD86, and MHC-class II antigen in expression patterns of the surface antigen markers. For example, when the pluripotent stem cell-enriched dental pulp-derived cells of the present invention are observed as a whole, the cells are negative for CD40, CD80, CD86, and MHC-class II antigen in the expression patterns of the surface antigen markers. It is known that, when cells positive for these surface antigen markers are transplanted into allogeneic individuals, the cells tend to be recognized as an antigen to be excluded from a living body. In a case where cells are negative for at least one of these surface antigen markers, this means that the pluripotent stem cell-enriched dental pulp-derived cells of the present invention have a property of low immunogenicity to that extent and hardly any tend to be excluded from a living body when transplanted into allogeneic individuals. In addition, the cells may remain negative for CD40, CD80, and CD86 and be positive for MHC-class II antigen in the expression patterns of the surface antigen markers when the cells are stimulated with IFN-γ. For example, the cells remain negative for CD40, CD80, and CD86 and become positive for MHC-class II antigen when the cells are stimulated with IFN-γ.

In one embodiment, when the pluripotent stem cell-enriched dental pulp-derived cells of the present invention are observed as a whole, the cells are, for example, positive for CD73, CD90, CD105, and CD166 and negative for CD34, CD40, CD45, CD80, CD86, and MHC-class II antigen in expression patterns of the surface antigen markers. At this time, for example, the cells remain negative for CD40, CD80, and CD86 and can become positive for MHC-class II antigen when the cells are stimulated with IFN-γ.

In one embodiment, regarding a characteristic (a-1), when the pluripotent stem cell-enriched dental pulp-derived cells (dental pulp-derived pluripotent stem cells) of the present invention are observed as a whole, the cells are positive for at least one, preferably all of CD29, CD46, CD47, CD59, CD73, CD81, CD90, CD147, and HLA-A, -B, and -C in expression patterns of the surface antigen markers.

Here, when the individual cells included in all of the cells are observed, preferably at least 70%, more preferably at least 80%, and still more preferably at least 90% of the observed cells are positive for these antigens.

In addition, in one embodiment, regarding a characteristic (a-2), when the pluripotent stem cell-enriched dental pulp-derived cells of the present invention are observed as a whole, the cells are positive for at least one, preferably all of CD9, CD44, CD49b, CD49c, CD55, CD98, and EGF-R in expression patterns of the surface antigen markers.

Here, when the individual cells included in all of the cells are observed, preferably at least 65%, more preferably at least 75%, and still more preferably at least 80% of the observed cells are positive for these antigens.

In addition, in one embodiment, regarding a characteristic (a-3), when the pluripotent stem cell-enriched dental pulp-derived cells of the present invention are observed as a whole, the cells are positive for at least one, preferably all of CD49f, CD140b, and CD166 in expression patterns of the surface antigen markers.

Here, when the individual cells included in all of the cells are observed, preferably at least 60%, more preferably at least 70%, and still more preferably at least 75% of the observed cells are positive for these antigens.

In addition, regarding a characteristic (a-4), when the pluripotent stem cell-enriched dental pulp-derived cells of the present invention are observed as a whole, the cells are positive for at least one, preferably all of CD10, CD13, CD58, CD63, CD105, CD151, and CD164 in expression patterns of the surface antigen markers.

Here, when the individual cells included in all of the cells are observed, preferably at least 60%, more preferably at least 65%, and still more preferably at least 70% of the observed cells are positive for these antigens.

In one embodiment, the pluripotent stem cell-enriched dental pulp-derived cells of the present invention preferably have two or more, more preferably three or more, and still more preferably all of the characteristics shown in the above-described (a-1), (a-2), (a-3), and (a-4). For example, the cells having the characteristics shown in the above-described (a-1) and (a-2) are a suitable embodiment of the present invention.

In one embodiment, regarding a characteristic (a-5), when the pluripotent stem cell-enriched dental pulp-derived cells of the present invention are observed as a whole, the cells are negative for at least one, preferably all of CD120b, CD132, CD158a, CD161, CD184, CD195, CD206, CD210, CD212, CD226, CD244, CD267, CD278, CD279, CD282, CD294, stage-specific embryonic antigen-1 (SSEA-1), TRA-1-60, stage-specific embryonic antigen-3 (SSEA-3), cutaneous lymphocyte-associated antigen (CLA), and integrin β7 in expression patterns of the surface antigen markers.

Here, when the individual cells included in all of the cells are observed, preferably only at most 10%, more preferably only at most 5%, and still more preferably only at most 2%, and still more preferably only at most 1% of the observed cells are positive for these antigens.

In one embodiment, regarding a characteristic (a-6), when the pluripotent stem cell-enriched dental pulp-derived cells of the present invention are observed as a whole, the cells are negative for at least one, preferably all of CD8b, CD11b, CD15s, CD16, CD19, CD24, CD31, CD32, CD62E, CD62P, CD66f, CD86, CD88, CD94, CD100, CD103, CD114, CD117, CD118, CD121b, CD122, CD123, CD124, CD126, CD127, CD128b, CD135, CD137, CD137 ligand, CD150, CD163, CD172b, CD177, CD178, CD180, CD197, CD220, CD229, CD231, CD255, CD268, CD305, CD314, CD321, CDw327, CDw328, CD329, CD335, CD336, leukotriene B4 receptor (BLTR-1), CLIP, CMRF-44, CMRF-56, N-formylmethionyl-leucyl-phenylalanine receptor (fMLP-R), Invariant NKT, and γδ T-cell receptor (γδ TCR) in expression patterns of the surface antigen markers.

Here, when the individual cells included in all of the cells are observed, preferably only at most 10%, more preferably only at most 5%, and still more preferably only at most 2% of the observed cells are positive for these antigens.

In one embodiment, regarding a characteristic (a-7), when the pluripotent stem cell-enriched dental pulp-derived cells of the present invention are observed as a whole, the cells are negative for at least one, preferably all of CD1a, CD1b, CD1d, CD2, CD3, CD5, CD6, CD7, CD8a, CD11c, CD15, CD18, CD21, CD22, CD23, CD25, CD26, CD27, CD28, CD33, CD34, CD35, CD37, CD38, CD40, CD41a, CD41b, CD42b, CD45, CD45RB, CD45RO, CD48, CD50, CD53, CD62L, CD64, CD66 (a, c, d, e), CD69, CD70, CD72, CD80, CD84, CD85, CD87, CD89, CDw93, CD97, CD106, CD134, CD138, CD144, CD154, CD158b, CD162, CD183, CD205, CD235a, CD271, CD309, CD326, CD337, αβ T-cell receptor (αβ TCR), and MHC-class II antigen in expression patterns of the surface antigen markers.

Here, when the individual cells included in all of the cells are observed, preferably only at most 10% and more preferably only at most 5% of the observed cells are positive for these antigens.

In one embodiment, the pluripotent stem cell-enriched dental pulp-derived cells of the present invention preferably have two or more, more preferably three or more, and still more preferably all of the characteristics shown in the above-described (a-1), (a-2), (a-3), and (a-4), (a-5), (a-6), and (a-7). For example, the cells having the characteristics shown in the above-described (a-1) and (a-5) are a suitable embodiment of the present invention.

In one embodiment of the present invention, when the pluripotent stem cell-enriched dental pulp-derived cells of the present invention are observed as a whole, the cells express, for example, prostaglandin $E_2$ ($PGE_2$) and/or vascular endothelial growth factor (VEGF), and the expression level of prostaglandin $E_2$ ($PGE_2$) is increased by stimulating the cells with TNF-α. Since VEGF is a substance having an angiogenic effect, the pluripotent stem cell-enriched dental pulp-derived cells can promote formation of blood vessels in the body using VEGF by administering the cells to humans. Therefore, the cells can be used as a therapeutic agent for diseases for which an angiogenic effect needs be exerted. In addition, since $PGE_2$ has a strong anti-inflammatory effect, the pluripotent stem cell-enriched dental pulp-derived cells act on an inflammatory site in the body through $PGE_2$ and can suppress tissue destruction accompanied by inflammation by administering the cells to humans. Therefore, the cells can be used as an inhibitor of tissue destruction accompanied by inflammation. However, the effects of the pluripotent stem cell-enriched dental pulp-derived cells are not limited to being exerted using VEGF or $PGE_2$.

In one embodiment of the present invention, when the pluripotent stem cell-enriched dental pulp-derived cells are cultured in the presence of IFN-γ, the amount of kynurenine secreted increases. Kynurenine can suppress proliferation of T cells and can differentiate monocytes into anti-inflammatory M2 macrophages. Accordingly, the pluripotent stem cell-enriched dental pulp-derived cells can exert an anti-inflammatory effect using kynurenine by being administered to humans.

In one embodiment of the present invention, when the pluripotent stem cell-enriched dental pulp-derived cells are observed as a whole, the cells are positive for at least one or all of CD47, CD81, and CD147 and negative for at least one or all of CD19, CD34, and CD206 in expression patterns of the surface antigen markers. In addition, in one embodiment of the present invention, when the pluripotent stem cell-enriched dental pulp-derived cells are observed as a whole, the cells are positive for at least one or all of CD47, CD81, and CD147 and negative for at least one or all of CD19, CD31, CD33, CD34, CD38, CD45, CD206, CD235a, and SSEA-1 in expression patterns of the surface antigen markers.

In one embodiment of the present invention, when the pluripotent stem cell-enriched dental pulp-derived cells are observed as a whole, the cells are negative for at least one, for example, all of CD19, CD26, CD106, CD117, and CD271 in expression patterns of the surface antigen markers.

In one embodiment of the present invention, when the pluripotent stem cell-enriched dental pulp-derived cells are observed as a whole, the cells are positive for at least one, for example, all of CD140b and HLA-A, -B, and -C in expression patterns of the surface antigen markers.

In one embodiment of the present invention, when the pluripotent stem cell-enriched dental pulp-derived cells are observed as a whole, the cells are positive for at least one, for example, all of CD10, CD46, CD47, CD55, CD58, and CD59 in expression patterns of the surface antigen markers.

In one embodiment of the present invention, when the pluripotent stem cell-enriched dental pulp-derived cells are observed as a whole, the cells are, for example, positive for at least one of CD73, CD90, CD105, and CD166 and negative for at least one of CD34 and CD45 in expression patterns of the surface antigen markers.

In one embodiment of the present invention, when the pluripotent stem cell-enriched dental pulp-derived cells are observed as a whole, the cells are positive for CD73, CD90, CD105, and CD166 and negative for CD34, CD40, CD45, CD80, CD86, and MHC-class II antigen in expression patterns of the surface antigen markers. At this time, for example, the cells remain negative for CD40, CD80, and CD86 and become positive for MHC-class II antigen when the cells are stimulated with IFN-γ. The cells express prostaglandin $E_2$ and/or vascular endothelial growth factor (VEGF), and the amount of prostaglandin $E_2$ secreted is increased by stimulating the cells with TNF-α. Furthermore, the amount of kynurenine secreted is increased by stimulating the cells with IFN-γ.

When the pluripotent stem cell-enriched dental pulp-derived cells of the present invention are cultured, the cells secrete various humoral factors.

(a-8) In one embodiment, when the pluripotent stem cell-enriched dental pulp-derived cells are cultured, the cells express at least one, preferably all of MMP-2, IGFBP-4, and Cystatin C.

(a-9) In one embodiment, when the pluripotent stem cell-enriched dental pulp-derived cells are cultured, the cells express at least one, preferably all of IL-6, IL-11, MCP-1, IL-8, GROα, HGF, VEGF, VCAM-I, TIMP-3, TIMP-2, and TIMP-1.

In addition, regarding a characteristic (a-10), when the pluripotent stem cell-enriched dental pulp-derived cells are cultured, the cells express at least one, preferably all of IL-23, TNF-α, IL-18, IL-33, IL-27, TARC, ENA-78, MIP-3α, MIP-1β, IP-10, SCF, and ICAM-1.

In addition, regarding a characteristic (a-11), in one embodiment, when the pluripotent stem cell-enriched dental pulp-derived cells are cultured, the cells do not or hardly express at least one, preferably all of IL-21, Eotaxin, MIP-1α, MIG, I-TAC, and GM-CSF.

In addition, regarding a characteristic (a-12), in one embodiment, in a case where the pluripotent stem cell-enriched dental pulp-derived cells are cultured in the presence of TNF-α or IFN-γ, the expression level of IL-6 is greater than that in a case where the cells are cultured in the absence thereof.

In addition, regarding a characteristic (a-13), in one embodiment, in a case where the pluripotent stem cell-enriched dental pulp-derived cells are cultured in the presence of TNF-α or IFN-γ, the expression level of IL-11 is greater than that in a case where the cells are cultured in the absence thereof.

In addition, regarding a characteristic (a-14), in one embodiment, in a case where the pluripotent stem cell-enriched dental pulp-derived cells are cultured in the presence of TNF-α or IFN-γ, the expression level of IP-10 is greater than that in a case where the cells are cultured in the absence thereof.

In addition, regarding a characteristic (a-15), in one embodiment, in a case where the pluripotent stem cell-enriched dental pulp-derived cells are cultured in the presence of TNF-α or IFN-γ, the expression level of MCP-1 is greater than that in a case where the cells are cultured in the absence thereof.

In addition, regarding a characteristic (a-16), in one embodiment, expression of GM-CSF is induced when the pluripotent stem cell-enriched dental pulp-derived cells are cultured in the presence of TNF-α, but the expression of GM-CSF is not induced when the cells are cultured in the presence of IFN-γ.

In addition, regarding a characteristic (a-17), in one embodiment, the expression level of HGF decreases when the pluripotent stem cell-enriched dental pulp-derived cells are cultured in the presence of TNF-α compared to a case where the cells are cultured in the absence thereof, whereas the expression level of HGF increases when the cells are cultured in the presence of IFN-γ compared to a case where the cells are cultured in the absence thereof.

In addition, regarding a characteristic (a-18), in one embodiment, in a case where the pluripotent stem cell-enriched dental pulp-derived cells are cultured in the presence of TNF-α, the expression level of IL-8 is greater than that in a case where the cells are cultured in the absence thereof.

In one embodiment, the pluripotent stem cell-enriched dental pulp-derived cells of the present invention preferably have two or more, more preferably three or more, and still more preferably four or more, and still more preferably all of the characteristics shown in the above-described (a-8) to (a-18). For example, the cells having the characteristics shown in the above-described (a-8) and (a-14) or the cells having the characteristics shown in the above-described (a-8), (a-13), and (a-14) are a suitable embodiment of the present invention.

The pluripotent stem cell-enriched dental pulp-derived cells of the present invention may be mostly accounted for dental pulp-derived pluripotent stem cells. In many cases, dental pulp-derived pluripotent stem cells in flat culture are observed under an optical microscope as substantially spindle-shaped adhered cells on a cell culture plate. However, they are not always observed as spindle-shaped adhered cells depending on the culture conditions.

"Dental pulp" in the present invention refers to a loose fibrous connective tissue which fills the pulp cavity of the teeth and consists of: connective tissues containing blood vessels, nerves, and lymph vessels; and an odontoblast layer having abilities of performing deposition and repairing from the inside of dentin in a side edge portion. In addition, dental pulp can be divided into crown pulp and root pulp according to its location. Dental pulp in the present invention refers to one including at least one of crown pulp and root pulp. In addition, the animal species from which dental pulp used in the present invention is derived are not particularly limited, but are preferably mammals, more preferably primates, and still more preferably humans.

The teeth for obtaining dental pulp may be permanent teeth or deciduous teeth, or may be any of incisor teeth, canine teeth, premolar teeth, and molar teeth. The teeth are preferably healthy teeth that are not affected by tooth decay or the like. For example, extracted healthy teeth which have been extracted through a medical procedure can be suitably used in the present invention. In particular, third molar teeth can be particularly preferably used because the amount of dental pulp that can be obtained from each tooth is large and the teeth are easily available as extracted healthy teeth due to reasons such as teeth correction or the like. Here, the age of humans whose teeth are obtained is not particularly limited. However, teeth obtained from humans aged 10 to 50 are preferable, and teeth obtained from humans aged 15 to 45 are more preferable. In addition, the gender of humans whose teeth are obtained is not particularly limited.

For teeth for obtaining dental pulp, those within 72 hours after tooth extraction are preferably used, those within 24 hours after tooth extraction are more preferably used, and those within 12 hours after tooth extraction are still more preferably used. Extracted teeth are washed with a bicarbonate Ringer's solution or the like and then sterilized with a sterilant. The sterilant used at this time is not particularly limited, but is preferably one exhibiting an antibacterial effect on both gram-positive bacteria and gram-negative bacteria. For example, a 1% chlorhexidine gluconate solution is used.

Dental pulp can be obtained from an extracted tooth by exposing the dental pulp by mechanically crushing the extracted tooth. The dental pulp taken out of the extracted tooth is preferably shredded using an instrument such as scissors and then digested with a protease.

By the digestion with a protease, individual cells constituting the dental pulp are released from the dental pulp obtained from the extracted tooth. The protease used at this time preferably includes a serine protease, a metalloprotease, or a mixture thereof. However, the present invention is not limited thereto, and any protease that can release individual cells constituting dental pulp, that is, that can decompose proteinaceous cell adhesion molecules may be used.

Examples of metalloproteases suitably used at this time include a matrix metalloprotease, a neutral metalloprotease, or a mixture thereof. A collagenase, gelatinase, or a mixture thereof can be suitably used as a matrix metalloprotease. In addition, collagenase I, collagenase II, or a mixture thereof is preferable as a collagenase. Thermolysin, Dispase, or a mixture thereof can be suitably used as a neutral metalloprotease.

Examples of a preferred combination of proteases include a mixture of collagenase I, collagenase II, and thermolysin. In a case where this mixture is used as a protease, the activity of the protease in a reaction solution is preferably adjusted to 0.17 to 1.33 unit/mL. Liberase (registered trademark) (Roche) containing collagenase I, collagenase II, and thermolysin can be suitably used as a protease.

In a case where a mixed solution of collagenase type II and Dispase is used, the respective concentrations of collagenase type II and Dispase are preferably 1 to 2 mg/mL and 3,000 to 7,000 unit/mL and more preferably about 1.5 mg/mL and about 5,000 unit/mL.

The suitable reaction temperature when digesting dental pulp obtained from an extracted tooth using a protease is 35°C to 37.5°C, and a suitable reaction time is 30 minutes to 3 hours. For example, dental pulp is treated with a protease for 2 hours at 37°C. However, the reaction temperature and reaction time need to be appropriately adjusted depending on, for example, the type and concentration of protease.

Since individual cells constituting dental pulp digested with a protease are released from the dental pulp, the dental pulp can be disassembled by mechanical means such as pipetting. At this time, the protease is preferably inactivated before disassembling the dental pulp through mechanical means. The inactivation can be performed through adding components that inactivate proteases to a reaction solution, and a chelating agent such as EDTA, a cell culture medium containing FBS, and the like can be used as such components. By disassembling the dental pulp, a suspension in which cells or the like constituting the dental pulp are present in a solution in a floating state can be obtained. Pluripotent stem cells released from the dental pulp are contained in this suspension. In order to remove the protease together with a supernatant, it is preferable to centrifuge this suspension once to precipitate insoluble substances such as the cells. The precipitated cells or the like are suspended again after removing the supernatant. A cell culture medium containing FBS can be used as a solution for resuspension, for example. The suspension of the dental pulp thus obtained includes not only the cells released from the dental pulp but also tissue pieces of the dental pulp.

The dental pulp suspension obtained by suspending the disassembled dental pulp in the cell culture medium is added to a cell culture plate and cultured on the plate. The cell culture medium used for this is preferably Dulbecco's Modified Eagle's Medium to which fetal bovine serum is added. The concentration (v/v%) of fetal bovine serum added to the medium is preferably 8% to 25% and more preferably 10% to 25%, and is, for example, 20%. An example of the detailed composition of the Dulbecco's Modified Eagle's Medium (before addition of fetal bovine serum) is shown in Table 1. Appropriately, 3 to 5 mM, for example, 4 mM L-alanyl-L-glutamine can be added to the medium. In addition, an antibiotic such as streptomycin may be added to the medium as desired. In the case where streptomycin is added to the medium, the concentration thereof in the medium is set to be 10 mg/L to 250 mg/L, for example, 100 mg/L. In addition, each component can be substituted with an equivalent thereof, for example, a salt thereof.

In a case of culturing a suspension of dental pulp obtained from one extracted tooth, for example, a third molar tooth of an adult on a cell culture plate, the culture area of the plate is preferably 0.3 to 4 $cm^2$ and more preferably 0.6 to 2 $cm^2$, and is, for example, 1 $cm^2$. The amount of medium to be added to the cell culture plate is preferably 250 $\mu$L to 1 mL per 1 $cm^2$ of a culture area, for example, 500 $\mu$L. For example, a suspension of dental pulp which has been obtained from a third molar tooth of an adult and suspended in a 500 $\mu$L medium is added to a cell culture plate having a culture area of 1 $cm^2$ to culture cells.

[Table 1]

Table 1 Composition of Dulbecco's Modified Eagle's Medium

| Component | mM (Range) | mM (Suitable example) |
|---|---|---|
| Aminoacetic acid | 0.2 to 0.6 | 0.4 |
| L-Arginine hydrochloride | 0.3 to 0.5 | 0.398 |
| L-Cystine dihydrochloride | 0.15 to 0.25 | 0.201 |
| L-Glutamine | 3 to 5 | 4 |
| L-Histidine hydrochloride monohydrate | 0.15 to 0.25 | 0.2 |
| L-Isoleucine | 0.65 to 0.95 | 0.802 |
| L-Leucine | 0.65 to 0.95 | 0.802 |
| L-Lysine hydrochloride | 0.65 to 0.95 | 0.798 |
| L-Methionine | 0.15 to 0.25 | 0.201 |
| L-Phenylalanine | 0.2 to 0.6 | 0.4 |
| L-Serine | 0.2 to 0.6 | 0.4 |
| L-Threonine | 0.65 to 0.95 | 0.798 |
| L-Tryptophan | 0.06 to 0.1 | 0.0784 |
| L-Tyrosine disodium dihydrate | 0.2 to 0.6 | 0.398 |
| L-Valine | 0.65 to 0.95 | 0.803 |
| Choline chloride | 0.02 to 0.04 | 0.0286 |
| Calcium D-pantothenate | 0.007 to 0.009 | 0.00839 |
| Folic acid | 0.008 to 0.01 | 0.00907 |
| Nicotinamide | 0.03 to 0.05 | 0.0328 |
| Pyridoxine hydrochloride | 0.015 to 0.025 | 0.0196 |
| Riboflavin | 0.0008 to 0.0012 | 0.00106 |
| Thiamine hydrochloride | 0.01 to 0.014 | 0.0119 |
| i-Inositol | 0.03 to 0.05 | 0.04 |
| Calcium chloride (anhydride) | 1.5 to 2.1 | 1.8 |
| Iron(III) nitrate nonahydrate | 0.0002 to 0.0004 | 0.000248 |
| Magnesium sulfate | 0.65 to 0.95 | 0.814 |

(continued)

| Component | mM (Range) | mM (Suitable example) |
|---|---|---|
| Potassium chloride | 5 to 6 | 5.33 |
| Sodium hydrogen carbonate | 40 to 48 | 44.05 |
| Sodium chloride | 100 to 120 | 110.34 |
| Sodium dihydrogen phosphate monohydrate | 0.65 to 0.95 | 0.906 |
| D-Glucose | 5 to 7 | 5.56 |
| Phenol red | 0.03 to 0.05 | 0.0399 |
| Sodium pyruvate | 0.8 to 1.2 | 1 |

The temperature at which the dental pulp suspension is cultured on the cell culture plate is preferably 35°C to 37.5°C, for example, 37°C. The culture of the dental pulp suspension is performed until, for example, cells proliferate to form a colony having a visible size on the plate. The colony at this time forms a substantially circular shape when viewed from above, and the diameter thereof is about 1 to 3 mm. However, the present invention is not particularly limited thereto. For example, the colony may be irregular. The medium is replaced with a fresh medium during the culture of the dental pulp suspension so that the components are kept within a certain range. The medium is replaced, for example, every 1 to 6 days, 2 to 4 days, 1 day, 2 days, 3 days, or 4 days. The total amount or a part of the medium may be replaced with a fresh medium. In a case of replacing a part of the medium with a fresh medium, for example, 1/4 to 4/5, 1/4, 1/3, 1/2, or 4/5 of the amount of the old medium is removed, and a fresh medium in an equal amount is subsequently added thereto. Floating cells, tissue pieces, and the like which have been contained in the dental pulp suspension can also be removed in the process of this medium exchange.

The cells that have formed a colony on the cell culture plate through culture of the dental pulp suspension can be treated with a protease such that they can be peeled off from the plate and collected. The protease used at this time is not particularly limited as long as it can decompose proteinaceous cell adhesion molecules, but is preferably a serine protease and more preferably trypsin. In a case of using trypsin, the concentration thereof is preferably 0.1% to 0.5% (w/v) and more preferably 0.25% (w/v).

The cells collected from the above-described plate are suspended in a cell culture medium and are then added to the cell culture plate to start culture on the plate. This is called initial cell culture. The medium used at this time is preferably Dulbecco's Modified Eagle's Medium to which fetal bovine serum is added. The concentration (v/v%) of fetal bovine serum added to the medium is preferably 8% to 25% and more preferably 8% to 20%, and is, for example, 10%. An example of the detailed composition of the Dulbecco's Modified Eagle's Medium (before addition of fetal bovine serum) is shown in Table 1. Appropriately, 3 to 5 mM, for example, 4 mM L-alanyl-L-glutamine can be added to the medium. In addition, an antibiotic such as streptomycin may be added to the medium as desired. However, no antibiotic is usually added to the medium. In the case where streptomycin is added to the medium, streptomycin is added thereto so that the concentration thereof is 10 mg/L to 250 mg/L, for example, 100 mg/L. In addition, each component can be substituted with an equivalent thereof, for example, a salt thereof.

When starting the above-described initial cell culture, cells are added so that the live cell density on a cell culture plate is preferably 2,000 to 30,000 cells/cm$^2$ and more preferably 3,000 to 20,000 cells/cm$^2$, and is, for example, 3,000 cells/cm$^2$, 5,000 cells/cm$^2$, 10,000 cells/cm$^2$, or 20,000 cells/cm$^2$. The medium is replaced during the culture of the cells so that the components are kept within a certain range. At this time, the medium is replaced, for example, every 1 to 6 days, 2 to 4 days, 1 day, 2 days, 3 days, or 4 days. The total amount or a part of the medium may be replaced with a fresh medium. In a case of replacing a part of the medium with a fresh medium, for example, 1/4 to 4/5, 1/4, 1/3, 1/2, or 4/5 of the amount of the old medium is removed, and a fresh medium in an equal amount is subsequently added thereto. Floating cells or the like contained in the original cells are removed in the process of this medium exchange, and as a result, cells adhered to the cell culture plate are selected.

The initial cell culture is performed until the cells on the cell culture plate preferably become almost confluent. For example, the culture is performed until at least 85%, at least 90%, or at least 95% of the surface of the cell culture plate on which the cells can be adhered is accounted for the cells. The cells that have been cultured until the cells become almost confluent on the cell culture plate can be treated with a protease such that they can be peeled off from the plate and collected. The protease used at this time is not particularly limited as long as it can decompose proteinaceous cell adhesion molecules, but is preferably a serine protease and more preferably trypsin. In a case of using trypsin, the concentration thereof is preferably 0.1% to 0.5% (w/v) and more preferably 0.25% (w/v).

After the cells collected from the above-described plate are suspended in a cell culture medium again, the cells are added to the cell culture plate and then cultured on the plate. The medium used at this time is preferably Dulbecco's Modified Eagle's Medium to which fetal bovine serum is added. The concentration (v/v%) of fetal bovine serum added to the medium is preferably 8% to 25% and more preferably 8% to 20%, and is, for example, 10%. An example of the detailed composition of the Dulbecco's Modified Eagle's Medium (before addition of fetal bovine serum) is shown in Table 1. Appropriately, 3 to 5 mM, for example, 4 mM L-alanyl-L-glutamine may be added to the medium. In addition, an antibiotic such as streptomycin may be added to the medium as desired. However, no antibiotic is usually added to the medium. In the case where streptomycin is added to the medium, streptomycin is added thereto so that the concentration of streptomycin in the medium is 10 mg/L to 250 mg/L, for example, 100 mg/L. In addition, each component can be substituted with an equivalent thereof, for example, a salt thereof. The cell culture is performed until the cells on the cell culture plate become almost confluent. For example, the culture is performed until at least 85%, at least 90%, or at least 95% of the surface of the cell culture plate on which the cells can be adhered is accounted for the cells.

By repeating the collection of cells from the cell culture plate and the culture on the cell culture plate, it is possible to increase the number of dental pulp-derived cells. In addition, the floating cells are removed by replacing the medium during culture, and cells adhered on the cell culture plate are preferentially collected (as a result, selected). Since the dental pulp-derived pluripotent stem cells have properties of being adhered to a cell culture plate, pluripotent stem cells are enriched by repeating this process. That is, cells which have undergone the step of culturing a dental pulp suspension on a cell culture plate and the step of collecting cells from the plate after the culture are pluripotent stem cell-enriched dental pulp-derived cells. By repeating the collection of cells from a cell culture plate and the culture of the cells thereon, the pluripotent stem cells are further enriched.

The collection of cells from a cell culture plate and the culture of the cells thereon are preferably repeated 3 to 20 times, more preferably repeated 3 to 8 times, and still more preferably 4 to 8 times including the initial cell culture, and are repeated, for example, 4 times, 5 times, 6 times, 7 times, or 8 times. Most of cells that can be obtained through proliferation on a cell culture plate are pluripotent dental pulp-derived cells showing a substantially spindle-shaped form when observed with an optical microscope at the completion of the initial cell culture, but also include other cells. By repeating the collection of cells from a cell culture plate and the culture of the cells thereon 3 times, dental pulp-derived pluripotent stem cells are enriched until the cells are mostly accounted for by pluripotent dental pulp-derived cells. When the collection of cells from a cell culture plate and the culture of the cells thereon are repeated 5 times or more, dental pulp-derived pluripotent stem cells are further enriched, and cells other than the pluripotent dental pulp-derived cells showing a substantially spindle-shaped form when observed under an optical microscope are rarely observed. That is, by repeating the collection of cells from a cell culture plate and the culture of the cells thereon 5 times or more, dental pulp-derived pluripotent stem cells are substantially isolated.

The proportion of the dental pulp-derived pluripotent stem cells in all cells contained in the pluripotent stem cell-enriched dental pulp-derived cells obtained in this manner is preferably greater than or equal to 99%, more preferably greater than or equal to 99.5%, still more preferably greater than or equal to 99.9%, and still more preferably greater than or equal to 99.95%. The proportion of the dental pulp-derived pluripotent stem cells can be obtained by, for example, dividing the number of cells showing a substantially spindle-shaped form when observed under an optical microscope by the number of all cells.

The pluripotent stem cell-enriched dental pulp-derived cells obtained in this manner can be cryopreserved in a state of being suspended in a cryopreservation liquid. For cryopreservation, the average doubling time of cells on a cell culture plate is preferably within 96 hours, more preferably within 84 hours, still more preferably within 72 hours, still more preferably within 48 hours, and particularly preferably, for example, within 36 hours or 24 hours. A standard may be set for the average doubling time, and cells having an average doubling time within a certain time may be cryopreserved and used thereafter. Such a standard can be appropriately set to be, for example, within 84 hours, 72 hours, or 48 hours. Cells that do not meet these standard values can be discarded without cryopreservation to selectively preserve only cells having an ability of undergoing cell division a certain number of times or more. It can be said that as cells have a shorter average doubling time, the cell division ability increases.

The composition of a cryopreservation liquid used at this time is not particularly limited as long as mammalian cells, particularly human pluripotent stem cell-enriched dental pulp-derived cells, can be frozen and thawed without dying. The cryopreservation liquid contains a cytoprotective agent. Cytoprotective agents have, for example, a function of suppressing formation of ice crystals inside cells and destruction of the cells when the cells are frozen. Suitable examples of cytoprotective agents include dimethyl sulfoxide (DMSO), ethylene glycol, propylene glycol, sericin, and glycerol. Two or more thereof can be combined and used as a cytoprotective agent. In a case of using dimethyl sulfoxide as a cytoprotective agent, the concentration thereof is preferably 5% to 15% (v/v) and more preferably 9% to 11% (v/v), and is, for example, 10% (v/v). The cryopreservation liquid may contain a buffer agent. A buffer agent that can adjust the pH of an aqueous solution to 6 to 8, for example, 6.8 to 7.8 is preferable. Examples of such buffer agents include ones containing carbonate ions, citrate ions, and sodium ions. The cryopreservation liquid may further contain human serum albumin. In a case of using human serum albumin, the concentration thereof is preferably 40 to 100 g/L and more preferably 46 to 56 g/L, and may be, for example, 51 g/L. One obtained by adding human serum albumin solution and dimethyl sulfoxide to a bicarbonate Ringer's solution to be described below is a suitable example of a cryopreservation liquid. Both a cryopreservation liquid for intermediate cells and a cryopreservation liquid for preparation cells which are to be described below are cryopreservation liquids. In a case of providing a step of freezing intermediate cells in a production process, the former is called a first cryopreservation liquid (cryopreservation liquid for intermediate cells) and the latter is called a second cryopreservation liquid (cryopreservation liquid for preparation cells) for convenience.

Cryopreserved pluripotent stem cell-enriched dental pulp-derived cells can be used as a dental pulp-derived cell preparation to be described below or intermediate cells (which are preserved for further proliferation later). The case of cryopreserving pluripotent stem cell-enriched dental pulp-derived cells as intermediate cells will be described in detail below.

In the case of cryopreserving pluripotent stem cell-enriched dental pulp-derived cells as intermediate cells (hereinafter, referred to as "a case where cells are frozen as intermediate cells"), the collection of cells from a cell culture plate and the culture of the cells thereon are repeated preferably 3 to 8 times and more preferably 4 to 8 times, for example, 4 times, 5 times, 6 times, 7 times, or 8 times including the initial cell culture.

In addition, in the case of freezing cells as intermediate cells, the cells collected from a colony formed on a cell culture plate through culture of a dental pulp suspension are cultured on the cell culture plate until the cells are frozen, and are preferably caused to undergo at least 10 divisions and more preferably caused to undergo at least 15 divisions. For example, cells that have undergone divisions 13 to 20 times, 13 to 23 times, or 14 to 20 times are cryopreserved. Theoretically, the number of cells that have undergone 15 divisions increases by 3 x $10^4$ times or more.

In addition, in the case of freezing cells as intermediate cells, the cells are usually proliferated until the number of cells obtained from one extracted tooth (for example, a third molar tooth) becomes preferably 3 x $10^5$ or more, more preferably 1 x $10^6$ or more, and still more preferably 1 x $10^9$ or more.

In addition, for cryopreservation of cells as intermediate cells, the average doubling time of the cells on a cell culture plate is preferably within 96 hours, more preferably within 84 hours, still more preferably within 72 hours, still more preferably within 48 hours, and particularly preferably, for example, within 24 hours or 36 hours. A standard may be set for the average doubling time, and cells having an average doubling time within a certain time may be cryopreserved and used thereafter. Such a standard can be appropriately set to be, for example, within 84 hours, 72 hours, or 48 hours. Cells that do not meet these standard values can be discarded without cryopreservation to selectively preserve only cells having an ability of undergoing cell division a certain number of times or more. It can be said that as cells have a shorter average doubling time, the ability of cell division increases.

In the case of freezing cells as intermediate cells, the pluripotent stem cell-enriched dental pulp-derived cells which have been peeled off and collected from a cell culture plate through a treatment with a protease are washed with a washing solution before cryopreservation. The washing solution used at this time is for sufficiently removing a medium and the protease, and the composition thereof is not particularly limited. Physiological saline, phosphate-buffered physiological saline, a Ringer's acetate solution, a bicarbonate Ringer's solution, or the like can be used as the washing solution, but a solution obtained by adding human serum albumin to a bicarbonate Ringer's solution is preferably used. Commercially available Ringer's acetate solutions and bicarbonate Ringer's solutions can be used. For example, PLASMA-LYTE (registered trademark) A (Baxter) and Physio (registered trademark) 140 (Otsuka Holdings Co., Ltd.) can be used as Ringer's acetate solutions, and BICARBON (Registered trademark) Injection (AJINOMOTO CO., INC.) can be used as a bicarbonate Ringer's solution. Suitable examples of the componential composition and the electrolyte concentration of a bicarbonate Ringer's solution are respectively shown in Tables 2 and 3.

[Table 2]

Table 2 Componential composition of bicarbonate Ringer's solution

| Component | mM (Range) | mM (Suitable example) |
|---|---|---|
| Sodium chloride | 94.5 to 116 | 105 |
| Potassium chloride | 3.62 to 4.42 | 4.02 |
| Calcium chloride dihydrochloride | 1.35 to 1.65 | 1.5 |
| Magnesium chloride hexahydrate | 0.45 to 0.55 | 0.5 |
| Sodium hydrogen carbonate | 22.5 to 27.5 | 25 |
| Sodium citrate dihydrate | 1.5 to 1.84 | 1.67 |

(continued)

| Component | mM (Range) | mM (Suitable example) |
|---|---|---|
| Osmotic pressure ratio of cryopreservation liquid for intermediate cells to physiological saline at pH of 6.8 to 7.8 being 0.9 to 1.0 | | |

[Table 3]

Table 3 Concentration of electrolytes contained in bicarbonate Ringer's solution

| Electrolyte | mEq/L (Range) | mEq/L (Suitable example) |
|---|---|---|
| $Na^{+}$ | 122 to 149 | 135 |
| $K^{+}$ | 3.6 to 4.4 | 4 |
| $Ca^{2+}$ | 2.7 to 3.3 | 3 |
| $Mg^{2+}$ | 0.9 to 1.1 | 1 |
| $Cl^{-}$ | 102 to 124 | 113 |
| $HCO_3^{-}$ | 22.5 to 27.5 | 25 |
| Citrate$^{3-}$ | 4.5 to 5.5 | 5 |

In addition, a suitable example of the componential composition of the washing solution obtained by adding human serum albumin to a bicarbonate Ringer's solution is shown in Table 4. In addition, a suitable example of the concentration of electrolytes contained in the washing solution obtained by adding human serum albumin to a bicarbonate Ringer's solution is shown in Table 5. Sodium acetyltryptophan and sodium caprylate can be excluded from the components of the washing solution shown in Tables 4 and 5.

[Table 4]

Table 4 Componential composition of washing solution

| Component | mM (Range) | mM (Suitable example) |
|---|---|---|
| Sodium chloride | 90 to 110 | 100 |
| Potassium chloride | 3.45 to 4.21 | 3.83 |
| Calsium chloride dihydrochloride | 1.29 to 1.57 | 1.43 |
| Magnesium chloride hexahydrate | 0.429 to 0.525 | 0.477 |
| Sodium hydrogen carbonate | 21.4 to 26.2 | 23.8 |
| Sodium citrate dihydrate | 1.43 to 1.75 | 1.59 |
| Human serum albumin | (10.7 to 13.1) | (11.9) |
| Sodium acetyltryptophan | 0.870 to 1.06 | 0.967 |
| Sodium caprylate | 0.874 to 1.07 | 0.971 |
| (Note) In the table, the unit of the concentration of human serum albumin is g/L. | | |

[Table 5]

Table 5 Concentration of electrolytes contained in washing solution (excluding albumin)

| Component | mM (Range) | mM (Suitable example) |
|---|---|---|
| $Na^+$ | 117.5 to 143.6 | 130.5 |
| $K^+$ | 3.45 to 4.21 | 3.83 |
| $Ca^{2+}$ | 2.57 to 3.15 | 2.86 |
| $Mg^{2+}$ | 0.86 to 1.05 | 0.95 |
| $Cl^-$ | 96.8 to 118 | 107.6 |
| $HCO_3^-$ | 21.4 to 26.2 | 23.8 |
| $Citrate^{3-}$ | 4.28 to 5.24 | 4.76 |
| Sodium acetyltryptophan | 0.870 to 1.06 | 0.967 |
| Sodium caprylate | 0.874 to 1.07 | 0.971 |

In the case of freezing cells as intermediate cells, the pluripotent stem cell-enriched dental pulp-derived cells after being washed with a washing solution are once collected through a method such as centrifugation. The collected cells are cryopreserved in a suspension state (intermediate cell suspension). However, the intermediate cell suspension can be prepared by changing the composition of the solution, in which the cells are suspended, without collecting cells using an ultrafiltration membrane or the like and can be cryopreserved. The composition of the solution portion (cryopreservation liquid for intermediate cells) of the intermediate cell suspension is not particularly limited as long as mammalian cells, particularly human pluripotent stem cell-enriched dental pulp-derived cells, can be frozen and thawed without dying. The cryopreservation liquid for intermediate cells contains a cytoprotective agent. Cytoprotective agents have, for example, a function of suppressing formation of ice crystals inside cells and destruction of the cells when the cells are frozen. Suitable examples of cytoprotective agents include dimethyl sulfoxide (DMSO), ethylene glycol, propylene glycol, sericin, and glycerol. Two or more thereof can be combined and used as a cytoprotective agent. In a case of using dimethyl sulfoxide as a cytoprotective agent, the concentration thereof is preferably 5% to 15% (v/v) and more preferably 9% to 11% (v/v), and is, for example, 10% (v/v). The cryopreservation liquid may contain a buffer agent. A buffer agent that can adjust the pH of an aqueous solution to 6 to 8, for example, 6.8 to 7.8 is preferable. Examples of such buffer agents include ones containing carbonate ions, bicarbonate ions, citrate ions, and sodium ions. The cryopreservation liquid for intermediate cells may further contain human serum albumin. In a case of using human serum albumin, the concentration thereof is preferably 40 to 100 g/L and more preferably 46 to 56 g/L, and may be, for example, 51 g/L.

One obtained by adding human serum albumin solution and dimethyl sulfoxide to a bicarbonate Ringer's solution is a suitable example of a cryopreservation liquid for intermediate cells. The cryopreservation liquid for intermediate cells contains 59 to 80.4 mM sodium chloride, 2.3 to 3.08 mM potassium chloride, 0.85 to 1.16 mM calcium chloride dihydrate, 0.28 to 0.385 mM magnesium chloride hexahydrate, 14 to 19.2 mM sodium hydrogen carbonate, 0.94 to 1.28 mM sodium citrate dihydrate, 46 to 56 g/L human serum albumin, 3.73 to 4.55 mM sodium acetyltryptophan, 3.74 to 4.58 mM sodium caprylate, and 9% to 11% (v/v) DMSO. A suitable example of the composition of the cryopreservation liquid for intermediate cells is shown in Table 6. That is, the composition of the cryopreservation liquid for intermediate cells is substantially 65.8 to 80.4 mM sodium chloride, 2.52 to 3.08 mM potassium chloride, 0.95 to 1.16 mM calcium chloride dihydrate, 0.315 to 0.385 mM magnesium chloride hexahydrate, 15.7 to 19.2 mM sodium hydrogen carbonate, 1.04 to 1.28 mM sodium citrate dihydrate, 46 to 56 g/L human serum albumin, 3.73 to 4.55 mM sodium acetyltryptophan, 3.74 to 4.58 mM sodium caprylate, and 9% to 11% (v/v) DMSO. Among these, sodium acetyltryptophan and sodium caprylate can be excluded. The osmotic pressure ratio of the cryopreservation liquid for intermediate cells to physiological saline is preferably 0.9 to 1.1.

In addition, a solution containing sodium ions, potassium ions, calcium ions, magnesium ions, hydrogen carbonate ions, citrate ions, human serum albumin, and dimethyl sulfoxide can be suitably used as the cryopreservation liquid for intermediate cells. For example, a solution containing sodium ions, potassium ions, calcium ions, magnesium ions, hydrogen carbonate ions, citrate ions, human serum albumin, and dimethyl sulfoxide respectively at concentrations of 91 to 113 mM, 2.52 to 3.08 mM, 0.95 to 1.16 mM, 0.315 to 0.385 mM, 15.6 to 19.2 mM, 1.04 to 1.28 mM, 46 to 56 g/L, and 9% to 11% (v/v) is a suitable example thereof. In addition, for example, a solution containing sodium ions, potassium ions, calcium ions, magnesium ions, hydrogen carbonate ions, citrate ions, human serum albumin, and dimethyl sulfoxide respectively at concentrations of 100 to 102 mM, 2.71 to 2.77 mM, 1.01 to 1.03 mM, 0.335 to 0.345 mM, 16.9 to 17.2 mM, 1.13 to 1.15 mM, 52.2 to 54.3 g/L, and 10.3% to 10.9% (v/v) is another suitable example thereof.

Furthermore, for example, a solution containing sodium ions, potassium ions, calcium ions, magnesium ions, hydrogen carbonate ions, citrate ions, human serum albumin, and dimethyl sulfoxide respectively at concentrations of 102 mM, 2.80 mM, 1.05 mM, 0.35 mM, 17.4 mM, 1.16 mM, 51 g/L, and 10% (v/v) is still another suitable example thereof. Furthermore, for example, a solution containing sodium ions, potassium ions, calcium ions, magnesium ions, hydrogen carbonate ions, citrate ions, human serum albumin, and dimethyl sulfoxide respectively at concentrations of 101 mM, 2.77 mM, 1.03 mM, 0.34 mM, 17.2 mM, 1.15 mM, 52 g/L, and 10% (v/v) is still another suitable example thereof. In a case where the cryopreservation liquid for intermediate cells further contains acetyltryptophan or a salt thereof, the concentration thereof is preferably 3.73 to 4.55 mM, or 4.24 to 4.41 mM, and is, for example, 4.14 mM or 4.24 mM. In a case where the cryopreservation liquid for intermediate cells further contains caprylic acid or a salt thereof, the concentration thereof is preferably 3.74 to 4.58 mM, or 4.25 to 4.43 mM, and is, for example, 4.16 mM or 4.25 mM.

[Table 6]

Table 6 Componential composition of cryopreservation liquid for intermediate cells

| Component | mM (Suitable range 1) | mM (Suitable range 2) | mM (Suitable example 1) | mM (Suitable example 2) |
|---|---|---|---|---|
| Sodium chloride | 65.8 to 80.4 | 70.8 to 72.3 | 73.1 | 72.3 |
| Potassium chloride | 2.52 to 3.08 | 2.71 to 2.77 | 2.80 | 2.77 |
| Calcium chloride dihydrochloride | 0.95 to 1.16 | 1.01 to 1.03 | 1.05 | 1.03 |
| Magnesium chloride hexahydrate | 0.315 to 0.385 | 0.335 to 0.345 | 0.35 | 0.34 |
| Sodium hydrogen carbonate | 15.67 to 19.15 | 16.9 to 17.2 | 17.41 | 17.2 |
| Sodium citrate dihydrate | 1.04 to 1.28 | 1.13 to 1.15 | 1.16 | 1.15 |
| Human serum albumin | (46 to 56) | (52.2 to 54.3) | (51) | (52.2) |
| Sodium acetyltryptophan | 3.73 to 4.55 | 4.24 to 4.41 | 4.14 | 4.24 |
| Sodium caprylate | 3.74 to 4.58 | 4.25 to 4.43 | 4.16 | 4.25 |
| DMSO | (9 to 11) | (10.3 to 10.9) | (10) | (10) |
| (Note) Unit of concentration of human serum albumin is g/L, and unit of concentration of DMSO is % (v/v) | | | | |

In the case of freezing cells as intermediate cells, the cell density in the suspension in which the pluripotent stem cell-enriched dental pulp-derived cells are suspended in the cryopreservation liquid for intermediate cells is not particularly limited, but is preferably $2 \times 10^6$ to $8 \times 10^7$ cells/mL, more preferably $4 \times 10^6$ to $3 \times 10^7$ cells/mL, and still more preferably $8 \times 10^6$ to $2 \times 10^7$ cells/mL, and is, for example, $1.1 \times 10^7$ cells/mL. The pluripotent stem cell-enriched dental pulp-derived cells suspended in the cryopreservation liquid for intermediate cells are dispensed into a cell cryopreservation container, and are then cryopreserved.

At this time, the amount of cell suspension to be dispensed into one cell cryopreservation container needs to be appropriately adjusted depending on applications or the like, and is preferably 1 to 20 mL. In addition, the number of cells to be dispensed into one cell cryopreservation container is preferably $5 \times 10^6$ to $9.2 \times 10^8$.

The container used as the cell cryopreservation container is not particularly limited as long as the material exhibits durability at low temperatures and the container is not damaged even if the contents are frozen and thawed. Commercially available containers can also be used as the cryopreservation containers. For example, cryotubes made of glass and plastic (for example, made of a polyolefin resin) can be suitably used as the cell cryopreservation containers. Here, a vial of which the material is particularly borosilicate glass can be suitably used as the container made of glass. In addition, here, the polyolefin resin includes polyethylene, high density polyethylene, low density polyethylene, polypropylene, a copolymer composed of ethylene and propylene, and thermoplastic elastomer. The freezing of cells is preferably performed by dispensing a cell suspension into a cell cryopreservation container, and then gradually lowering the temperature. For example, a method for lowering the temperature at a rate of 1°C per minute up to -6°C and rapidly freezing the cells thereafter can be employed as the method for freezing cells. The cells are preserved in a frozen state.

The temperature at which the cells are cryopreserved is preferably lower than or equal to -130°C, more preferably lower than or equal to -170°C, and still more preferably lower than or equal to -180°C. For example, the cells can be preserved in a state in which the cell cryopreservation container is immersed in liquid nitrogen (boiling point: -196°C).

Such cells cryopreserved as intermediate cells are thawed when in use to be used for the next step for producing a dental pulp-derived cell preparation.

Providing a step of temporarily preserving proliferated cells as intermediate cells in the process of producing pluripotent stem cell-enriched dental pulp-derived cells is extremely significant in terms of process management of pluripotent stem cell-enriched dental pulp-derived cells. For example, in a case where a desired number of extracted teeth cannot be obtained at one time, if culture of dental pulp-derived cells starts at all times using obtained extracted teeth as a raw material to produce final products, the lot sizes of the final products are small and the lot management thereof becomes complicated. However, if intermediate cells are produced and preserved, once the intermediate cells are accumulated to a certain amount, the next production process can be started with these intermediate cells as one batch. Therefore, it is possible to increase the lot size of final products, and lot management becomes easier.

In addition, by providing the step of preserving cells as intermediate cells, it is possible to perform quality tests for properties or the like of the cells at an intermediate stage of the production process. Accordingly, only when cells obtained as intermediate cells meet desired quality standards, the cells can be used for the next production process. That is, cells that do not meet desired quality standards at, before, or after the completion of the production process of intermediate cells can be excluded. Accordingly, the cells that do not meet desired quality standards are not brought into subsequent steps. Therefore, it is possible to increase probability of producing final products that meet desired quality standards. That is, it is possible to increase the yield at the time of production and to reduce production costs.

Cryopreservation of cells in the production process usually involves a risk of a change in properties of the cells before and after freezing of the cells. In addition, cell proliferation abilities of cells sometimes deteriorate after the cryopreservation. In contrast, in the cells obtained as intermediate cells in the present invention, neither a change in properties of cells before and after freezing nor deterioration in cell proliferation abilities after cryopreservation is recognized.

The pluripotent stem cell-enriched dental pulp-derived cells to be preserved as intermediate cells preferably have a high proportion of live cells contained in the cells. The proportion (cell viability) of live cells before cryopreservation is preferably greater than or equal to 50%, more preferably greater than or equal to 60%, and still more preferably greater than or equal to 70%, and is preferably, for example, greater than or equal to 80%, greater than or equal to 90%, or greater than or equal to 95%. It is also possible to preserve only cells having a certain value or more of a cell viability before cryopreservation as intermediate cells according to appropriately set standards.

Pluripotent stem cell-enriched dental pulp-derived cells to be preserved as intermediate cells have preferably undergone at least 10 cell divisions in an in vitro environment. For example, the cells have undergone at least 15, 16, or 17 cell divisions. The average cell division time during the cell division period is preferably within 48 hours.

In the present invention, causing cell division in an in vitro environment means that, for example, cell division is caused in a state where cells are added to a culture container such as a cell culture flask together with a cell culture medium. The culture temperature at this time is preferably 34°C to 38°C and more preferably 36°C to 38°C, and is, for example, 37°C. In addition, the cells are preferably cultured in a moist environment in the presence of 5% $CO_2$. The cell culture medium used at this time is not particularly limited as long as it can be used for culturing mammalian cells, and is, for example, Dulbecco's Modified Eagle's Medium (DMEM) containing fetal bovine serum (FBS). The concentration of FBS contained in the medium is preferably 2% to 20% and more preferably 5% to 20%, and still more preferably 8% to 15%, and is, for example, 10%. In addition, the concentration of glucose contained in DMEM is 5 to 20 mM, for example, 5 mM. Examples of suitable culture conditions include a culture temperature of 37°C, a moist environment in the presence of 5% $CO_2$, and DMEM containing about 5 mM glucose and 10% FBS as a medium. A method for culturing dental pulp-derived cells described in Example 5 (that is, on a cell culture plate, a DMEM medium which contains 5.56 mM glucose and to which 10% FBS is added, in the presence of 5% $CO_2$, and 37°C) is an example of a suitable method for causing cell division in an in vitro environment.

Subculture of cells in a case of causing cell division in an in vitro environment is performed such that the cells are peeled off from a culture container, and are then seeded in a new culture container so that 1/20 to 1/3 of the bottom area of the culture container is covered with the cells. In a case where at least 70%, at least 80%, or at least 90% of the bottom area of the culture container is covered with cells due to cell division, the subculture is repeated.

In one embodiment, when the pluripotent stem cell-enriched dental pulp-derived cells to be preserved as intermediate cells are cultured in a medium containing a substance known to induce differentiation into chondrocytes, an increase in expression level of aggrecan is observed as a whole. In addition, when the pluripotent stem cell-enriched dental pulp-derived cells to be preserved as intermediate cells are cultured in a medium containing a substance known to induce differentiation into osteocytes, an increase in the amount of calcium accumulated in the cells is observed as a whole. That is, the pluripotent stem cell-enriched dental pulp-derived cells have abilities to differentiate into chondrocytes and osteocytes when observed as a whole.

In one embodiment, when the pluripotent stem cell-enriched dental pulp-derived cells to be preserved as intermediate cells are observed as a whole, the cells are positive for at least one of CD73, CD90, CD105, and CD166 and negative for at least one of CD34 and CD45 in expression patterns of the surface antigen markers. For example, when the cells are observed as a whole, the cells are positive for CD73 and CD90 and negative for CD34 in the expression patterns of the surface antigen markers. In addition, for example, when the cells are observed as a whole, the cells are positive for CD73, CD90, CD105, and CD166 and negative for CD34 and CD45 in the expression patterns of the surface antigen markers.

In one embodiment, when the pluripotent stem cell-enriched dental pulp-derived cells to be preserved as intermediate cells are observed as a whole, the cells are, for example, negative for at least one of CD40, CD80, CD86, and MHC-class II antigen in expression patterns of the surface antigen markers. For example, when the cells are observed as a whole, the cells are negative for CD40, CD80, CD86, and MHC-class II antigen in the expression patterns of the surface antigen markers. It is known that, when cells positive for these surface antigen markers are transplanted into allogeneic individuals, the cells tend to be recognized as an antigen to be excluded from a living body. In a case where cells are negative for at least one of these surface antigen markers, this means that the pluripotent stem cell-enriched dental pulp-derived cells to be preserved as intermediate cells have a property of low immunogenicity to that extent and hardly tend to be excluded from a living body when transplanted into allogeneic individuals. In addition, the cells may remain negative for CD40, CD80, and CD86 and be positive for MHC-class II antigen in the expression patterns of the surface antigen markers when the cells are stimulated with IFN-$\gamma$. For example, the cells remain negative for CD40, CD80, and CD86 and are positive for MHC-class II antigen in the expression patterns of the surface antigen markers when the cells are stimulated with IFN-$\gamma$.

In one embodiment, when the pluripotent stem cell-enriched dental pulp-derived cells to be preserved as intermediate cells are observed as a whole, the cells are, for example, positive for CD73, CD90, CD105, and CD166 and negative for CD34, CD40, CD45, CD80, CD86, and MHC-class II antigen in expression patterns of the surface antigen markers. In addition, the cells remain negative for CD40, CD80, and CD86 and become positive for MHC-class II antigen when the cells are stimulated with IFN-$\gamma$.

In one embodiment, regarding a characteristic (b-1), when the pluripotent stem cell-enriched dental pulp-derived cells to be preserved as intermediate cells are observed as a whole, the cells are positive for at least one, preferably all of CD47, CD81, CD90, CD147, and HLA-A, -B, and -C in expression patterns of the surface antigen markers.

Here, when the individual cells included in all of the cells are observed, preferably at least 80%, more preferably at least 90%, and still more preferably at least 95% of the observed cells are positive for these antigens.

In addition, in one embodiment, regarding a characteristic (b-2), when the pluripotent stem cell-enriched dental pulp-derived cells to be preserved as intermediate cells are observed as a whole, the cells are positive for at least one, preferably all of CD29, CD46, CD55, CD59, CD73, and CD140b in expression patterns of the surface antigen markers.

Here, when the individual cells included in all of the cells are observed, preferably at least 70%, more preferably at least 80%, and still more preferably at least 90% of the observed cells are positive for these antigens.

In addition, in one embodiment, regarding a characteristic (b-3), when the pluripotent stem cell-enriched dental pulp-derived cells to be preserved as intermediate cells are observed as a whole, the cells are positive for at least one, preferably all of CD9, CD44, CD49b, CD49c, CD98, and EGF-R in expression patterns of the surface antigen markers.

Here, when the individual cells included in all of the cells are observed, preferably at least 65%, more preferably at least 75%, and still more preferably at least 80% of the observed cells are positive for these antigens.

In addition, in one embodiment, regarding a characteristic (b-4), when the pluripotent stem cell-enriched dental pulp-derived cells to be preserved as intermediate cells are observed as a whole, the cells are positive for at least one, preferably all of CD49f and CD166 in expression patterns of the surface antigen markers.

Here, when the individual cells included in all of the cells are observed, preferably at least 60%, more preferably at least 70%, and still more preferably at least 75% of the observed cells are positive for these antigens.

In addition, in one embodiment, regarding a characteristic (b-5), when the pluripotent stem cell-enriched dental pulp-derived cells to be preserved as intermediate cells are observed as a whole, the cells are positive for at least one, preferably all of CD10, CD13, CD58, CD63, CD105, CD151, and CD164 in expression patterns of the surface antigen markers.

Here, when the individual cells included in all of the cells are observed, preferably at least 60%, more preferably at least 65%, and still more preferably at least 70% of the observed cells are positive for these antigens.

In one embodiment, the pluripotent stem cell-enriched dental pulp-derived cells to be preserved as intermediate cells preferably have two or more, more preferably three or more, and still more preferably all of the characteristics shown in the above-described (b-1), (b-2), (b-3), (b-4) and (b-5). For example, the cells having the characteristics shown in the above-described (b-1) and (b-2) are a suitable embodiment of the present invention.

In one embodiment, regarding a characteristic (b-6), when the pluripotent stem cell-enriched dental pulp-derived cells to be preserved as intermediate cells are observed as a whole, the cells are negative for at least one, preferably all of CD120b, CD132, CD158a, CD161, CD184, CD195, CD206, CD210, CD212, CD226, CD244, CD267, CD278, CD279, CD282, CD294, NKB1, SSEA-1, TRA-1-60, TRA-1-81, Vβ23, SSEA-3, CLA, and integrin β7 in expression patterns of the surface antigen markers.

Here, when the individual cells included in all of the cells are observed, preferably only at most 10%, more preferably only at most 5%, and still more preferably only at most 2%, and still more preferably only at most 1% of the observed cells are positive for these antigens.

In one embodiment, regarding a characteristic (b-7), when the pluripotent stem cell-enriched dental pulp-derived cells to be preserved as intermediate cells are observed as a whole, the cells are negative for at least one, preferably all of CD8b, CD11b, CD15s, CD16, CD19, CD24, CD31, CD32, CD62E, CD62P, CD66f, CD86, CD88, CD94, CD100, CD103, CD104, CD114, CD117, CD118, CD121b, CD122, CD123, CD124, CD126, CD127, CD128b, CD135, CD137, CD137 ligand, CD150, CD163, CD172b, CD177, CD178, CD180, CD197, CD220, CD229, CD231, CD255, CD268, CD305, CD314, CD321, CDw327, CDw328, CD329, CD335, CD336, BLTR-1, CLIP, CMRF-44, CMRF-56, fMLP-R, Vβ8, Invariant NKT, and γδ TCR in expression patterns of the surface antigen markers.

Here, when the individual cells included in all of the cells are observed, preferably only at most 10%, more preferably only at most 5%, and still more preferably only at most 2% of the observed cells are positive for these antigens.

In one embodiment, regarding a characteristic (b-8), when the pluripotent stem cell-enriched dental pulp-derived cells to be preserved as intermediate cells are observed as a whole, the cells are negative for at least one, preferably all of CD1a, CD1b, CD1d, CD2, CD3, CD5, CD6, CD7, CD8a, CD11c, CD15, CD18, CD21, CD22, CD23, CD25, CD26, CD27, CD28, CD33, CD34, CD35, CD37, CD38, CD40, CD41a, CD41b, CD42b, CD45, CD45RB, CD45RO, CD48, CD50, CD53, CD62L, CD64, CD66 (a, c, d, e), CD69, CD70, CD72, CD74, CD80, CD84, CD85, CD87, CD89, CDw93, CD97, CD106, CD134, CD138, CD141, CD144, CD154, CD158b, CD162, CD183, CD205, CD235a, CD271, CD309, CD326, CD337, αβ TCR, and MHC-class II antigen in expression patterns of the surface antigen markers.

Here, when the individual cells included in all of the cells are observed, preferably only at most 10% and more preferably only at most 5% of the observed cells are positive for these antigens.

In one embodiment, the pluripotent stem cell-enriched dental pulp-derived cells to be preserved as intermediate cells preferably have two or more, more preferably three or more, and still more preferably all of the characteristics shown in the above-described (b-1), (b-2), (b-3), (b-4), (b-5), (b-6), (b-7), and (b-8). For example, the cells having the characteristics shown in the above-described (b-1) and (b-6) are a suitable embodiment of the present invention.

In one embodiment, when the pluripotent stem cell-enriched dental pulp-derived cells to be preserved as intermediate cells are observed as a whole, the cells express, for example, prostaglandin $E_2$ ($PGE_2$) and/or vascular endothelial growth factor (VEGF), and the expression level of prostaglandin $E_2$ ($PGE_2$) is increased by stimulating the cells with TNF-α.

In one embodiment of the present invention, when the pluripotent stem cell-enriched dental pulp-derived cells to be preserved as intermediate cells are cultured in the presence of IFN-γ, the amount of kynurenine secreted increases.

In one embodiment of the present invention, when the pluripotent stem cell-enriched dental pulp-derived cells to be preserved as intermediate cells are observed as a whole, the cells are positive for at least one or all of CD47, CD81, and CD147 and negative for at least one or all of CD19, CD34, and CD206 in expression patterns of the surface antigen markers. In addition, in another embodiment, cells are positive for at least one or all of CD47, CD81, and CD147 and negative for at least one or all of CD19, CD31, CD33, CD34, CD38, CD45, CD206, CD235a, and SSEA-1 in expression patterns of the surface antigen markers.

In one embodiment of the present invention, when the pluripotent stem cell-enriched dental pulp-derived cells to be preserved as intermediate cells are observed as a whole, the cells are, for example, positive for at least one of CD73, CD90, CD105, and CD166 and negative for at least one of CD34 and CD45 in expression patterns of the surface antigen markers.

In one embodiment, when the pluripotent stem cell-enriched dental pulp-derived cells to be preserved as intermediate cells are observed as a whole, the cells are positive for CD73, CD90, CD105, and CD166 and negative for CD34, CD40, CD45, CD80, CD86, and MHC-class II antigen in expression patterns of the surface antigen markers. At this time, for example, the cells remain negative for CD40, CD80, and CD86 and become positive for MHC-class II antigen when the cells are stimulated with IFN-γ. In addition, the cells express prostaglandin $E_2$ ($PGE_2$) and/or vascular endothelial growth factor (VEGF), and the amount of prostaglandin $E_2$ ($PGE_2$) secreted is increased by stimulating the cells with TNF-α.

In one embodiment, when the pluripotent stem cell-enriched dental pulp-derived cells to be preserved as intermediate cells are observed as a whole, the expression level of aggrecan increases in a case where the cells are cultured in a medium containing a substance known to induce differentiation into chondrocytes. In addition, in a case where the cells are cultured in a medium containing a substance known to induce differentiation into osteocytes, the amount of calcium accumulated in the cells increases. That is, the pluripotent stem cell-enriched dental pulp-derived cells have abilities to differentiate into chondrocytes and osteocytes when observed as a whole.

A method in which only cells showing specific expression patterns are preserved as intermediate cells according to appropriately set standards based on the above-described expression patterns of the surface antigen markers shown by the pluripotent stem cell-enriched dental pulp-derived cells of the present invention and/or other gene expression patterns can also be used to manage the production process.

The pluripotent stem cell-enriched dental pulp-derived cells to be preserved as intermediate cells may be mostly accounted for pluripotent stem cells (dental pulp-derived pluripotent stem cells) derived from dental pulp. These are observed as substantially spindle-shaped adhered cells in flat culture under an optical microscope, and the proportion of substantially spindle-shaped cells in all cells is preferably greater than or equal to 99%, more preferably greater than or equal to 99.5%, still more preferably greater than or equal to 99.9%, and still more preferably greater than or equal to 99.95%. The proportion of the dental pulp-derived pluripotent stem cells can be obtained by dividing the number of cells showing a substantially spindle-shaped form when observed under an optical microscope by the number of all cells. A method in which only cell groups having a certain value or more of a proportion of substantially spindle-shaped cells are preserved as intermediate cells according to appropriately set standards based on the above can also be used to manage the production process.

In one embodiment, when the pluripotent stem cell-enriched dental pulp-derived cells to be preserved as intermediate cells are observed as a whole, the cells preferably have an ability to differentiate into chondrocytes and an ability to differentiate into osteocytes. That is, in a case where the intermediate cells are cultured in a medium containing a substance known to induce differentiation into chondrocytes, the expression level of aggrecan increases. In addition, when the intermediate cells are observed as a whole, in a case where the cells are cultured in a medium containing a substance known to induce differentiation into osteocytes, the amount of calcium accumulated in the cells increases. The fact that the intermediate cells have an ability to differentiate into chondrocytes and an ability to differentiate into osteocytes can be examined respectively through methods described in Examples 20 and 19. Only cell groups shown to have an ability to differentiate into chondrocytes and an ability to differentiate into osteocytes through these methods can be regarded as intermediate cells to be preserved. However, the methods for examining the differentiation abilities are not limited to the methods described in Examples 20 and 19. Only cell groups shown to have an ability to differentiate into chondrocytes and an ability to differentiate into osteocytes through other appropriate methods can also be regarded as intermediate cells to be preserved.

In one embodiment, the pluripotent stem cell-enriched dental pulp-derived cells to be preserved as intermediate cells may be the same as or have substantially the same properties as intermediate cells used for a further culture step which will be described in detail below.

The properties of cells cryopreserved as intermediate cells are preferably retained before and after thawing. That is, when pluripotent stem cell-enriched dental pulp-derived cells which have been cryopreserved as intermediate cells are thawed, the viability, expression patterns of surface antigen markers, cell morphology, and the differentiation ability of the cells are preferably substantially the same as those of cells before thawing. Only cells with properties retained before and after thawing can be used for a subsequent culture step as intermediate cells by checking the properties of the intermediate cells after thawing or when cultured after thawing.

The viability of intermediate cells, to be used for a further culture step, after thawing is preferably greater than or equal to 50%, more preferably greater than or equal to 60%, and still more preferably greater than or equal to 70%, and is preferably, for example, greater than or equal to 80%, greater than or equal to 90%, or greater than or equal to 95%. It is possible to use only intermediate cells having a certain value or more of viability after thawing for a subsequent culture step according to appropriately set standards. The cell viability after thawing is substantially 80% to 98% or 85% to 95%.

When the cells cryopreserved as intermediate cells are cultured after thawing, the cells preferably have an ability of undergoing at least 10 cell divisions and more preferably have an ability of undergoing at least 15 cell divisions, and have, for example, an ability of undergoing at least 14 cell divisions. In addition, when the cells cryopreserved as intermediate cells are cultured after thawing, the average doubling time of the cells on a cell culture plate is preferably within 96 hours, more preferably within 84 hours, still more preferably 72 hours, still more preferably 48 hours, and still more preferably within 36 hours. For example, standards can be set for the cell division ability and the average doubling time, and only cells which have an ability of undergoing cell division a certain number of times or more and of which the average doubling time is within a certain time can be used for subsequent use. Such standards can be appropriately set. For example, cells having an ability of undergoing at least 10 cell divisions and an average doubling time within 96 hours, cells having an ability of undergoing at least 14 cell divisions and an average doubling time within 72 hours, and cells having an ability of undergoing at least 15 cell divisions and an average doubling time within 72 hours. Only intermediate cells having an ability of undergoing cell division a certain number of times or more can be used for a subsequent culture step by not using cells that do not meet these standards. The culture conditions in this step may be the same as or equivalent to those described above for the pluripotent stem cell-enriched dental pulp-derived cells.

Cells to be cryopreserved as intermediate cells have preferably undergone at least 10 cell divisions in an in vitro environment. For example, the cells have undergone at least 15, 16, or 17 cell divisions. The average cell division time during the cell division period is preferably within 48 hours.

When intermediate cells to be used for a further culture step are cultured in a medium containing a substance known to induce differentiation into chondrocytes, an increase in expression level of aggrecan is observed as a whole. In addition, when the pluripotent stem cell-enriched dental pulp-derived cells of the present invention are cultured in a medium containing a substance known to induce differentiation into osteocytes, an increase in the amount of calcium accumulated in the cells is observed as a whole. The cells have abilities to differentiate into chondrocytes and osteocytes when observed as a whole.

In one embodiment, when the intermediate cells to be used for a further culture step are observed as a whole, the cells are positive for at least one of CD73, CD90, CD105, and CD166 and negative for at least one of CD34 and CD45 in expression patterns of the surface antigen markers after the cells are thawed or when the cells are cultured after thawing. For example, the cells are positive for CD73 and CD90 and negative for CD34. In addition, for example, the cells are positive for CD73 and CD90 and negative for CD34. The cells are positive for CD73, CD90, CD105, and CD166 and negative for CD34 and CD45.

In addition, in one embodiment, when the intermediate cells to be used for a further culture step are observed as a whole, the cells are negative for at least one of CD40, CD80, CD86, and MHC-class II antigen in expression patterns of the surface antigen markers immediately after the cells are thawed or when the cells are cultured after thawing. For example, the cells are negative for CD40, CD80, CD86, and MHC-class II antigen. It is known that, when cells positive for these surface antigen markers are transplanted into allogeneic individuals, the cells tend to be recognized as an antigen to be excluded from a living body. In a case where cells are negative for these surface antigen markers, this means that intermediate cells to be used for a further culture step are hypoimmunogenic to that extent and tend to be hardly excluded from a living body when transplanted into allogeneic individuals. In addition, the cells may remain negative for CD40, CD80, and CD86 and be positive for MHC-class II antigen in the expression patterns of the surface antigen markers when the cells are stimulated with IFN-$\gamma$. For example, the cells remain negative for CD40, CD80, and CD86 and are positive for MHC-class II antigen in the expression patterns of the surface antigen markers when the cells are stimulated with IFN-$\gamma$.

In one embodiment, when the intermediate cells to be used for a further culture step are observed as a whole immediately after thawing or when cultured after thawing, the cells are positive for CD73, CD90, CD105, and CD166 and negative for CD34, CD40, CD45, CD80, CD86, and MHC-class II antigen in expression patterns of the surface antigen markers. In addition, the cells remain negative for CD40, CD80, and CD86 and become positive for MHC-class II antigen when the cells are stimulated with IFN-$\gamma$.

In one embodiment, regarding a characteristic (c-1), when the intermediate cells to be used for a further culture step are observed as a whole, the cells are positive for at least one, preferably all of CD47, CD81, CD90, CD147, and HLA-A, -B, and -C in expression patterns of the surface antigen markers.

Here, when the individual cells included in all of the cells are observed, preferably at least 80%, more preferably at least 90%, and still more preferably at least 95% of the observed cells are positive for these antigens.

In one embodiment, regarding a characteristic (c-2), when the intermediate cells to be used for a further culture step are observed as a whole, the cells are positive for at least one, preferably all of CD29, CD46, CD55, CD59, CD73, and CD140b in expression patterns of the surface antigen markers.

Here, when the individual cells included in all of the cells are observed, preferably at least 70%, more preferably at least 80%, and still more preferably at least 90% of the observed cells are positive for these antigens.

In one embodiment, regarding a characteristic (c-3), when the intermediate cells to be used for a further culture step are observed as a whole, the cells are positive for at least one, preferably all of CD9, CD44, CD49b, CD49c, CD98, and EGF-R in expression patterns of the surface antigen markers.

Here, when the individual cells included in all of the cells are observed, preferably at least 65%, more preferably at least 75%, and still more preferably at least 80% of the observed cells are positive for these antigens.

In one embodiment, regarding a characteristic (c-4), when the intermediate cells to be used for a further culture step are observed as a whole, the cells are positive for at least one, preferably all of CD49f and CD166 in expression patterns of the surface antigen markers.

Here, when the individual cells included in all of the cells are observed, preferably at least 60%, more preferably at least 70%, and still more preferably at least 75% of the observed cells are positive for these antigens.

In addition, in one embodiment, regarding a characteristic (c-5), when the pluripotent stem cell-enriched dental pulp-derived cells to be preserved as intermediate cells are observed as a whole, the cells are positive for at least one, preferably all of CD10, CD13, CD58, CD63, CD105, CD151, and CD164 in expression patterns of the surface antigen markers.

Here, when the individual cells included in all of the cells are observed, preferably at least 60%, more preferably at least 65%, and still more preferably at least 70% of the observed cells are positive for these antigens.

In one embodiment, the intermediate cells to be used for a further culture step preferably have two or more, more preferably three or more, and still more preferably all of the characteristics shown in the above-described (c-1), (c-2), (c-3), (a-4), and (c-5). For example, the cells having the characteristics shown in the above-described (c-1) and (c-2) are a suitable embodiment of the present invention.

In one embodiment, regarding a characteristic (c-6), when the intermediate cells to be used for a further culture step are observed as a whole, the cells are negative for at least one, preferably all of CD120b, CD132, CD158a, CD161, CD184, CD195, CD206, CD210, CD212, CD226, CD244, CD267, CD278, CD279, CD282, CD294, NKB1, SSEA-1, TRA-1-60, TRA-1-81, Vβ23, SSEA-3, CLA, and integrin β7 in expression patterns of the surface antigen markers.

Here, when the individual cells included in all of the cells are observed, preferably at most 10%, more preferably at most 5%, and still more preferably at most 2%, and still more preferably at most 1% of the observed cells are positive for these antigens.

In one embodiment, regarding a characteristic (c-7), when the intermediate cells to be used for a further culture step are observed as a whole, the cells are negative for at least one, preferably all of CD8b, CD11b, CD15s, CD16, CD19, CD24, CD31, CD32, CD62E, CD62P, CD66f, CD86, CD88, CD94, CD100, CD103, CD104, CD114, CD117, CD118, CD121b, CD122, CD123, CD124, CD126, CD127, CD128b, CD135, CD137, CD137ligand, CD150, CD163, CD172b, CD177, CD178, CD180, CD197, CD220, CD229, CD231, CD255, CD268, CD305, CD314, CD321, CDw327, CDw328, CD329, CD335, CD336, BLTR-1, CLIP, CMRF-44, CMRF-56, fMLP-R, Vβ8, Invariant NKT, and γδ TCR in expression patterns of the surface antigen markers.

Here, when the individual cells included in all of the cells are observed, preferably at most 10%, more preferably at most 5%, and still more preferably at most 2% of the observed cells are positive for these antigens.

In one embodiment, regarding a characteristic (c-8), when the intermediate cells to be used for a further culture step are observed as a whole, the cells are negative for at least one, preferably all of CD1a, CD1b, CD1d, CD2, CD3, CD5, CD6, CD7, CD8a, CD11c, CD15, CD18, CD21, CD22, CD23, CD25, CD26, CD27, CD28, CD33, CD34, CD35, CD37, CD38, CD40, CD41a, CD41b, CD42b, CD45, CD45RB, CD45RO, CD48, CD50, CD53, CD62L, CD64, CD66 (a, c, d, e), CD69, CD70, CD72, CD74, CD80, CD84, CD85, CD87, CD89, CDw93, CD97, CD106, CD134, CD138, CD141, CD144, CD154, CD158b, CD162, CD183, CD205, CD235a, CD271, CD309, CD326, CD337, αβ TCR, and MHC-class II antigen in expression patterns of the surface antigen markers.

Here, when the individual cells included in all of the cells are observed, preferably at most 10% and more preferably at most 5% of the observed cells are positive for these antigens.

In one embodiment, the intermediate cells to be used for a further culture step preferably have two or more, more preferably three or more, and still more preferably all of the characteristics shown in the above-described (c-1), (c-2), (c-3), (a-4), (c-5), (c-6), (c-7), and (c-8). For example, the cells having the characteristics shown in the above-described (c-1) and (c-6) are a suitable embodiment of the present invention.

In addition, when the intermediate cells to be used for a further culture step are observed as a whole, the cells express, for example, prostaglandin $E_2$ ($PGE_2$) and/or vascular endothelial growth factor (VEGF), and the expression level of prostaglandin $E_2$ ($PGE_2$) is increased by stimulating the cells with TNF-α.

In one embodiment of the present invention, when the intermediate cells to be used for a further culture step are cultured in the presence of IFN-γ, the amount of kynurenine secreted increases.

In one embodiment of the present invention, when the intermediate cells to be used for a further culture step are observed as a whole, the cells are positive for at least one or all of CD47, CD81, and CD147 and negative for at least one or all of CD19, CD34, and CD206 in expression patterns of the surface antigen markers. In addition, in one embodiment of the present invention, when the pluripotent stem cell-enriched dental pulp-derived cells are observed as a whole, the cells are positive for at least one or all of CD47, CD81, and CD147 and negative for at least one or all of CD19, CD31, CD33, CD34, CD38, CD45, CD206, CD235a, and SSEA-1 in expression patterns of the surface antigen markers.

In one embodiment, when the intermediate cells to be used for a further culture step are observed as a whole, the cells are negative for at least one, for example, all of CD19, CD26, CD106, CD117, and CD271 in expression patterns of the surface antigen markers.

In one embodiment, when the intermediate cells to be used for a further culture step are observed as a whole, the cells are positive for at least one, for example, all of CD140b and HLA-A, -B, and -C in expression patterns of the surface antigen markers.

In one embodiment of the present invention, when the intermediate cells to be used for a further culture step are observed as a whole, the cells are positive for at least one, for example, all of CD46, CD47, CD55, CD58, and CD59 in expression patterns of the surface antigen markers.

In one embodiment of the present invention, when the intermediate cells to be used for a further culture step are observed as a whole, the cells are, for example, positive for at least one of CD73, CD90, CD105, and CD166 and negative for at least one of CD34, and CD45 in expression patterns of the surface antigen markers.

In addition, in one embodiment, when the intermediate cells to be used for a further culture step are observed as a whole, the cells are positive for CD73, CD90, CD105, and CD166 and negative for CD34, CD40, CD45, CD80, CD86, and MHC-class II antigen in expression patterns of the surface antigen markers. At this time, the cells remain negative for CD40, CD80, and CD86 and become positive for MHC-class II antigen when the cells are stimulated with IFN-γ. In addition, the cells express prostaglandin $E_2$ ($PGE_2$) and/or vascular endothelial growth factor (VEGF), and the

amount of prostaglandin $E_2$ ($PGE_2$) secreted is increased by stimulating the cells with TNF-α. Furthermore, the amount of kynurenine secreted is increased by stimulating the cells with IFN-γ.

When the cells contained in the dental pulp-derived cell preparations are cultured, the cells secrete various humoral factors.

In one embodiment, regarding a characteristic (c-9), when the cells contained in dental pulp-derived cell preparations are cultured, the cells express at least one, preferably all of MMP-2, IGFBP-4, and Cystatin C.

In one embodiment, regarding a characteristic (c-10), when the intermediate cells to be used for a further culture step are cultured, the cells express at least one, preferably all of IL-6, IL-11, MCP-1, IL-8, GROα, HGF, VEGF, VCAM-1, TIMP-3, TIMP-2, and TIMP-1.

In addition, regarding a characteristic (c-11), in one embodiment, when the intermediate cells to be used for a further culture step are cultured, the cells express at least one, preferably all of IL-23, TNF-α, IL-18, IL-33, IL-27, TARC, ENA-78, MIP-3α, MIP-1β, IP-10, SCF, and ICAM-1.

In addition, regarding a characteristic (c-12), in one embodiment, when the intermediate cells to be used for a further culture steps are cultured, the cells do not or hardly express at least one, preferably all of IL-21, IFN-α, Eotaxin, MIP-1α, MIG, I-TAC, and GM-CSF.

In addition, regarding a characteristic (c-13), in one embodiment, in a case where the intermediate cells to be used for a further culture step are cultured in the presence of TNF-α or IFN-γ, the expression level of IL-6 is greater than that in a case where the cells are cultured in the absence thereof.

In addition, regarding a characteristic (c-14), in one embodiment, in a case where the intermediate cells to be used for a further culture step are cultured in the presence of TNF-α or IFN-γ, the expression level of IL-11 is greater than that in a case where the cells are cultured in the absence thereof.

In addition, regarding a characteristic (c-15), in one embodiment, in a case where the intermediate cells to be used for a further culture step are cultured in the presence of TNF-α or IFN-γ, the expression level of IP-10 is greater than that in a case where the cells are cultured in the absence thereof.

In addition, regarding a characteristic (c-16), in one embodiment, in a case where the intermediate cells to be used for a further culture step are cultured in the presence of TNF-α or IFN-γ, the expression level of MCP-1 is greater than that in a case where the cells are cultured in the absence thereof.

In addition, regarding a characteristic (c-17), in one embodiment, expression of GM-CSF is induced when the intermediate cells to be used for a further culture step are cultured in the presence of TNF-α, but the expression of GM-CSF is not induced when the cells are cultured in the presence of IFN-γ.

In addition, regarding a characteristic (c-18), in one embodiment, the expression level of HGF decreases when the intermediate cells to be used for a further culture step are cultured in the presence of TNF-α compared to a case where the cells are cultured in the absence thereof, whereas the expression level of HGF increases when the cells are cultured in the presence of IFN-γ compared to a case where the cells are cultured in the absence thereof.

In addition, regarding a characteristic (c-19), in one embodiment, in a case where the intermediate cells to be used for a further culture step are cultured in the presence of TNF-α, the expression level of IL-8 is greater than that in a case where the cells are cultured in the absence thereof.

In one embodiment, the intermediate cells to be used for a further culture step preferably have two or more, more preferably three or more, still more preferably four or more, and still more preferably all of the characteristics shown in the above-described (c-9) to (c-19). For example, the cells having the characteristics shown in the above-described (c-9) and (a-15) and the cells having the characteristics shown in the above-described (c-9), (c-14), and (c-15) are a suitable embodiment of the present invention.

In addition, when the intermediate cells to be used for a further culture step are observed as a whole immediately after thawing or when cultured after thawing, the cells have an ability to differentiate into chondrocytes and an ability to differentiate into osteocytes. That is, the expression level of aggrecan increases in a case where the cells are cultured in a medium containing a substance known to induce differentiation into chondrocytes, and the amount of calcium accumulated in the cells increases in a case where the cells are cultured in a medium containing a substance known to induce differentiation into osteocytes. The fact that the intermediate cells have an ability to differentiate into chondrocytes and an ability to differentiate into osteocytes can be examined respectively through methods described in Examples 20 and 19. Only cell groups shown to have an ability to differentiate into chondrocytes and an ability to differentiate into osteocytes through these methods can be regarded as intermediate cells to be used for a further culture step. However, the methods for examining these differentiation abilities are not limited to the methods described in the examples. Only cell groups shown to have an ability to differentiate into chondrocytes and an ability to differentiate into osteocytes through other appropriate methods can also be used as intermediate cells in a subsequent culture step.

A method in which only cells showing specific expression patterns are regarded as intermediate cells to be used for a further culture step according to appropriately set standards based on the above-described expression patterns of the surface antigen markers shown by the pluripotent stem cell-enriched dental pulp-derived cells of the present invention and/or other gene expression patterns can also be used to manage the production process.

In addition, when the intermediate cells to be used for a further culture step are observed as a whole immediately after thawing or when cultured after thawing, the cells are mostly accounted for dental pulp-derived pluripotent stem cells. These are observed as substantially spindle-shaped adhered cells in flat culture under an optical microscope, and the proportion of substantially spindle-shaped cells in all cells is preferably greater than or equal to 99%, more preferably greater than or equal to 99.5%, still more preferably greater than or equal to 99.9%, and still more preferably greater than or equal to 99.95%. Only cell groups having a certain value or more of a proportion of substantially spindle-shaped cells can be used as intermediate cells in a subsequent culture step according to appropriately set standards based on the above.

In addition, when the intermediate cells to be used for a further culture step are cultured after thawing, the cells preferably have an ability of undergoing at least 10 cell divisions and more preferably have an ability of undergoing at least 15 cell divisions, and have, for example, an ability of undergoing at least 14 cell divisions. In addition, the average doubling time of the intermediate cells to be used for a further culture step when these are thawed and cultured on a cell culture plate is preferably within 96 hours, more preferably within 84 hours, still more preferably within 72 hours, still more preferably within 48 hours, and still more preferably within 36 hours. Culture conditions of the cells at this time which are the same as or equivalent to those of the above-described pluripotent stem cell-enriched dental pulp-derived cells, for example, conditions (that is, on a cell culture plate, a DMEM medium which contains 5.56 mM glucose and to which 10% FBS is added, in the presence of 5% $CO_2$, and 37°C) described in Example 5 can be used. Only cell groups having an ability of undergoing cell division a certain number of times or more can be used as intermediate cells in a subsequent culture step according to appropriately set standards based on the above. In addition, only cell groups having an average doubling time within a certain time can be used as intermediate cells in a subsequent culture step according to appropriately set standards. Such standards are, for example, cells having an ability of undergoing at least 10 cell divisions and an average doubling time within 96 hours, cells having an ability of undergoing at least 14 cell divisions and an average doubling time within 72 hours, and cells having an ability of undergoing at least 15 cell divisions and an average doubling time within 72 hours.

That is, the intermediate cells to be used for a further culture step retain properties of stem cells which have a high division ability and can be differentiated into two or more cells. The intermediate cells to be used for a further culture step can also be called cells having a high colony-forming ability. Here, the colony-forming ability refers to an ability of dividing cells on a plate to form colonies when the cells are seeded in the plate and cultured.

When the intermediate cells are thawed, an average diameter of the cells in a state in which the cells are made to float in a solution, for example, a medium is preferably less than or equal to 20 μm or less than or equal to 18 μm, and is, for example, 10 to 20 μm, 10 to 18 μ, 12 to 20 μm, 14 to 20 μ, and 16 to 20 μm.

Items of quality tests carried out at, before, or after completion of a production process of intermediate cells are exemplified below as Quality Tests a to k. The quality tests are performed on one or more items thereof. All of these items may be carried out. Cells to be used for the quality tests at this time are either cells (1), as intermediate cells, obtained by fractionating some cells before being suspended in a cryopreservation liquid, cells (2), as intermediate cells, obtained by fractionating some cells before being suspended in a cryopreservation liquid and further subculturing these fractionated cells, cells (3), as intermediate cells, before freezing which have been suspended in a cryopreservation liquid for freezing, cells (4), as intermediate cells, immediately after thawing frozen cells, or cells (5), as intermediate cells, obtained by thawing frozen cells and further culturing the thawed cells. Unless otherwise specified, the same quality test may be performed on two or more of these cells (1) to (5).

(Quality Test a) Verifying that a proportion of the number of cells showing a substantially spindle-shaped form in all cells when observed under an optical microscope is greater than or equal to 99%, greater than or equal to 99.5%, greater than or equal to 99.9%, or greater than or equal to 99.95,
(Quality Test b) Verifying that a cell viability is greater than or equal to 50%, greater than or equal to 60%, greater than or equal to 70%, greater than or equal to 80%, greater than or equal to 90%, or greater than or equal to 95%,
(Quality Test c) Verifying that cells are positive for CD73, CD90, CD105, and CD166 and negative for CD34 and CD45 in expression patterns of the cell surface antigen markers, or verifies that cells are positive for at least one of CD73 and CD90 and negative for CD34 in expression patterns of the cell surface antigen markers,
(Quality Test d) Verifying that cells are negative for CD40, CD80, CD86, and MHC-class II antigen in expression patterns of the cell surface antigen markers, or verifies that cells are negative for at least CD40, CD80, CD86, and MHC-class II antigen in expression patterns of the cell surface antigen markers,
(Quality Test e) Verifying that cells remain negative for CD40, CD80, and CD86 and become positive for MHC-class II antigen in expression patterns of the cell surface antigen markers when the cells are stimulated with IFN-γ, or verifies at least one that cells are negative for CD40, negative for CD80, negative for CD86, and positive for MHC-class II antigen in expression patterns of the cell surface antigen markers when the cells are stimulated with IFN-γ,
(Quality Test f) Verifying that cells express prostaglandin $E_2$ ($PGE_2$) and/or vascular endothelial growth factor (VEGF) and an expression level of prostaglandin $E_2$ ($PGE_2$) is increased by stimulating the cells with TNF-α,

(Quality Test g) Verifying that an expression level of aggrecan increases when cells are cultured in a medium containing a substance known to induce differentiation into chondrocytes,
(Quality Test h) Verifying that an amount of calcium accumulated in cells increases when the cells are cultured in a medium containing a substance known to induce differentiation into osteocytes,
(Quality Test i) Verifying that cells have an ability of undergoing at least 10, 14, or 15 cell divisions on a cell culture plate,
(Quality Test j) Verifying that an average doubling time of cells on a cell culture plate is within 96 hours, 84 hours, 72 hours, 48 hours, or 36 hours during a period of Quality Test i.
(Quality Test k) Verifying that an average diameter of cells in a state where the cells are made to float in a medium is less than or equal to 20 μm, less than or equal to 18 μm, 10 to 20 μm, 10 to 20 μm, 10 to 18 μm, 12 to 20 μm, 14 to 20 μm, or 16 to 20 μm.

The above-described quality tests may be carried out on 10 items (Quality Test a) to (Quality Test j). In addition, arbitrary 9 items may be selected among these 10 items to carry out the quality tests. In the case where 9 items are selected to carry out the quality tests, for example, Quality Tests a, b, c, d, e, f, g, i, and j may be selected, but there is no limitation. In addition, arbitrary 8 items may be selected among these 10 items to carry out the quality tests. In the case where 8 items are selected to carry out the quality tests, for example, Quality Tests a, b, c, d, e, f, i, and j may be selected, but there is no limitation. In addition, arbitrary 7 items may be selected among these 10 items to carry out the quality tests. In the case where 7 items are selected to carry out the quality tests, for example, Quality Tests a, b, c, d, e, i, and j may be selected, but there is no limitation. In addition, arbitrary 6 items may be selected among these 10 items to carry out the quality tests. In the case where 6 items are selected to carry out the quality tests, for example, Quality Tests a, b, c, d, i, and j may be selected, but there is no limitation. In addition, arbitrary 5 items may be selected among these 10 items to carry out the quality tests. In the case where 5 items are selected to carry out the quality tests, for example, Quality Tests a, b, c, i, and j may be selected, but there is no limitation. In addition, arbitrary 4 items may be selected among these 10 items to carry out the quality tests. In the case where 4 items are selected to carry out the quality tests, for example, Quality Tests a, b, c, and j may be selected, but there is no limitation. In addition, arbitrary 3 items may be selected among these 10 items to carry out the quality tests. In the case where 3 items are selected to carry out the quality tests, for example, Quality Tests a, b, and c may be selected, but there is no limitation. In addition, arbitrary 2 items may be selected among these 10 items to carry out the quality tests. In the case where 2 items are selected to carry out the quality tests, for example, Quality Tests a and b, Quality Tests a and c, Quality Tests a and d, Quality Tests a and e, Quality Tests a and f, Quality Tests a and g, Quality Tests a and h, Quality Tests a and i, Quality Tests a and j, Quality Tests b and c, Quality Tests b and d, Quality Tests b and e, Quality Tests b and f, Quality Tests b and g, Quality Tests b and h, Quality Tests b and i, Quality Tests b and j, Quality Tests c and d, Quality Tests c and e, Quality Tests c and f, Quality Tests c and g, Quality Tests c and h, Quality Tests c and i, Quality Tests c and j, Quality Tests d and e, Quality Tests d and f, Quality Tests d and g, Quality Tests d and h, Quality Tests d and i, Quality Tests d and j, Quality Tests e and f, Quality Tests e and g, Quality Tests e and h, Quality Tests e and i, Quality Tests e and j, Quality Tests f and i, Quality Tests f and j, or Quality Tests i and j may be selected, but there is no limitation.

[Production Culture]

A further culture step performed on intermediate cells is a culture step leading to production of pluripotent stem cell-enriched dental pulp-derived cells which are final products, and this step is particularly called production culture.
A medium used for production culture is preferably Dulbecco's Modified Eagle's Medium to which fetal bovine serum is added. The concentration (v/v%) of fetal bovine serum added to the medium is preferably 8% to 25% and more preferably 8% to 20%, and is, for example, 10%. An example of the detailed composition of the Dulbecco's Modified Eagle's Medium (before addition of fetal bovine serum) is shown in Table 1. As desired, 3 to 5 mM, for example, 4 mM L-alanyl-L-glutamine may be added to the medium. In addition, no antibiotic is usually added thereto in production culture. However, an antibiotic such as streptomycin can be added to the medium. In the case where streptomycin is added to the medium, streptomycin is added thereto so that the concentration of streptomycin in the medium is 10 mg/L to 250 mg/L, for example, 100 mg/L. In addition, each component can be substituted with an equivalent thereof, for example, a salt thereof.
Production culture of cells may be performed on a cell culture plate, or may be performed on microcarriers in a cell culture device, for example, in a bioreactor. In addition, cells may be cultured and proliferated on a cell culture plate to further perform the production culture on microcarriers in a cell culture device, for example, in a bioreactor. In addition, in the case of culturing cells on microcarriers, a shaking flask can also be used instead of a bioreactor. In the case of culturing cells on a cell culture plate in the production culture, the conditions are the same as or similar to the culture conditions of the above-described cells obtained by culturing a dental pulp suspension. That is, culture of intermediate cells after thawing on a cell culture plate and collection of the cells therefrom are repeated to increase the number of cells. Bioreactors commercially available from GE Healthcare, Merck KGaA, Sartorius AG, and the like can be suitably used. Hereinafter, a method for culturing cells using a bioreactor as a cell culture device will be particularly described in detail.

In a case of culturing cells on microcarriers using a bioreactor in production culture, the microcarriers to which the cells are adhered are placed in the bioreactor, and the cells are cultured while stirring the microcarriers in a medium. Microcarriers are particles (small particles) of a material to which cells can adhere, and are used for culturing cells on surfaces thereof (including surfaces inside pores in a case of porous microcarriers). In that sense, microcarriers can also be referred to as carrier particles or small carrier particles. The diameters (particle diameters) of microcarriers during cell culture (that is, during hydration) are preferably 50 to 400 μm, and are, for example, 80 to 300 μm, 80 to 250 μm, 100 to 200 μm, 130 to 180 μm. Microcarriers may be porous having pores inward from the particle surface. The diameters (pore diameters) of porous microcarriers during cell culture are preferably 3 to 40 μm, and are, for example, 5 to 25 μm, 10 to 20 μm, 5 to 15 μm.

The materials of microcarriers are not particularly limited, and any material can be used as long as cells can be cultured in a state of being adhered to its surface. Examples of microcarriers include ones obtained by molding a polymer compound into a particle shape, ceramic particles, and glass particles. Examples of such a polymer compound include a vinyl resin, a urethane resin, an epoxy resin, polystyrene, polymethyl methacrylate polyester, polyamides, polyimide, a silicone resin, a phenol resin, a melamine resin, a urea resin, an aniline resin, an ionomer resin, polycarbonate, collagen, dextran, gelatin, cellulose, alginate, and mixtures thereof. Surfaces of microcarriers may be further modified with cell adhesion molecules or the like. Examples of such cell adhesion molecules include gelatin, keratin, elastin, heparan sulfate, dextran, dextran sulfate, chondroitin sulfate, sodium hyaluronate, n-isopropylacrylamide, collagen I to XIX, fibronectin, vitronectin, laminin-1 to -12, nidogen, tenascin, thrombospondin, osteopontin, fibrinogen, poly-D-lysine, poly-L-lysine, chitin, chitosan, sepharose, laminin, entactin I, or fragments or peptides containing cell adhesion domains of these cell adhesion molecules, and mixtures thereof. In addition, the specific gravity of microcarriers to a medium is preferably close to 1, or 0.9 to 1.2, and is, for example, 0.9 to 1.1 or about 1.0.

Microcarriers which are made of a gelatin matrix and have particle diameters during cell culture of 130 to 180 μm and pore diameters of 10 to 20 μm (or 5 to 15 μm) can be suitably used.

Adhesion of cells to microcarriers in production culture is performed by mixing the cells with microcarriers while stirring them in a solution. This step is called an adhesion step of cells to microcarriers. In the adhesion step, cells in contact with microcarriers during stirring are sequentially adhered to surfaces of the microcarriers. A solution used in this step is preferably a cell culture medium, but the present invention is not particularly limited thereto. The stirring of the cells and the microcarriers in the adhesion step may be performed intermittently. In this case, the cells and the microcarriers are, for example, stirred for 1 minute to 20 minutes and are then allowed to stand for 10 minutes to 2 hours.

In a case of performing an adhesion step using a cell culture medium as a solution, the step can be performed in a bioreactor. In the case of performing the adhesion step in a bioreactor, a cell culture medium, cells and microcarriers are placed and stirred in the bioreactor to make the cells to adhere to the microcarriers. After the adhesion of the cells, the cell culture can be continued as it is in the bioreactor. The stirring of the cells and the microcarriers in the bioreactor in the adhesion step may be performed intermittently. In this case, the cells and the microcarriers are, for example, stirred for 1 minute to 20 minutes and are then allowed to stand for 10 minutes to 2 hours.

In general, the bioreactor refers to a biochemical reactor such as an immobilized enzyme reaction device and a biological or microbiological reactor such as a fermentation tank or a biological wastewater treatment device. A bioreactor used in examples to be described below in the present invention is suitable for making pluripotent stem cell-enriched dental pulp-derived cells to adhere to microcarriers, and culturing the cells while performing stirring in a medium. The bioreactor has a culture tank for culturing cells, a stirrer for stirring a medium, a gas-phase inlet, liquid-phase inlet, a drain port, and a sensor unit. The culture tank is a tank having a substantially cylindrical cavity. In this state, it is possible to culture pluripotent stem cell-enriched dental pulp-derived cells to which microcarriers are adhered in a culture tank. A stirrer for a medium in a culture tank is provided in the cavity of the culture tank. The stirrer is, for example, an impeller, and a medium is stirred by rotation of the impeller. The stirring of a medium with an impeller is adjusted so that most of microcarrier to which cells are adhered float in a medium without being precipitated on the bottom surface of the cavity of a culture tank. The rotational speed of an impeller needs to be appropriately selected depending on the shape of a culture tank, the shape of the impeller, the types of microcarriers, or the like, and is, for example, 30 to 100 rpm, 50 to 80 rpm, or about 70 rpm. The gas-phase inlet is for sending gas phases such as air, oxygen, and carbon dioxide into a culture tank. The gas-phase inlet may have an outlet under a liquid level of a medium. In this case, a gas phase is sent to the medium as bubbles. The liquid-phase inlet is for charging various solutions such as a medium or a glucose solution into a culture tank. The drain port is for discharging a medium in a culture tank. A medium is discharged from this drain port during medium exchange. The drain port is also used for taking out microcarriers, to which cells are adhered, together with the medium. This drain port can be used when sampling microcarriers during culture or when collecting microcarriers after the completion of culture. The sensor unit includes sensors that measure the temperature, pH, and concentration of dissolved oxygen of a medium in a culture tank. The sensor unit can further include sensors that measure the concentrations of glucose and lactic acids. The sensors for measuring the temperature, pH, and concentration of dissolved oxygen of a solution, and the concentrations of glucose and lactic acids can be appropriately selected from well-known ones for installation.

A culture tank of a bioreactor is fixed or removably installed in the bioreactor. By installing a culture tank removably, washing of the culture tank becomes easy. Culture tanks are usually made of metal or a hard resin, and are washed after use and repeatedly used. Instead of such a culture tank, a flexible resin culture bag can be set in a bioreactor and a medium and cells are placed in the bag to perform culture. The culture bag is supported in the bioreactor so that it can be handled similarly to a usual culture tank. Since the culture bag is disposable, no washing operation after use is required, whereby the work efficiency during culture can be improved.

In a case where the concentration of glucose in a medium in production culture falls below a certain value, the medium is supplemented with glucose. For example, in a case where the concentration of glucose is lower than or equal to 0.1 mM, the medium is supplemented with glucose so that the final concentration of glucose becomes 5 to 7 mM. In addition, in a case where the concentration of lactic acids exceeds a certain value in production culture, the entire or a part of the medium is replaced. In a case where the concentration of lactic acids is, for example, higher than or equal to 20 mM, the entire medium is replaced or a part of the medium is replaced so that the concentration of lactic acids becomes, for example, lower than or equal to 5 mM or lower than or equal to 2 mM.

Oxygen, air, and $CO_2$ are supplied to a medium through a gas-phase inlet during culture in a bioreactor. The amount of oxygen supplied is controlled so that the concentration of dissolved oxygen in a medium becomes 50% to 100% of the saturated concentration, for example, so that the concentration of dissolved oxygen is maintained at 50% or 100% thereof. In addition, regarding the flow rate of $CO_2$, $CO_2$ is supplied so that the pH of a medium is maintained, for example, at 7.0 to 7.8 so that cells can suitably grow. The concentration of dissolved oxygen and the pH are monitored over time. Therefore, when the monitored numerical values deviate from set values, the concentration of dissolved oxygen and the pH can be kept constant by reducing the amount of oxygen and $CO_2$ supplied accordingly. In addition, additional microcarriers can be supplied into a bioreactor as cells proliferate.

The culture in a bioreactor is performed until the surfaces of microcarriers are accounted for cells up to a certain proportion. The proportion at this time can be appropriately set, for example, to 20%, 40%, 85%, 95%, or 98%. In addition, the culture in a bioreactor is performed until the surfaces of microcarriers are accounted for cells until the cell density on the surfaces thereof becomes a certain value. The cell density at this time can be appropriately set, for example, to 3,000 cells/$cm^2$, 5,000 cells/$cm^2$, 10,000 cells/$cm^2$, 20,000 cells/$cm^2$, or 22,000 cells/$cm^2$.

Cells to be frozen as a dental pulp-derived cell preparation after the completion of the production culture are preferably caused to undergo at least 15 divisions and more preferably caused to undergo at least 20 divisions after being collected as cells forming a colony on a cell culture plate through culture of a dental pulp suspension. For example, cells that have undergone divisions 15 to 30 times, 15 to 23 times, 15 to 20 times, 20 to 30 times, 20 to 28 times, or 23 to 26 times are cryopreserved.

Cells to be frozen as a dental pulp-derived cell preparation after the completion of the production culture through the intermediate cells are preferably caused to undergo at least 10 divisions and more preferably caused to undergo at least 15 divisions after being collected as cells forming a colony on a cell culture plate through culture of a dental pulp suspension until the cells are regarded as intermediate cells. For example, cells that have undergone divisions 13 to 20 times, 13 to 23 times, or 14 to 20 times are cryopreserved as intermediate cells. Subsequently, in the production culture which is started by thawing intermediate cells, cells are preferably caused to undergo at least 5 divisions until a dental pulp-derived cell preparation is obtained from the start of the production culture, and are preferably caused to undergo, for example, at least 8 times or at least 10 times. In particular, when performing culture using microcarriers in the production culture, cells are preferably caused to undergo 2 to 5 divisions, and are preferably caused to undergo, for example, 2 to 3 divisions.

In a case where cells are cultured on a cell culture plate, the average doubling time of the cells when the cells are cultured and caused to undergo division until a dental pulp-derived cell preparation is obtained from intermediate cells is preferably within 96 hours, more preferably within 84 hours, still more preferably within 72 hours, still more preferably within 48 hours, and still more preferably within 36 hours. In a case where cells are cultured using microcarriers, the average doubling time of the cells when the cells are cultured and caused to undergo division until a dental pulp-derived cell preparation is obtained from intermediate cells is preferably within 8 days, more preferably within 6 days, still more preferably within 5 days, and still more preferably within 4 days, and is, for example, 2 to 8 days, 2 to 7 days, 2 to 6 days, 2.5 to 6 days, or 2.5 to 5.5 days.

A standard can be set for the average doubling time of cells during the entire production culture period or during culture using microcarriers, and only cells having an average doubling time within the standard can be cryopreserved to be used for subsequent use. Such standards can be appropriately set, for example, to be within 84 hours, within 72 hours, within 48 hours, or within 36 hours in the case of culture on a cell culture plate, and to be within 8 days, within 6 days, within 5 days, within 4 days, within 3 days, or the like in the case of culture using microcarriers. Cells that do not meet these standard values can be discarded without cryopreservation to selectively preserve only cells having an ability of undergoing cell division a certain number of times or more as a dental pulp-derived cell preparation.

In the production culture, cells are proliferated until the number of pluripotent stem cells that can be obtained from one extracted tooth (for example, a third molar tooth) until a dental pulp-derived cell preparation is obtained becomes preferably $1 \times 10^6$ or more, more preferably $1 \times 10^7$ or more, still more preferably $3 \times 10^7$ or more, still more preferably $1 \times 10^8$ or more, and still more preferably $1 \times 10^9$ or more, and becomes, for example, $1 \times 10^7$ to $1 \times 10^{10}$. Theoretically, the number of cells that have undergone 20 divisions, 25 divisions, and 30 divisions increases by 1 x $10^6$ times or more, 3 x $10^7$ times or more, and 1 x $10^9$ times or more.

[Production of Preparation]

Microcarriers after the completion of the production culture are collected and washed in a state where cells are adhered thereto. A solution that can be used as a washing liquid for washing microcarriers at this time is not particularly limited as long as it does not damage cells and does not inhibit an enzyme activity of a serine protease and a metalloprotease, especially trypsin. However, physiological saline, PBS, a DMEM Low Glucose medium (containing no serum), and the washing solution having the composition shown in Table 4 are suitably used, and a DMEM Low Glucose medium (containing no serum) can be used, for example.

The washed microcarriers are treated with a protease. The protease that can be used at this time is not particularly limited as long as it can decompose proteinaceous cell adhesion molecules, but a serine protease, a metalloprotease, or a mixture thereof is preferable. Trypsin is one of the suitable serine proteases, and gelatinase is one of the suitable metalloproteases. Cells are released from the microcarriers by being treated with a protease. In addition, in a case where the materials of the microcarriers are proteins such as gelatin, the microcarriers themselves are decomposed through the treatment with a protease. At this time, in a case where the microcarriers are porous, cells that have been adhered to surfaces inside pores are also released from the microcarriers. In a case of microcarriers made of a porous gelatin matrix, gelatin is decomposed through the treatment with a protease, and a suspension containing released cells and decomposition products of gelatin. In this case, trypsin, gelatinase, or a mixture thereof is particularly suitable as a protease.

In the case where the materials of the microcarriers are proteins such as gelatin, a suspension containing cells released from microcarriers due to a protease and decomposition products (decomposition products of gelatin in a case where the materials are gelatin) of proteins which are the materials of the microcarriers is obtained. In order to formulate a preparation of released dental pulp-derived cells, it is preferable to remove the protease and impurities such as the decomposition products of the microcarrier materials from this suspension through an operation of washing cells. Hereinafter, the operation of washing cells in the case where the materials of the microcarriers are gelatin will be described in detail.

The suspension obtained through the treatment with a protease can be washed by repeating collection through centrifugation and suspension using a washing solution. The protease and fine decomposition products of gelatin can be removed through centrifugation. However, the decomposition products of gelatin also include residues with a size which are precipitated together with cells when centrifuged. Removal of such coarse residues of gelatin can be performed by means such as membrane filtration. From this point of view, in the case of performing a membrane filtration, the pore diameter of a filtration membrane is preferably 20 to 80 μm. For example, a filtration membrane having a pore diameter of 25 μm, 26 μm, 27 μm, 28 μm, 30 μm, 50 μm, 70 μm, or 80 μm can be used. The filtration with a filtration membrane can be efficiently performed by preventing clogging of filtration membranes by filtering with a filtration membrane having a large pore diameter and then filtering with a filtration membrane having a small pore diameter. For example, filtering may be first performed with a filtration membrane having a pore diameter of 60 to 100 μm and then performed with a filtration membrane having a pore diameter of 25 to 50 μm, or may be first performed with a filtration membrane having a pore diameter of 60 to 75 μm and then performed with a filtration membrane having a pore diameter of 25 to 30 μm. The filtration with a filtration membrane can also be performed through filtering only with a filtration membrane having a small pore diameter. The pore diameter of a filtration membrane used in this case is preferably 25 to 50 μm and more preferably 25 to 30 μm. For example, a filtration membrane having a pore diameter of 25 μm, 26 μm, 27 μm, 28 μm, or 30 μm is used. At this time, coarse residues of gelatin are left behind without passing through the filtration membrane, and cells in the filtrate are collected. However, the solution in which cells are suspended can be replaced with a cryopreservation liquid (cryopreservation liquid for preparation cells) to be described below using an ultrafiltration membrane or the like without collecting the cells to prepare a pre-freezing preparation cell suspension.

The washing solution used in the above-described washing operation is for sufficiently removing a medium and a protease. In the case where the microcarriers are proteins such as gelatin, the washing solution is also used for removing decomposition products thereof. The composition of such a washing solution is not particularly limited. Physiological saline, phosphate-buffered physiological saline, a Ringer's acetate solution, a bicarbonate Ringer's solution, or the like can be used as the washing solution, but a solution obtained by adding human serum albumin to a bicarbonate Ringer's solution is preferably used. Commercially available Ringer's acetate solutions and bicarbonate Ringer's solutions can be used. For example, PLASMA-LYTE A (Baxter) and Physio 140 (Otsuka Holdings Co., Ltd.) can be used as Ringer's acetate solutions, and BICARBON Injection (AJINOMOTO CO., INC.) can be used as a bicarbonate Ringer's solution. Suitable examples of the componential composition and the electrolyte concentration of a bicarbonate Ringer's solution are respectively shown in Tables 2 and 3.

A suitable example of the componential composition of the washing solution is shown in Table 4. In addition, a suitable example of the concentration of electrolyte contained in the washing solution is shown in Table 5. Sodium acetyltryptophan and sodium caprylate can also be excluded from the components of the washing solution shown in Tables 4 and 5.

In a case of performing centrifugation and membrane filtration to remove impurities after the treatment with a protease, centrifugation may be performed first, followed by membrane filtration, or membrane filtration may be performed first, followed by centrifugation. However, it is preferable that membrane filtration be performed first, followed by centrifugation.

The collected cells after washing with a washing solution are suspended in a cryopreservation liquid (cryopreservation liquid for preparation cells). A dental pulp-derived cell preparation obtained by encapsulating and freezing this suspension (pre-freezing preparation cell suspension) in a cell cryopreservation container is an optimal form for preservation or transportation among dental pulp-derived cell preparations. The composition of the solution portion (cryopreservation liquid for preparation cells) of the pre-freezing preparation cell suspension is not particularly limited as long as mammalian cells, particularly human pluripotent stem cell-enriched dental pulp-derived cells, can be frozen and thawed without dying. The cryopreservation liquid for preparation cells contains a cytoprotective agent. Cytoprotective agents have, for example, a function of suppressing formation of ice crystals inside cells and destruction of the cells when the cells are frozen. Suitable examples of cytoprotective agents include dimethyl sulfoxide (DMSO), ethylene glycol, propylene glycol, sericin, and glycerol. Two or more thereof can be combined and used as a cytoprotective agent. In a case of using dimethyl sulfoxide as a cytoprotective agent, the concentration thereof is preferably 5% to 15% (v/v) and more preferably 9% to 11% (v/v), and is, for example, 10% (v/v). The cryopreservation liquid may contain a buffer agent. A buffer agent that can adjust the pH of an aqueous solution to 6 to 8, for example, 6.8 to 7.8 is preferable. Examples of such buffer agents include ones containing carbonate ions, bicarbonate ions, citrate ions, and sodium ions. The cryopreservation liquid for preparation cells may further contain human serum albumin. In a case of using human serum albumin, the concentration thereof is preferably 40 to 100 g/L and more preferably 46 to 56 g/L, and may be, for example, 51 g/L.

One obtained by adding human serum albumin solution and dimethyl sulfoxide to a bicarbonate Ringer's solution is a suitable example of a cryopreservation liquid for preparation cells. The cryopreservation liquid for preparation cells contains 59 to 80.4 mM sodium chloride, 2.3 to 3.08 mM potassium chloride, 0.85 to 1.16 mM calcium chloride dihydrate, 0.28 to 0.385 mM magnesium chloride hexahydrate, 14 to 19.2 mM sodium hydrogen carbonate, 0.94 to 1.28 mM sodium citrate dihydrate, 46 to 56 g/L human serum albumin, 3.73 to 4.55 mM sodium acetyltryptophan, 3.74 to 4.58 mM sodium caprylate, and 9% to 11% (v/v) DMSO. The cryopreservation liquid for intermediate cells shown in Table 6 can also be suitably used as the cryopreservation liquid for preparation cells.

That is, a cryopreservation liquid for preparation cells that can be substantially used as a cryopreservation liquid for intermediate cells can be suitably used. For example, the composition thereof is, 65.8 to 80.4 mM sodium chloride, 2.52 to 3.08 mM potassium chloride, 0.95 to 1.16 mM calcium chloride dihydrate, 0.315 to 0.385 mM magnesium chloride hexahydrate, 15.7 to 19.2 mM sodium hydrogen carbonate, 1.04 to 1.28 mM sodium citrate dihydrate, 46 to 56 g/L human serum albumin, 3.73 to 4.55 mM sodium acetyltryptophan, 3.74 to 4.58 mM sodium caprylate, and 9% to 11% (v/v) DMSO. In addition to the composition shown in Table 6, another composition thereof is, for example, 70.8 to 71.3 mM sodium chloride, 2.71 to 2.73 mM potassium chloride, 1.01 to 1.02 mM calcium chloride dihydrate, 0.335 to 0.345 mM magnesium chloride hexahydrate, 16.9 to 17.0 mM sodium hydrogen carbonate, 1.12 to 1.14 mM sodium citrate dihydrate, 53.6 to 54.3 g/L human serum albumin, 4.36 to 4.41 mM sodium acetyltryptophan, 4.37 to 4.43 mM sodium caprylate, and 10.6% to 10.9% (v/v) DMSO. Among these, sodium acetyltryptophan and sodium caprylate can be excluded. The osmotic pressure ratio of the cryopreservation liquid for preparation cells to physiological saline is preferably 0.9 to 1.1.

In addition, a solution containing sodium ions, potassium ions, calcium ions, magnesium ions, hydrogen carbonate ions, citrate ions, human serum albumin, and dimethyl sulfoxide can be suitably used as the cryopreservation liquid for intermediate cells. For example, a solution containing sodium ions, potassium ions, calcium ions, magnesium ions, hydrogen carbonate ions, citrate ions, human serum albumin, and dimethyl sulfoxide respectively at concentrations of 91 to 113 mM, 2.52 to 3.08 mM, 0.95 to 1.16 mM, 0.315 to 0.385 mM, 15.6 to 19.2 mM, 1.04 to 1.28 mM, 46 to 56 g/L, and 9% to 11% (v/v) is a suitable example thereof. In addition, for example, a solution containing sodium ions, potassium ions, calcium ions, magnesium ions, hydrogen carbonate ions, citrate ions, human serum albumin, and dimethyl sulfoxide respectively at concentrations of 100 to 102 mM, 2.71 to 2.77 mM, 1.01 to 1.03 mM, 0.335 to 0.345 mM, 16.9 to 17.2 mM, 1.13 to 1.15 mM, 52.2 to 54.3 g/L, and 10.3% to 10.9% (v/v) is another suitable example thereof. Furthermore, for example, a solution containing sodium ions, potassium ions, calcium ions, magnesium ions, hydrogen carbonate ions, citrate ions, human serum albumin, and dimethyl sulfoxide respectively at concentrations of 102 mM, 2.80 mM, 1.05 mM, 0.35 mM, 17.4 mM, 1.16 mM, 51 g/L, and 10% (v/v) is still another suitable example thereof. Furthermore, for example, a solution containing sodium ions, potassium ions, calcium ions, magnesium ions, hydrogen carbonate ions, citrate ions, human serum albumin, and dimethyl sulfoxide respectively at concentrations of 101 mM, 2.77 mM, 1.03 mM, 0.34 mM, 17.2 mM, 1.15 mM, 52 g/L, and 10% (v/v) is still another suitable example thereof. Furthermore, for example, a solution containing sodium ions, potassium ions, calcium ions, magnesium ions, hydrogen carbonate ions, citrate ions, human serum albumin, and dimethyl sulfoxide respectively at concentrations of 100 mM, 2.71 mM, 1.01 mM, 0.34 mM, 16.9 mM, 1.13 mM, 53.6 g/L, and 10.7% (v/v) is still another suitable example thereof.

In a case where the cryopreservation liquid for preparation cells further contains acetyltryptophan or a salt thereof, the concentration thereof is preferably 3.73 to 4.55 mM and more preferably 4.24 to 4.41 mM, and is, for example, 4.14 mM, 4.24 mM, or 4.36 mM. In a case where the cryopreservation liquid for preparation cells further contains caprylic acid or a salt thereof, the concentration thereof is preferably 3.74 to 4.58 mM and more preferably 4.25 to 4.43 mM, and is, for example, 4.16 mM, 4.25 mM, or 4.37 mM.

The cell density in the suspension in which the pluripotent stem cell-enriched dental pulp-derived cells are suspended in the cryopreservation liquid for preparation cells is not particularly limited, but is preferably $5 \times 10^6$ to $8 \times 10^7$ cells/mL, more preferably $1.5 \times 10^7$ to $3 \times 10^7$ cells/mL, and still more preferably $2.1 \times 10^7$ to $3.0 \times 10^7$ cells/mL, and is, for example, $2.5 \times 10^7$ cells/mL. The suspended pluripotent stem cell-enriched dental pulp-derived cells are dispensed into a cell cryopreservation container, and are then cryopreserved.

The composition of the cell suspension in which the pluripotent stem cell-enriched dental pulp-derived cells are suspended in the cryopreservation liquid for preparation cells varies depending on the density of cells to be suspended, but is substantially 59 to 61 mM sodium chloride, 2.3 to 2.6 mM potassium chloride, 0.85 to 0.98 mM calcium chloride dihydrate, 0.28 to 0.32 mM magnesium chloride hexahydrate, 14 to 16 mM sodium hydrogen carbonate, 0.94 to 1.08 mM sodium citrate dihydrate, 49 to 50 g/L human serum albumin, 4.0 to 4.1 mM sodium acetyltryptophan, 4.0 to 4.1 mM sodium caprylate, and 9% to 11% (v/v) DMSO.

The amount of cell suspension to be dispensed into one cell cryopreservation container needs to be appropriately adjusted, and is preferably 1 to 20 mL. In addition, the number of cells to be dispensed into one cell cryopreservation container is preferably $5 \times 10^6$ to $9.2 \times 10^8$.

Suitable containers for cryopreserving cells suspended in a cryopreservation liquid for preparation cells, and procedures and conditions for freezing and preserving cells are the same as those already described for the cryopreservation of intermediate cells.

Cells cryopreserved in this manner can be used as pharmaceuticals (dental pulp-derived cell preparations) containing pluripotent stem cell-enriched dental pulp-derived cells as active components. In this case, the pluripotent stem cell-enriched dental pulp-derived cells are transported in a frozen state, and thawed when in use to be administered to a patient.

The viability of cells contained in the dental pulp-derived cell preparations obtained through the production culture, immediately after thawing is preferably greater than or equal to 50%, more preferably greater than or equal to 60%, and still more preferably greater than or equal to 70%, and is preferably, for example, greater than or equal to 80%, greater than or equal to 90%, or greater than or equal to 95%. It is possible to use only cells having a certain value or more of viability as dental pulp-derived cell preparations, which are pharmaceuticals, according to appropriately set standards.

When the cells contained in the dental pulp-derived cell preparations are cultured in a medium containing a substance known to induce differentiation into chondrocytes, an increase in expression level of aggrecan is observed as a whole. In addition, when the cells are cultured in a medium containing a substance known to induce differentiation into osteocytes, an increase in the amount of calcium accumulated in the cells is observed as a whole. That is, the cells have abilities to differentiate into chondrocytes and osteocytes when observed as a whole.

In one embodiment, when the cells contained in the dental pulp-derived cell preparations are observed as a whole, the cells are positive for at least one of CD73, CD90, CD105, and CD166 in expression patterns of the surface antigen markers. Similarly, the cells are negative for at least one of CD34 and CD45. For example, when the cells contained in the dental pulp-derived cell preparations are observed as a whole, the cells are positive for CD73 and CD90 and negative for CD34, or, for example, positive for CD73, CD90, CD105, and CD166 and negative for CD34 and CD45 in expression patterns of the surface antigen markers immediately after the cells are thawed or when the cells are cultured after thawing.

In addition, in one embodiment, when the cells contained in the dental pulp-derived cell preparations are observed as a whole, the cells are negative for at least one of CD40, CD80, CD86, and MHC-class II antigen in expression patterns of the surface antigen markers. For example, when the cells contained in the dental pulp-derived cell preparations are observed as a whole, the cells are negative for CD40, CD80, CD86, and MHC-class II antigen in the expression patterns of the surface antigen markers immediately after the cells are thawed or when the cells are cultured after thawing. It is known that, when cells positive for these surface antigen markers are transplanted into allogeneic individuals, the cells tend to be recognized as an antigen to be excluded from a living body. In a case where cells are negative for at least one of these surface antigen markers, this means that the pluripotent stem cell-enriched dental pulp-derived cells are hypoimmunogenic to that extent and tend to be hardly excluded from a living body when transplanted into allogeneic individuals. In addition, the cells may remain negative for CD40, CD80, and CD86 and be positive for MHC-class II antigen in the expression patterns of the surface antigen markers when the cells are stimulated with IFN-γ. The cells remain, for example, negative for CD40, CD80, and CD86 and become positive for MHC-class II antigen when, for example, the cells are stimulated with IFN-γ.

In addition, in one embodiment, when the cells contained in the dental pulp-derived cell preparations are observed as a whole, the cells are, for example, positive for CD73, CD90, CD105, and CD166 and negative for CD34, CD40, CD45, CD80, CD86, and MHC-class II antigen in expression patterns of the surface antigen markers immediately after the cells are thawed or when the cells are cultured after thawing. The cells remain negative for CD40, CD80, and CD86 and become positive for MHC-class II antigen when the cells are stimulated with IFN-γ.

In one embodiment, regarding a characteristic (d-1), when the cells contained in the dental pulp-derived cell preparations are observed as a whole, the cells are positive for at least one, preferably all of CD29, CD44, CD59, and CD164 in expression patterns of the surface antigen markers.

Here, when the individual cells included in all of the cells are observed, preferably at least 80%, more preferably at least 90%, and still more preferably at least 95% of the observed cells are positive for these antigens.

In addition, in one embodiment, regarding a characteristic (d-2), when the cells contained in the dental pulp-derived cell preparations are observed as a whole, the cells are positive for at least one, preferably all of CD9, CD13, CD46, CD47, CD58, CD63, CD73, CD81, CD90, CD98, CD147, HLA-A, -B, and -C, and EGF-R in expression patterns of the surface antigen markers.

Here, when the individual cells included in all of the cells are observed, preferably at least 70%, more preferably at least 80%, and still more preferably at least 90% of the observed cells are positive for these antigens.

In addition, in one embodiment, regarding a characteristic (d-3), when the cells contained in the dental pulp-derived cell preparations are observed as a whole, the cells are positive for at least one, preferably all of CD49b, CD49c, CD49e, CD55, CD95, CD151, and CD166 in expression patterns of the surface antigen markers.

Here, when the individual cells included in all of the cells are observed, preferably at least 65%, more preferably at least 75%, and still more preferably at least 80% of the observed cells are positive for these antigens.

In addition, in one embodiment, regarding a characteristic (d-4), when the cells contained in the dental pulp-derived cell preparations are observed as a whole, the cells are positive for at least one, preferably all of CD10, CD49f, CD105, and CD140b in expression patterns of the surface antigen markers.

Here, when the individual cells included in all of the cells are observed, preferably at least 60%, more preferably at least 70%, and still more preferably at least 75% of the observed cells are positive for these antigens.

In one embodiment, the cells contained in the dental pulp-derived cell preparations preferably have two or more, more preferably three or more, and still more preferably all of the characteristics shown in the above-described (d-1), (d-2), (d-3), and (d-4). For example, the cells having the characteristics shown in the above-described (d-1) and (d-2) are a suitable embodiment of the present invention.

In one embodiment, regarding a characteristic (d-5), when the cells contained in the dental pulp-derived cell preparations are observed as a whole, the cells are negative for at least one, preferably all of CD1a, CD1d, CD2, CD3, CD4, CD5, CD7, CD8a, CD8b, CD11b, CD11c, CD15, CD15s, CD16, CD18, CD19, CD20, CD21, CD22, CD23, CD24, CD25, CD28, CD30, CD31, CD32, CD33, CD34, CD35, CD37, CD38, CD41a, CD86, CD87, CD88, CD89, CDw93, CD94, CD100, CD102, CD103, CD114, CD117, CD118, CD120b, CD121b, CD122, CD123, CD124, CD126, CD127, CD128b, CD132, CD134, CD135, CD137, CD137ligand, CD138, CD144, CD150, CD153, CD154, CD158a, CD158b, CD161, CD162, CD163, CD172b, CD177, CD178, CD180, CD184, CD195, CD196, CD197, CD205, CD206, CD210, CD212, CD220, CD226, CD229, CD231, CD235a, CD244, CD255, CD267, CD268, CD278, CD279, CD282, CD294, CD305, CD309, CD314, CD321, CDw327, CDw328, CD329, CD335, CD336, CD337, BLTR-1, CLIP, CMRF-44, CMRF-56, fMLP-R, SSEA-1, TRA-1-60, CLA, integrin β7, and Invariant NKT in expression patterns of the surface antigen markers.

Here, when the individual cells included in all of the cells are observed, preferably only at most 10%, more preferably only at most 5%, and still more preferably only at most 2%, and still more preferably only at most 1% of the observed cells are positive for these antigens.

In one embodiment, regarding a characteristic (d-6), when the cells contained in the dental pulp-derived cell preparations are observed as a whole, the cells are negative for at least one, preferably all of CD1b, CD6, CD27, CD41b, CD42a, CD42b, CD43, CD45, CD45RB, CD48, CD50, CD53, CD57, CD62E, CD62L, CD62P, CD64, CD66b, CD66f, CD69, CD70, CD72, CD75, CD84, CD85, CD97, CD99R, CD183, CD193, SSEA-3, and γδ TCR in expression patterns of the surface antigen markers.

Here, when the individual cells included in all of the cells are observed, preferably only at most 10%, more preferably only at most 5%, and still more preferably only at most 2% of the observed cells are positive for these antigens.

In one embodiment, regarding a characteristic (d-7), when the cells contained in the dental pulp-derived cell preparations are observed as a whole, the cells are negative for at least one, preferably all of CD4v4, CD14, CD36, CD45RA, CD45RO, CD66 (a, c, d, e), CD79b, CD83, CD106, CD152, CD209, CD271, CD275, CD326, MIC A/B, and αβ TCR in expression patterns of the surface antigen markers.

Here, when the individual cells included in all of the cells are observed, preferably at most 10% and more preferably at most 5% of the observed cells are positive for these antigens.

In one embodiment, regarding a characteristic (d-8), when the cells contained in the dental pulp-derived cell preparations are observed as a whole, the cells are negative for at least one, preferably all of CD26, CD40, CD56, CD80, CD146, and MHC-class II antigen in expression patterns of the surface antigen markers.

Here, when the individual cells included in all of the cells are observed, preferably only at most 20% and more preferably only at most 10% of the observed cells are positive for these antigens.

In one embodiment, the cells contained in the dental pulp-derived cell preparations preferably have two or more, more preferably three or more, and still more preferably all of the characteristics shown in the above-described (d-1), (d-2), (d-3), (d-4), (d-5), (d-6), (d-7), and (d-8). For example, the cells having the characteristics shown in the above-described (d-1) and (d-5) are a suitable embodiment of the present invention.

In addition, when the cells contained in the dental pulp-derived cell preparations are observed as a whole, the cells express prostaglandin $E_2$ ($PGE_2$) and/or vascular endothelial growth factor (VEGF) immediately after thawing or during culture after thawing, and the expression level of prostaglandin $E_2$ ($PGE_2$) is increased by stimulating the cells with TNF-α.

In addition, in one embodiment, when the cells contained in the dental pulp-derived cell preparations are cultured in the presence of IFN-γ, the amount of kynurenine secreted increases.

In one embodiment, when the cells contained in the dental pulp-derived cell preparations are observed as a whole, the cells are positive for at least one or all of CD47, CD81, and CD147 and negative for at least one or all of CD19, CD34, and CD206 in expression patterns of the surface antigen markers. In addition, in one embodiment of the present invention, when the cells contained in the dental pulp-derived cell preparations are observed as a whole, the cells are positive for at least one or all of CD47, CD81, and CD147 and negative for at least one or all of CD19, CD31, CD33, CD34, CD38, CD45, CD206, CD235a, and SSEA-1 in expression patterns of the surface antigen markers.

In one embodiment, the cells contained in the dental pulp-derived cell preparations are negative for at least one, for example, all of CD19, CD26, CD34, CD56, CD106, CD117, CD146, and CD271.

In one embodiment, when the cells contained in the dental pulp-derived cell preparations are observed as a whole, the cells are positive for at least one, for example, all of CD140b and HLA-A, -B, and -C in expression patterns of the surface antigen markers.

In one embodiment, when the cells contained in the dental pulp-derived cell preparations are observed as a whole, the cells are positive for at least one, for example, all of CD10, CD49e, and CD95 in expression patterns of the surface antigen markers.

In one embodiment of the present invention, when the cells contained in the dental pulp-derived cell preparations are observed as a whole, the cells are positive for at least one, for example, all of CD46, CD47, CD55, CD58, and CD59 in expression patterns of the surface antigen markers.

In one embodiment of the present invention, when the cells contained in the dental pulp-derived cell preparations are observed as a whole, the cells are, for example, positive for at least one of CD73, CD90, CD105, and CD166 and negative for at least one of CD34 and CD45 in expression patterns of the surface antigen markers.

In addition, when the cells contained in the dental pulp-derived cell preparations are observed as a whole immediately after thawing or when cultured after thawing, the cells are positive for CD73, CD90, CD105, and CD166 and negative for CD34, CD40, CD45, CD80, CD86, and MHC-class II antigen in expression patterns of the surface antigen markers. In addition, the cells remain negative for CD40, CD80, and CD86 and become positive for MHC-class II antigen when the cells are stimulated with IFN-γ. In addition, the cells express prostaglandin $E_2$ ($PGE_2$) and/or vascular endothelial growth factor (VEGF), and the amount of prostaglandin $E_2$ ($PGE_2$) secreted is increased by stimulating the cells with TNF-α.

When the cells contained in the dental pulp-derived cell preparations are cultured, the cells secrete various humoral factors.

In one embodiment, regarding a characteristic (d-9), when the cells contained in dental pulp-derived cell preparations are cultured, the cells express at least one, preferably all of MMP-2, IGFBP-4, and Cystatin C.

In addition, regarding a characteristic (d-10), in one embodiment, when the cells contained in the dental pulp-derived cell preparations are cultured, the cells express at least one, preferably all of IL-6, IL-11, MCP-1, IL-8, GROα, HGF, VEGF, VCAM-1, TIMP-3, TIMP-2, and TIMP-1.

In addition, regarding a characteristic (d-11), in one embodiment, when the cells contained in the dental pulp-derived cell preparations are cultured, the cells express at least one, preferably all of IL-23, IFN-α, TNF-α, IL-18, IL-27, TARC, ENA-78, MIP-3α, MIP-1β, IP-10, SCF, and ICAM-1.

In addition, regarding a characteristic (d-12), in one embodiment, when the cells contained in the dental pulp-derived cell preparations are cultured, the cells do not or hardly express at least one, preferably all of IL-21, Eotaxin, MIP-1α MIG, I-TAC, IP-10, and GM-CSF.

In addition, regarding a characteristic (d-13), in one embodiment, when the cells contained in the dental pulp-derived cell preparations are cultured, at least one of the expression levels of IL-6, IL-23, IL-11, MCP-1, ENA-78, HGF, VEGF, MMP-2, IGFBP-4, Cystatin C, TIMP-3, TIMP-2, and TIMP-1 increase compared to the expression levels of intermediate cells. For example, the expression levels of IL-6, IL-11, HGF, TIMP-3, and TIMP-1 increase compared to the expression levels of intermediate cells.

In addition, regarding a characteristic (d-14), in one embodiment, in a case where the cells contained in the dental pulp-derived cell preparations are cultured in the presence of TNF-α or IFN-γ, the expression level of IL-6 is greater than that in a case where the cells are cultured in the absence thereof.

In addition, regarding a characteristic (d-15), in one embodiment, in a case where the cells contained in the dental pulp-derived cell preparations are cultured in the presence of TNF-α or IFN-γ, the expression level of IL-11 is greater than that in a case where the cells are cultured in the absence thereof.

In addition, regarding a characteristic (d-16), in one embodiment, in a case where the cells contained in the dental pulp-derived cell preparations are cultured in the presence of TNF-α or IFN-γ, the expression level of IP-10 is greater than that in a case where the cells are cultured in the absence thereof.

In addition, regarding a characteristic (d-17), in one embodiment, in a case where the cells contained in the dental pulp-derived cell preparations are cultured in the presence of TNF-α or IFN-γ, the expression level of MCP-1 is greater than that in a case where the cells are cultured in the absence thereof.

In addition, regarding a characteristic (d-18), in one embodiment, expression of GM-CSF is induced when the cells contained in the dental pulp-derived cell preparations are cultured in the presence of TNF-α, but the expression of GM-CSF is not induced when the cells are cultured in the presence of IFN-γ.

In addition, regarding a characteristic (d-19), in one embodiment, the expression level of HGF decreases when the cells contained in the dental pulp-derived cell preparations are cultured in the presence of TNF-α compared to a case where the cells are cultured in the absence thereof, whereas the expression level of HGF increases when the cells are cultured in the presence of IFN-γ compared to a case where the cells are cultured in the absence thereof.

In addition, regarding a characteristic (d-20), in one embodiment, in a case where the cells contained in the dental pulp-derived cell preparations are cultured in the presence of TNF-α, the expression level of IL-8 is greater than that in a case where the cells are cultured in the absence thereof.

In one embodiment, the pluripotent stem cell-enriched dental pulp-derived cells of the present invention preferably have two or more, more preferably three or more, and still more preferably four or more, and still more preferably all of the characteristics shown in the above-described (d-9) to (d-20). For example, the cells having the characteristics shown in the above-described (d-9) and (d-16) and the cells having the characteristics shown in the above-described (d-9), (d-15), and (d-16) are a suitable embodiment of the present invention.

In addition, when the cells contained in the dental pulp-derived cell preparations are observed as a whole immediately after thawing or when cultured after thawing, the expression level of aggrecan increases in a case where the cells are cultured in a medium containing a substance known to induce differentiation into chondrocytes. In addition, when the pluripotent stem cell-enriched dental pulp-derived cells are cultured in a medium containing a substance known to induce differentiation into osteocytes and observed as a whole, the amount of calcium accumulated in the cells increases. That is, the pluripotent stem cell-enriched dental pulp-derived cells have abilities to differentiate into chondrocytes and osteocytes. The fact that the cells contained in the dental pulp-derived cell preparations have an ability to differentiate into chondrocytes and an ability to differentiate into osteocytes can be examined respectively through methods described in Examples 20 and 19. Only cell groups confirmed to have an ability to differentiate into chondrocytes and an ability to differentiate into osteocytes through these methods can be supplied to medical institutions as products.

According to appropriately set standards, only cells showing predetermined expression patterns of surface antigen markers and/or gene expression patterns can be preserved as dental pulp-derived cell preparations.

In addition, when the cells contained in the dental pulp-derived cell preparations are observed as a whole immediately after thawing or when cultured after thawing, the cells are mostly accounted for dental pulp-derived pluripotent stem cells. The dental pulp-derived pluripotent stem cells in flat culture are observed under an optical microscope as substantially spindle-shaped adhered cells. The proportion of substantially spindle-shaped cells in all cells in flat culture when observed under an optical microscope is preferably greater than or equal to 99%, more preferably greater than or equal to 99.5%, still more preferably greater than or equal to 99.9%, and still more preferably greater than or equal to 99.95%. Only dental pulp-derived cell preparations having a certain value or more of a proportion of substantially spindle-shaped cells can be supplied to medical institutions as products according to appropriately set standards.

In addition, when the cells contained in the dental pulp-derived cell preparations are cultured after thawing, the cells have an ability of undergoing at least 3 cell divisions, preferably have an ability of undergoing at least 4 cell divisions, more preferably have an ability of undergoing at least 5 cell divisions, and still more preferably an ability of undergoing at least 10 cell divisions. In addition, the average doubling time of the dental pulp-derived cell preparations when these are thawed and cultured on a cell culture plate is preferably within 96 hours, more preferably within 84 hours, still more preferably within 72 hours, still more preferably within 48 hours, and still more preferably within 36 hours. The culture conditions of the cells at this time which are the same as or equivalent to those of the above-described pluripotent stem cell-enriched dental pulp-derived cells described in, for example, Example 5 are used. Only dental pulp-derived cell preparations having an ability of undergoing cell division a certain number of times or more can be supplied to medical institutions as products according to appropriately set standards. In addition, only dental pulp-derived cell preparations having an average doubling time within a certain time can also be supplied to medical institutions as products according to appropriately set standards. Such standards are, for example, cells having an ability of undergoing at least 3 cell divisions and an average doubling time within 72 hours, cells having an ability of undergoing at least 4 cell divisions and an average doubling time within 72 hours, and cells having an ability of undergoing at least 5 cell divisions and an average doubling time within 72 hours.

That is, the cells contained in the dental pulp-derived cell preparations retain properties of stem cells which have a high division ability and can be differentiated into two or more cells. The intermediate cells to be used for a further culture step can also be called cells having a high colony-forming ability.

The cells contained in the dental pulp-derived cell preparations preferably have undergone at least 16 cell divisions in an in vitro environment. For example, the cells have undergone at least 21, 22, or 23 cell divisions.

When the cells contained in the dental pulp-derived cell preparations are thawed, an average diameter of the cells in a state in which the cells are made to float in a solution, for example, a medium is less than or equal to 20 μm or less than or equal to 18 μm, and is, for example, 10 to 20 μm, 10 to 18 μ, 12 to 20 μm, 14 to 20 μ, and 16 to 20 μm.

Items of quality tests of the cells contained in the dental pulp-derived cell preparations are exemplified below as Quality Tests a' to k'. The quality tests are performed on one or more items thereof. All of these items may be carried out. Cells to be used for the quality tests at this time are either cells (1), as a dental pulp-derived cell preparation, obtained by fractionating some cells before being suspended in a cryopreservation liquid, cells (2), as a dental pulp-derived cell preparation, obtained by fractionating some cells before being suspended in a cryopreservation liquid and further subculturing these fractionated cells, cells (3), as a dental pulp-derived cell preparation, before freezing which have been suspended in a cryopreservation liquid for freezing, cells (4), as a dental pulp-derived cell preparation, immediately after thawing frozen cells, or cells (5), as a dental pulp-derived cell preparation, obtained by thawing frozen cells and further culturing the thawed cells. Unless otherwise specified, the same quality test may be performed on two or more of these cells (1) to (5).

(Quality Test a') Verifying that a proportion of the number of cells showing a substantially spindle-shaped form in all cells when observed under an optical microscope is greater than or equal to 99%, greater than or equal to 99.5%, greater than or equal to 99.9%, or greater than or equal to 99.95.

(Quality Test b') Verifying that a cell viability is greater than or equal to 50%, greater than or equal to 60%, greater than or equal to 70%, greater than or equal to 80%, greater than or equal to 90%, or greater than or equal to 95%.

(Quality Test c') Verifying that cells are positive for CD73, CD90, CD105, and CD166 and negative for CD34 and CD45 in expression patterns of the cell surface antigen markers, or verifies that cells are positive for at least one of CD73 and CD90 and negative for CD34 in expression patterns of the cell surface antigen markers.

(Quality Test d') Verifying that cells are negative for CD40, CD80, CD86, and MHC-class II antigen in expression patterns of the cell surface antigen markers, or verifies that cells are negative for at least CD40, CD80, CD86, and MHC-class II antigen in expression patterns of the cell surface antigen markers.

(Quality Test e') Verifying that cells remain negative for CD40, CD80, and CD86 and become positive for MHC-class II antigen in expression patterns of the cell surface antigen markers when the cells are stimulated with IFN-γ, or verifies at least one that cells are negative for CD40, negative for CD80, negative for CD86, and negative for MHC-class II antigen in expression patterns of the cell surface antigen markers when the cells are stimulated with IFN-γ.

(Quality Test f') Verifying that cells express prostaglandin $E_2$ ($PGE_2$) and/or vascular endothelial growth factor (VEGF) and an expression level of prostaglandin $E_2$ ($PGE_2$) is increased by stimulating the cells with TNF-α.

(Quality Test g') Verifying that an expression level of aggrecan increases when cells are cultured in a medium containing a substance known to induce differentiation into chondrocytes.

(Quality Test h') Verifying that an amount of calcium accumulated in cells increases when the cells are cultured in a medium containing a substance known to induce differentiation into osteocytes.

(Quality Test i') Verifying that cells have an ability of undergoing at least 3, 4, or 5 cell divisions on a cell culture plate.

(Quality Test j') Verifying that an average doubling time of cells on a cell culture plate is within 96 hours, 84 hours, 72 hours, 48 hours, or 36 hours during a period of Quality Test i'.

(Quality Test k') Verifying that an average diameter of cells in a state where the cells are made to float in a medium is less than or equal to 20 μm, less than or equal to 18 μm, 10 to 20 μm, 10 to 18 μm, 12 to 20 μm, 14 to 20 μm, or 16 to 20 μm.

The above-described quality tests may be carried out on arbitrary 10 items (Quality Test a') to (Quality Test j'). In addition, arbitrary 9 items may be selected among these 10 items to carry out the quality tests. In the case where 9 items are selected to carry out the quality tests, for example, Quality Tests a', b', c', d', e', f, g', i', and j' may be selected, but there is no limitation. In addition, arbitrary 8 items may be selected among these 10 items to carry out the quality tests. In the case where 8 items are selected to carry out the quality tests, for example, Quality Tests a', b', c', d', e', f', i', and j' may be selected, but there is no limitation. In addition, arbitrary 7 items may be selected among these 10 items to carry out the quality tests. In the case where 7 items are selected to carry out the quality tests, for example, Quality Tests a', b', c', d', e', i', and j' may be selected, but there is no limitation. In addition, arbitrary 6 items may be selected among these 10 items to carry out the quality tests. In the case where 6 items are selected to carry out the quality tests, for example, Quality Tests a', b', c', d', i', and j' may be selected, but there is no limitation. In addition, arbitrary 5 items may be selected among these 10 items to carry out the quality tests. In the case where 5 items are selected to carry out the quality tests, for example, Quality Tests a', b', c', i', and j' may be selected, but there is no limitation. In addition, arbitrary 4 items may be selected among these 10 items to carry out the quality tests. In the case where 4 items are selected to carry out the quality tests, for example, Quality Tests a', b', c', and j' may be selected, but there is no limitation. In addition, arbitrary 3 items may be selected among these 10 items to carry out the quality tests. In the case where 3 items are selected to carry out the quality tests, for example, Quality Tests a', b', and c' may be selected, but there is no limitation. In addition, arbitrary 2 items may be selected among these 10 items to carry out the quality tests. In the case where 2 items are selected to carry out the quality tests, for example, Quality Tests a' and b', Quality Tests a' and c', Quality Tests a' and d', Quality Tests a' and e', Quality Tests a' and f', Quality Tests a' and g', Quality Tests a' and h', Quality Tests a' and i', Quality Tests a' and j', Quality Tests b' and c', Quality Tests b' and d', Quality Tests b' and e', Quality Tests b' and f', Quality Tests b' and g', Quality Tests b' and h', Quality Tests b and i, Quality Tests b and j, Quality Tests c and d, Quality Tests c and e, Quality Tests c' and f', Quality Tests c' and g', Quality Tests c' and h', Quality Tests c' and i', Quality Tests c' and j', Quality Tests d' and e', Quality Tests d' and f', Quality Tests d' and g', Quality Tests d' and h', Quality Tests d' and i', Quality Tests d' and j', Quality Tests e' and f', Quality Tests e' and g', Quality Tests e' and h', Quality Tests e' and i', Quality Tests e' and j', Quality Tests f' and i', Quality Tests f' and j', or Quality Tests i' and j' may be selected, but there is no limitation.

In the method for producing dental pulp-derived cells enriched with pluripotent stem cells of the present invention, pluripotent stem cells contained in dental pulp are cultured, proliferated, and cryopreserved as intermediate cells, and are then thawed, further cultured, and proliferated to produce a dental pulp-derived cell preparation. The process up to the preparation of a dental pulp-derived cell preparation from intermediate cells includes a step of culturing pluripotent stem cells in a state in which the pluripotent stem cells are adhered to the surfaces of particles. By including such a step, in one embodiment, cells contained in dental pulp-derived cell preparations have properties different from the intermediate cells.

For example, as for positive rates of surface antigens, when the cells contained in the dental pulp-derived cell preparations are observed as a whole, the cells have high positive rates of any one or all of CD39, CD49a, CD61, CD107a, CD107b, and CD143 in expression patterns of the surface antigen markers compared to the intermediate cells. For example, the cells contained in the dental pulp-derived cell preparations have a high positive rate of CD107b compared to the intermediate cells. The positive rates of these surface antigens of the cells contained in the dental pulp-derived cell preparations are preferably at least 10% higher and more preferably at least 20% higher than that of the intermediate cells. In addition, the cells contained in the dental pulp-derived cell preparations have a low positive rate of CD146 compared to the intermediate cells. The positive rate of CD146 of the cells contained in the dental pulp-derived cell preparations is at least 20% lower or at least 50% lower than that of the intermediate cells.

In addition, for example, as for the expression levels of humoral factors, the cells contained in the dental pulp-derived cell preparations have high expression levels of any one or all of IL-6, IL-23, IL-11, MCP-1, ENA-78, HGF, VEGF, MMP-2, IGFBP-4, Cystatin C, TIMP-3, TIMP-2, and TIMP-1 compared to the intermediate cells. In particular, the cells contained in the dental pulp-derived cell preparations have high expression levels of any one or all of IL-6, IL-11, HGF, IGFBP-4, TIMP-3, and TIMP-1 compared to the intermediate cells. In particular, the cells contained in the dental pulp-derived cell preparations have high expression levels of any one or all of IL-6 and HGF compared to the intermediate cells.

The intermediate cells and the cells contained in the dental pulp-derived cell preparations retain properties of stem cells which have a high division ability and can be differentiated into two or more cells. These cells can also be called cells having a high colony-forming ability.

When the intermediate cells and the cells contained in the dental pulp-derived cell preparations are observed as a whole, the cells are, for example, positive for at least one of CD73, CD90, CD105, and CD166 and negative for at least one of CD34 and CD45 in expression patterns of the surface antigen markers. For example, the intermediate cells and the cells contained in the dental pulp-derived cell preparations are, for example, positive for CD73 and CD90 and negative for CD34 in the expression patterns of the surface antigen markers. These expression patterns are common to mesenchymal stem cells. However, these expression patterns also have characteristic properties.

In one embodiment of the present invention, when the intermediate cells and the cells contained in the dental pulp-derived cell preparations are observed as a whole, the cells are positive for at least one or all of CD47, CD81, and CD147 and negative for at least one or all of CD19, CD34, and CD206 in expression patterns of the surface antigen markers. In addition, in one embodiment of the present invention, when the pluripotent stem cell-enriched dental pulp-derived cells are observed as a whole, the cells are positive for at least one or all of CD47, CD81, and CD147 and negative for at least one or all of CD19, CD31, CD33, CD34, CD38, CD45, CD206, CD235a, and SSEA-1 in expression patterns of the surface antigen markers.

In one embodiment, when the cells contained in the intermediate cells and the cells contained in the dental pulp-derived cell preparations are observed as a whole, the cells are negative for at least one, for example, all of CD19, CD26, CD34, CD106, CD117, and CD271 in expression patterns of the surface antigen markers. In addition, the cells contained in the dental pulp-derived cell preparations are negative for at least one, for example, all of CD19, CD26, CD34, CD56, CD106, CD117, CD146, and CD271. Among these, although the function of CD106 has not been sufficiently elucidated, it is considered that CD106 is involved in activation of inflammatory signals because it is expressed in vascular endothelial cells at an inflammatory site. The expression patterns of these surface antigens are different from those usually known as stem cells having a high colony-forming ability, but nevertheless, it is surprising that the intermediate cells and the cells contained in the dental pulp-derived cell preparations have a high colony-forming ability.

In one embodiment, when the intermediate cells and the cells contained in the dental pulp-derived cell preparations are observed as a whole, the cells are positive for at least one, for example, all of CD140b and HLA-A, -B, and -C in expression patterns of the surface antigen markers.

In one embodiment, when the cells contained in the dental pulp-derived cell preparations are observed as a whole, the cells are positive for at least one, for example, all of CD49e, and CD95 in expression patterns of the surface antigen markers.

In one embodiment, when the intermediate cells and the cells contained in the dental pulp-derived cell preparations are observed as a whole, the cells are positive for CD10 in an expression pattern of the surface antigen marker. Although the function of CD10 has not been sufficiently elucidated, there is a possibility that CD10 may be involved in convergence of inflammatory responses by decomposing inflammation-related peptides and inflammation-related substances such as enkephalin or a substance P.

In addition, in one embodiment, when the cells contained in the dental pulp-derived cell preparations are observed as a whole, the cells have a high positive rate of CD39 in an expression pattern of the surface antigen marker as compared to the intermediate cells. In addition, all of these cells are positive for CD73. CD39 cooperates with CD73 to convert ATP to adenosine, thereby increasing the extracellular adenosine concentration. Adenosine has a function of suppressing inflammation by negatively controlling an immune response. It is thought that this function in a dental pulp-derived cell preparation having a high positive rate of CD39 is higher than that in the intermediate cells.

In addition, in one embodiment, when the intermediate cells and the cells contained in the dental pulp-derived cell preparations are observed as a whole (population), the cells are positive for CD46 in an expression pattern of the surface antigen marker. CD46 is thought to be involved in avoidance of complement-dependent cytotoxicity by suppressing an activity of complement (C3).

In addition, in one embodiment, when these cells are observed as a whole, these cells are positive for CD47 in an expression pattern of the surface antigen marker. CD47 imparts resistance to phagocytosis of macrophages to cells.

In addition, in one embodiment, when these cells are observed as a whole, these cells are positive for CD55 in an expression pattern of the surface antigen marker. It is thought that CD55 has a function of adjusting a classical or second path of complement and is involved in avoidance of cytotoxicity due to complement.

In addition, in one embodiment, when these cells are observed as a whole, these cells are positive for CD58 in an expression pattern of the surface antigen marker. Although the function of CD58 has not been sufficiently elucidated, it is suggested that CD58 contributes to convergence of inflammatory responses by inducing regulatory T cells (Treg cells).

In addition, in one embodiment, these cells are positive for CD59. CD59 is thought to be involved in avoidance of cytotoxicity due to complement by inhibiting formation of membrane damaging complexes by acting on complement (C9).

The intermediate cells and the cells contained in the dental pulp-derived cell preparations secrete various humoral factors. In one embodiment, the intermediate cells and the cells contained in the dental pulp-derived cell preparations secrete at least one, for example, all of MMP-2, IGFBP-4, Cystatin C, IL-6, IL-11, MCP-1, IL-8, HGF, vascular endothelial cell growth factor (VEGF), TIMP-1, TIMP-2, and TIMP-3. In addition, the intermediate cells and the cells contained in the dental pulp-derived cell preparations secrete at least one, for example, all of GROα, VCAM-I, and IP-10.

In one embodiment, the intermediate cells and the cells contained in the dental pulp-derived cell preparations secrete IL-6. The amount of IL-6 secreted increases due to TNF-α stimulation and IFN-γ stimulation. Although IL-6 is also known as an inflammatory cytokine, it is known that an inflammatory response in the liver is exacerbated in IL-6-deficient mice. Therefore, it is thought that IL-6 has an anti-inflammatory effect.

The intermediate cells and the cells contained in the dental pulp-derived cell preparations secrete various humoral factors. In one embodiment, the intermediate cells and the cells contained in the dental pulp-derived cell preparations secrete IL-11. The amount of IL-11 secreted increases due to TNF-α stimulation and IFN-γ stimulation. It is thought that IL-11 exhibits an anti-inflammatory effect by suppressing secretion of an inflammatory cytokine.

The intermediate cells and the cells contained in the dental pulp-derived cell preparations secrete various humoral factors. In one embodiment, the intermediate cells and the cells contained in the dental pulp-derived cell preparations secrete IP-10. The amount of IP-10 secreted increases due to TNF-α stimulation and IFN-γ stimulation. It is thought that IP-10 exhibits an anti-inflammatory effect by suppressing secretion of an inflammatory cytokine.

Dental pulp-derived cells have functions such as an anti-inflammatory effect. Some or all of the above-described surface antigens and humoral factors, and other elements cooperate with each other to exert their functions.

A pharmaceutical composition containing the dental pulp-derived cell preparation of the present invention as an active component can be used as therapeutic agents for various diseases. For example, the dental pulp-derived cell preparation can be used for treating and preventing diseases or disorders selected from the group consisting of autoimmune diseases, inflammatory diseases, rheumatoid arthritis, Crohn's disease, chronic inflammatory bowel disease, myocardial infarction, cerebral infarction (including chronic cerebral infarction and acute cerebral infarction), chronic inflammatory demyelinating polyneuritis, multiple sclerosis, systemic lupus erythematosus, liver cirrhosis (including decompensated liver cirrhosis), sepsis, osteoarthritis, psoriasis, and organ graft rejection. A cell preparation containing stem cells such as mesenchymal stem cells are known as a cell preparation that can be used as a therapeutic agent for autoimmune diseases or the like. However, well-known cell preparations do not show significant therapeutic effects in all patients, and their usage is also limited. The dental pulp-derived cell preparation of the present invention adds diversity to cell preparations that can be used as therapeutic agents for autoimmune diseases or the like. For example, it is possible to provide new treatment opportunities for patients who have not obtained any therapeutic effects from well-known cell preparations or patients suffering from diseases for which no therapeutic effect has been proven with well-known cell preparations, by using the dental pulp-derived cell preparation of the present invention as therapeutic agents for the patients.

The dental pulp-derived cell preparation is administered to patients suffering from the above-described diseases by means such as intravenous drip infusion or local injection.

The dental pulp-derived cell preparation is thawed when in use. At the time of use, the dental pulp-derived cell preparation is taken out of a liquid nitrogen storage container and thawed. The thawing is performed through heating in a water bath, for example, at 36.5°C to 37.5°C. The thawed dental pulp-derived cell preparation can be administered to humans as pharmaceutical products through means such as intravenous drip infusion or local injection. In the case of intravenous drip infusion, the dental pulp-derived cell preparation is transferred to a dialysis bag from a vial and then administered to a patient through intravenous drip infusion. In the case of local injection, the dental pulp-derived cell preparation is transferred to a syringe from a vial and then injected locally into a patient.

It is expected that thawing of the dental pulp-derived cell preparation before use will be carried out in medical institutions. Supply of the dental pulp-derived cell preparation to medical institutions is performed, for example, as follows, but the present invention is not particularly limited thereto. Dental pulp-derived cell preparations which have been cryopreserved in storages of their manufacturers, distributors, or the like are shipped in a frozen state in response to requests from medical institutions and transported to the medical institutions. The preparations which have been carried in the medical institutions in a frozen state are thawed immediately before administration to patients, and are then administered to patients through intravenous injection or the like. A mobile low-temperature workbench (WO2017/099105) can be suitably used as a device for handling the dental pulp-derived cell preparations in a frozen state.

## Examples

Hereinafter, the present invention will be described in detail with reference to examples, but the present invention is not intended to be limited to the examples.

[Example 1: Preparation of Medium and Reagent]

DMEM (10% FBS) Medium: Obtained by adding FBS (GE Healthcare) to DMEM Low Glucose (at a glucose concentration of 5.56 mM, GE Healthcare) so that the final concentration of FBS became 10%.

DMEM (20% FBS) Medium: Obtained by adding FBS (GE Healthcare) to DMEM Low Glucose (at a glucose concentration of 5.56 mM, GE Healthcare) so that the final concentration of FBS became 20%.

Aqueous streptomycin solution: Obtained by dissolving streptomycin sulfate in pure water so that the final concentration of streptomycin sulfate became 0.01 g/mL.

Protease solution: Prepared by adding DMEM Low Glucose to 5 mg of Liberase (Roche) so that the final concentration of DMEM Low Glucose became 2.5 mg/mL. It is a solution containing collagenase I, collagenase II, and thermolysin as proteases.

DMEM (20% FBS) streptomycin medium: Obtained by mixing a DMEM (20% FBS) medium with a streptomycin solution at a volume ratio of 100:1.

Trypsin-EDTA solution: 0.25% Trypsin-EDTA solution (Thermo Fisher Scientific Inc.)

1% Chlorhexidine gluconate solution: Obtained by diluting a 5% (w/v) Fermajin solution (SIOE PHARMACEUTICAL CO., LTD.) by 5 times with injection water.
Bicarbonate Ringer's solution: A BICARBON infusion solution (AY Pharmaceuticals Co., Ltd.) having the composition shown in Tables 7 and 8 below.

[Table 7]

Table 7 Componential composition of bicarbonate Ringer's solution

| Component | Concentration (g/L) | Concentration (mM) |
|---|---|---|
| Sodium chloride | 6.14 | 105 |
| Potassium chloride | 0.3 | 4.02 |
| Calcium chloride dihydrochloride | 0.22 | 1.5 |
| Magnesium chloride hexahydrate | 0.102 | 0.501 |
| Sodium hydrogen carbonate | 2.1 | 25 |
| Sodium citrate dihydrate | 0.49 | 1.67 |
| Osmotic pressure ratio of cryopreservation liquid for intermediate cells to physiological saline at pH of 6.8 to 7.8 being 0.9 to 1.0 | | |

[Table 8]

Table 8 Concentration of electrolyte contained in bicarbonate Ringer's solution

| Electrolyte | Concentration (mEq/L) |
|---|---|
| $Na^+$ | 135 |
| $K^+$ | 4 |
| $Ca^{2+}$ | 3 |
| $Mg^{2+}$ | 1 |
| $Cl^-$ | 113 |
| $HCO_3^-$ | 25 |
| $Citrate^{3-}$ | 5 |

Human serum albumin solution: KENKETSU Albumin 25-NICHIYAKU (NIHON PHARMACEUTICAL CO., LTD.) having the composition shown Table 9 below.

[Table 9]

Table 9 Componential composition of human serum albumin solution

| Component | Concentration (mg/mL) | Concentration (mM) |
|---|---|---|
| Human serum albumin | 250 | - |

(continued)

| Component | Concentration (mg/mL) | Concentration (mM) |
|---|---|---|
| Sodium acetyltryptophan | 5.472 | 20.3 |
| Sodium caprylate | 3.395 | 20.4 |
| (Note) Concentration of sodium ions in solution: 40.7 mEq/L | | |

Washing solution: Obtained by adding 50 mL of a 25% (w/v) human serum albumin solution (NIHON PHARMACEUTICAL CO., LTD.) to 1,000 mL of a bicarbonate Ringer's solution (BICARBON infusion solution, AY Pharmaceuticals Co., Ltd.) so that the final concentration of human serum albumin became 1.19% (w/v). The composition of the washing solution is shown in Tables 10 and 11.

[Table 10]

Table 10 Componential composition of washing solution

| Component | Concentration (g/L) | Concentration (mM) |
|---|---|---|
| Sodium chloride | 5.85 | 100 |
| Potassium chloride | 0.286 | 3.83 |
| Calsium chloride dihydrochloride | 0.21 | 1.43 |
| Magnesium chloride hexahydrate | 0.097 | 0.477 |
| Sodium hydrogen carbonate | 2.00 | 23.8 |
| Sodium citrate dihydrate | 0.467 | 1.59 |
| Human serum albumin | 11.9 | - |
| Sodium acetyltryptophan | 0.261 | 0.967 |
| Sodium caprylate | 0.162 | 0.971 |

[Table 11]

Table 11 Concentration of electrolyte contained in washing solution (excluding albumin)

| Component | Concentration (mEq/L) |
|---|---|
| $Na^{+}$ | 130.5 |
| $K^{+}$ | 3.83 |
| $Ca^{2+}$ | 2.86 |
| $Mg^{2+}$ | 0.952 |
| $Cl^{-}$ | 107.6 |
| $HCO_3^{-}$ | 23.8 |
| $Citrate^{3-}$ | 4.76 |
| Sodium acetyltryptophan | 0.967 |
| Sodium caprylate | 0.971 |

Cytoprotective solution: Obtained by mixing a bicarbonate Ringer's solution (BICARBON infusion solution, AY Pharmaceuticals Co., Ltd.), 25% human serum albumin solution, and dimethyl sulfoxide (DMSO, Mylan Inc.) at a volume ratio of 11:9:5.

[Table 12]

Table 12 Concentration of cytoprotective solution

| Component | mM |
|---|---|
| Sodium chloride | 46.2 |
| Potassium chloride | 1.77 |
| Calcium chloride dihydrochloride | 0.66 |
| Magnesium chloride hexahydrate | 0.22 |
| Sodium hydrogen carbonate | 11 |
| Sodium citrate dihydrate | 0.73 |
| Human serum albumin | (90) |
| Sodium acetyltryptophan | 7.31 |
| Sodium caprylate | 7.34 |
| DMSO | (20) |
| (Note) Unit of concentration of human serum albumin is g/L, and unit of concentration of DMSO is % (v/v) | |

[Example 2: Acquisition of Dental Pulp from Extracted Human Tooth]

One extracted human tooth (third molar tooth) acquired by obtaining informed consent was lightly washed with a bicarbonate Ringer's solution (BICARBON Injection), and then the surface of the extracted tooth was sterilized with a 1% chlorhexidine gluconate solution. Subsequently, the extracted tooth was crushed to excise dental pulp from other tissues.

[Example 3: Enzymatic Treatment of Dental Pulp]

After adding protease solution diluted with DMEM Low Glucose by about 10 times to the dental pulp obtained in Example 2, the dental pulp was allowed to stand in a water bath set at 37°C until it was visually observed that tissues were sufficiently decomposed.

Next, the total amount of cells after the enzymatic treatment was transferred to a centrifuge tube, a DMEM (20% FBS) streptomycin medium was added thereto to stop the enzymatic reaction, and the resultant was centrifuged to precipitate tissue pieces, the cells, and the like. A supernatant was removed, a DMEM (20% FBS) streptomycin medium was added to the obtained precipitate to suspend the tissue pieces, the cells, and the like, and the suspension was centrifuged again to precipitate the cells and the like. A DMEM (20% FBS) streptomycin medium was added to the precipitate, the cells were gently suspended through pipetting to obtain a dental pulp suspension containing tissue pieces, dental pulp-derived cells, and the like.

[Example 4: Culture of Dental Pulp Suspension]

The dental pulp suspension prepared in Example 3 was added to a cell culture plate to start culture at 37°C in the presence of 5% $CO_2$. The culture was continued until a colony formed on the plate was visually observed while replacing the DMEM (20% FBS) streptomycin medium every 2 to 4 days.

[Example 5: Culture of Dental Pulp-Derived Cells]

The medium was removed from the cell culture plate in which the colony was visually observed, a trypsin-EDTA solution was added to the plate so that the surfaces of cells are covered with the solution, and the plate was allowed to stand for 5 to 10 minutes at 37°C and peeled off. Subsequently, a DMEM (10% FBS) medium was added to the cell culture plate to stop the reaction and suspend the cells. This cell suspension was collected in a centrifuge tube. The collected cells were centrifuged (300 × g, 5 minutes) and precipitated, and a supernatant was removed. A DMEM (10% FBS) medium was added to the centrifuge tube, and the cells were suspended to obtain a cell suspension.

After measuring the number of live cells contained in the cell suspension, the cells were seeded in the cell culture plate so as to have a cell density of 3,000 to 20,000 cells/cm$^2$, and culture at 37°C was started in the presence of 5% $CO_2$. The culture was continued until the cells became confluent on the plate while replacing the DMEM (10% FBS) medium every 2 to 4 days.

After removing the medium from the cell culture plate on which the cells became confluent, a trypsin-EDTA solution was added to the plate so that the surfaces of the cells were covered with the solution. The plate was allowed to stand for 5 to 10 minutes at 37°C to peel off the cells. Subsequently, the same amount of a DMEM (10% FBS) medium as the trypsin-EDTA solution was added to the plate to stop the reaction and suspend the cells. This cell suspension was collected in a centrifuge tube. The collected cells were centrifuged (300 × g, 5 minutes) and precipitated to remove a supernatant, and a DMEM (10% FBS) medium was added thereto to suspend the cells.

The above-described collection and culture of cells were repeated, and the cells were proliferated until the number of live cells became 1 × 10$^8$ or more. The number of cell divisions (that is, the number of divisions in an in vitro environment) during this culture period was 16 and 17, and the average doubling time of the cells was within 2 days (48 hours) throughout the culture period.

[Example 6: Collection of Dental Pulp-Derived Cells (Intermediate Cells)]

The medium was removed from the cell culture plate on which the cells became confluent in the last culture in Example 5, and a trypsin-EDTA solution was added to the plate so that the surfaces of the cells were covered with the solution. The plate was allowed to stand for 5 to 10 minutes at 37°C to peel off the cells. Subsequently, the same amount of a DMEM (10% FBS) medium as the trypsin-EDTA solution was added to the cell culture plate to stop the reaction and suspend the cells. This cell suspension was collected in a container and centrifuged and to precipitate the cells, and a supernatant was removed. 500 mL to 1,000 mL of a washing solution was added to the container to wash the cells, and the cells were suspended. Thereafter, the suspension was centrifuged again to precipitate the cells, and a supernatant was removed. This washing operation of cells was repeatedly performed, and finally the cells were collected as precipitates after centrifugation. The cells obtained in this manner were regarded as intermediate cells.

[Example 7: Freezing of Dental Pulp-Derived Cells (Intermediate Cells)]

A washing solution and a cytoprotective solution were added to the precipitated cells prepared in Example 6, and the cells were suspended so that the cell concentration became 11 × 10$^6$ cells/mL (±5%). A cryopreservation vial (material: cyclic olefin copolymer, Sterile Closed Vial, Aseptic Technologies) was filled with the cell suspension obtained in this manner and sealed. This cell suspension was regarded as an intermediate cell suspension. The components contained in the solution portion (cryopreservation liquid for intermediate cells) of the intermediate cell suspension are shown in Table 13. The intermediate cell suspension was frozen with a program freezer and was then transferred to and preserved in a liquid nitrogen storage container. This frozen intermediate cell suspension was regarded as an intermediate cell-frozen product.

[Table 13]

Table 13 Componential composition of cryopreservation liquid for intermediate cells

| Component | mM (Range) | mM (Suitable example) |
|---|---|---|
| Sodium chloride | 65.8 to 80.4 | 73.1 |
| Potassium chloride | 2.52 to 3.08 | 2.80 |
| Calcium chloride dihydrochloride | 0.95 to 1.16 | 1.05 |
| Magnesium chloride hexahydrate | 0.315 to 0.385 | 0.35 |
| Sodium hydrogen carbonate | 15.67 to 19.15 | 17.41 |
| Sodium citrate dihydrate | 1.04 to 1.28 | 1.16 |
| Human serum albumin | (46 to 56) | (51) |

(continued)

| Component | mM (Range) | mM (Suitable example) |
|---|---|---|
| Sodium acetyltryptophan | 3.73 to 4.55 | 4.14 |
| Sodium caprylate | 3.74 to 4.58 | 4.16 |
| DMSO | (9 to 11) | (10) |
| (Note) Unit of concentration of human serum albumin is g/L, and unit of concentration of DMSO is % (v/v) | | |

[Example 8: Thawing of Dental Pulp-Derived Cells (Intermediate Cells)]

The vial filled with the dental pulp-derived cells (intermediate cells) prepared in Example 7 was taken out of the liquid nitrogen storage container and thawed by heating in a water bath at 36.5°C to 37.5°C. The thawed cell suspension was transferred to a centrifuge tube, and 30 mL of a DMEM (10% FBS) medium was added thereto to suspend the cell suspension. The cells were centrifuged (300 × g, 5 minutes) and precipitated, and a supernatant was removed. Subsequently, 20 mL of a DMEM (10% FBS) medium was added thereto to suspend the intermediate cells.

[Example 9: Production Culture (Preculture) of Dental Pulp-Derived Cells]

After measuring the number of intermediate live cells contained in the cell suspension prepared in Example 8, the cells were seeded in the cell culture plate so as to have a density of 20,000 cells/cm$^2$, and culture at 37°C was started in the presence of 5% $CO_2$. The culture was continued until the cells became confluent on the cell culture plate while replacing the DMEM (10% FBS) medium every 2 to 4 days. The viability (number of live cells / number of all cells × 100%) of the cells contained in the thawed intermediate cells which were calculated from the number of live cells was approximately 85% to 95%.

The medium on the cell culture plate on which the cells became confluent was removed, a trypsin-EDTA solution was added to the plate so that the surfaces of the cells were covered with the solution, and the plate was allowed to stand for 5 to 60 minutes at 37°C to peel off the cells. Subsequently, the same amount of a DMEM (10% FBS) medium as the trypsin-EDTA solution was added to the cell culture plate to stop the reaction and suspend the cells. The cell suspension was collected in a container and centrifuged to precipitate the cells, and a supernatant was removed. A DMEM (10% FBS) medium was added thereto to suspend the cells, the suspension was then centrifuged again to precipitate the cells, and a supernatant was removed. A DMEM (10% FBS) medium was added thereto to suspend the cells again.

After measuring the number of live cells contained in the cell suspension, the cells were seeded in the cell culture plate so as to have a density of less than or equal to 20,000 cells/cm$^2$, and culture at 37°C was started in the presence of 5% $CO_2$. The culture was continued until the cells became confluent on the plate while replacing the DMEM (10% FBS) medium every 2 to 4 days.

The above-described collection and culture of cells were repeated, and the cells were proliferated until the number of live cells became $1 \times 10^9$ or more. The number of cell divisions during this culture period of these intermediate cells on the cell culture plate was 5 and 6, and the average doubling time of the cells was within 2 days (48 hours) throughout the culture period.

The medium on the cell culture plate on which the cells became confluent was removed, and a trypsin-EDTA solution was added thereto. The plate was allowed to stand for 5 to 60 minutes at 37°C to peel off the cells. Subsequently, a DMEM (10% FBS) medium was added to the cell culture plate to stop the reaction and suspend the cells. This cell suspension was collected in a container. The collected cells were centrifuged and precipitated, and a supernatant was removed. 500 mL to 600 mL of a DMEM (10% FBS) medium was added thereto to suspend the cells, the suspension was then centrifuged again to precipitate the cells, and a supernatant was removed. 500 mL to 600 mL of a DMEM (10% FBS) medium was added thereto to suspend the cells again.

[Example 10: Production Culture of Dental Pulp-Derived Cells (Preparation of Bioreactor)]

50 g (±1.0 g) of microcarriers were weighed with an electronic balance and placed in a reagent bottle, 2,000 mL of PBS was added thereto, and then sterilization was performed in an autoclave. The microcarriers used were gelatin microcarriers for cell culture which have particle diameters of 130 to 180 μm (during hydration) and have heat resistance so that the microcarriers can be subjected to sterilization treatment in an autoclave.

A 50 L bag for a reactor, a vent filter, and a sensor loop were set in a bioreactor, and a temperature sensor, a pH meter, and a dissolved oxygen meter were further installed therein. 10 L of a DMEM (10% FBS) medium and 50 g (dry weight) of microcarriers were added to (the bag of) the bioreactor, and the bioreactor was heated at 37°C while stirring the microcarriers. The stirring was performed using an impeller provided in the device.

[Example 11: Production culture of Dental Pulp-Derived Cells (Adhesion Step of Cells on Microcarriers)]

After measuring the number of live cells contained in the cell suspension prepared in Example 9, the cells were added to the bioreactor so as to have a density of 1,000 to 5,000 cells/cm$^3$. After stirring inside the reactor for several minutes, the stirring was stopped and the reactor was allowed to stand for 1 hour or longer. These stirring and standing were repeatedly performed to make the cells adhere to the microcarriers.

[Example 12: Production Culture (Cell Culture Step) of Dental Pulp-Derived Cells]

After the completion of the adhesion step, culture was started while stirring the inside of the bioreactor. The inside of the bioreactor was periodically observed during the culture. In a case where the microcarriers had settled to a bottom portion of the reactor, the rotation frequency of the agitation in the reactor was increased so that the microcarriers were made to float in the medium.

The production culture was performed until the cells proliferate until the number of live cells became $5 \times 10^9$ or more according to measurement described in Example 13. The number of cell divisions during the culture period on the microcarriers was 2 and 3, and the average doubling time of the cells was approximately 3 to 6 days. The cells which had undergone the production culture had undergone at least 23 cell divisions in an in vitro environment, and had undergone 23 to 27 cell divisions.

[Example 13: Production Culture of Dental Pulp-Derived Cells (Measurement of Number of Cells)]

30 to 40 mL of the culture solution containing the microcarriers was collected from the reactor, transferred to a centrifuge tube, and was allowed to stand for 5 minutes or longer to allow the microcarriers to settle, and then a supernatant was removed. 25 mL of PBS was added thereto to suspend the cells, and the suspension was allowed to stand for 5 minutes or longer to remove a supernatant. This operation was performed once again. After the removal of the supernatant, a trypsin-EDTA solution was added to the microcarriers. After the mixture was shaken in a water bath at 37°C for 5 to 10 minutes, the same amount of a DMEM (10% FBS) medium as the trypsin-EDTA solution was added thereto to stop the reaction and suspend the cells. This cell suspension was centrifuged ($300 \times g$, 5 minutes) to precipitate the cells and remove a supernatant. 1 to 5 mL of a DMEM (10% FBS) medium was added thereto to suspend the cells, and then the number of live cells contained in the cell suspension was measured.

[Example 14: Production culture of Dental Pulp-Derived Cells (Measurement of Glucose concentration and Lactic acid concentration)]

5 to 40 mL of the cell suspension in the culture was collected from the reactor, transferred to a centrifuge tube, and allowed to stand for 5 minutes or longer to allow the microcarriers to settle. Then, a part of a supernatant was transferred to a new 1.5 mL tube. 50 $\mu$L of the supernatant was collected using a microsyringe, and the concentrations of glucose and lactic acids in the supernatant were measured with a biosensor (BF-7D, Oji Scientific Instruments). In a case where the concentration of glucose was low, the medium was supplemented with glucose or all or part of the medium was replaced so that the concentration of glucose did not fall below 0.1 mM. In addition, in a case where the concentration of lactic acids increased, all or part of the medium was replaced so that the concentration of lactic acids did not exceed 20 mM.

[Example 15: Collection of Dental Pulp-Derived Cells Obtained in Production Culture]

After confirming that the cells proliferated until the number of cells reached a predetermined level, the stirring of the reactor and the sensor loop was stopped, and the cell suspension was allowed to stand for 10 minutes or longer to allow the microcarriers to settle. After removing as much supernatant as possible, the cells were suspended in the remaining medium. The cell suspension was collected in a 10 L bag (Thermo Fisher Scientific Inc.) and allowed to stand for 5 minutes or longer to allow the microcarriers to settle, and then a supernatant was removed.

DMEM Low Glucose was added thereto to suspend the microcarriers, the suspension was allowed to stand for 5 minutes or longer to allow the microcarriers to settle, and then a supernatant was removed. This operation was repeated several times. Subsequently, a trypsin-EDTA solution was added to the microcarriers after the removal of the supernatant. After adding the trypsin-EDTA solution thereto, the mixture was shaken in a water bath at 37°C for 30 to 60 minutes to peel off the cells from the microcarriers and decompose the microcarriers.

In order to remove fragments, cell aggregations, and the like of the microcarriers remaining in the cell suspension, the collected filtrate was passed through a filter having a pore diameter of 20 to 35 μm to collect the cells from the filtrate of the filter. The collected filtrate was centrifuged to precipitate the cells, and a supernatant was removed. The washing solution prepared in Example 1 was added thereto to wash the cells, and the cells were suspended. The suspension was centrifuged again to precipitate the cells, and a supernatant was removed. This washing operation was repeatedly performed, and finally the cells were collected as precipitates after centrifugation.

[Example 16: Formulating and Cryopreserving Dental Pulp-Derived Cells]

A washing solution and a cytoprotective solution were added to the precipitated cells collected in Example 15, and the cells were suspended so that the cell concentration became 25 to 31 $\times$ $10^6$ cells/mL. A cryopreservation vial (material: cyclic olefin copolymer, Sterile Closed Vial, Aseptic Technologies) was filled with the cell suspension obtained in this manner and sealed. This cell suspension was regarded as a pre-freezing dental pulp-derived cell preparation. The components contained in the solution portion (cryopreservation liquid for preparation cells) of the pre-freezing dental pulp-derived cell preparation are shown in Table 14.

[Table 14]

Table 14 Componential composition of cryopreservation liquid for preparation cells

| Component | mM (Range) | mM (Suitable example) |
|---|---|---|
| Sodium chloride | 65.8 to 80.4 | 70.8 |
| Potassium chloride | 2.52 to 3.08 | 2.71 |
| Calcium chloride dihydrochloride | 0.95 to 1.16 | 1.01 |
| Magnesium chloride hexahydrate | 0.315 to 0.385 | 0.34 |
| Sodium hydrogen carbonate | 15.67 to 19.15 | 16.9 |
| Sodium citrate dihydrate | 1.04 to 1.28 | 1.13 |
| Human serum albumin | (46 to 56) | (53.6) |
| Sodium acetyltryptophan | 3.73 to 4.55 | 4.36 |
| Sodium caprylate | 3.74 to 4.58 | 4.37 |
| DMSO | (9 to 11) | (10.7) |
| (Note) Unit of concentration of human serum albumin is g/L, and unit of concentration of DMSO is % (v/v) | | |

The pre-freezing dental pulp-derived cell preparation, with which the vial was filled, was frozen using a program freezer through a usual method and was then transferred to a liquid nitrogen storage container for preservation. This frozen cell suspension was regarded as a preparation (dental pulp-derived cell preparation) containing dental pulp-derived cells as active components.

[Example 17: Properties (such as Cell Morphology) of Dental Pulp-Derived Cells)]

The intermediate cell-frozen product prepared in Example 7 and the dental pulp-derived cell preparation prepared in Example 16 were thawed, 10 mL of a DMEM (10% FBS) medium was added thereto, and each suspension was gently shaken. Thereafter, each suspension was centrifuged (1,500 rpm, 5 minutes) to precipitate the cells. A supernatant was removed, 10 mL of a DMEM (10% FBS) medium was added thereto to suspend the cells, the suspension was then centrifuged (1,500 rpm, 5 minutes) again to precipitate the cells. Subsequently, the cells were suspended in a DMEM (10% FBS) medium, seeded in a cell culture plate so as to have a density of 5,000 to 10,000 cells/cm$^2$, and cultured so as to become 90% to 100% confluent. The cell morphology on the cell culture plate was observed with a phase contrast microscope. The cultured cells contained in the intermediate cell-frozen product and the dental pulp-derived cell preparation were all substantially spindle-shaped adhered cells, and did not include cells with other shapes. The viability (number of live cells / number of all cells $\times$ 100%) of the cells contained in the thawed intermediate cell-frozen product and dental pulp-derived cell preparation which were calculated from the number of live cells was approximately 85% to 95%.

[Example 18: Properties (Particle Diameter) of Dental Pulp-Derived Cell Preparation]

The intermediate cell-frozen product prepared in Example 7 and the dental pulp-derived cell preparation prepared in Example 16 were thawed and respectively diluted with D-PBS(-) solutions to prepare cell-diluted solutions at about $5 \times 10^6$ cells/mL. The cell particle diameter was measured through an image analysis method in which an automated cell viability analyzer (Vi-Cell XR, Beckman Coulter Inc.) was used. The image analysis method in which the automated cell viability analyzer is used will be outlined below. Trypan blue is added to a cell-diluted solution to stain cells, this cell-diluted solution is sent through an elongated flow path, and a plurality of microscopic magnified images of the flow path are photographed. Since trypan blue permeates through cell membranes of dead cells and stains the dead cells, cells observed as colored particles on the images are measured as dead cells and cells observed as transparent particles are measured as live cells. The cell particle diameter is calculated using the diameter of a particle measured on the image and the magnification of the microscope. An average value is obtained from values of particle diameters on all the photographed images.

(Results)

An average value of the cell particle diameters (diameters of the cells) of the thawed intermediate cell-frozen product was 17.36 $\mu$m $\pm$ 1.40 $\mu$m (average value $\pm$ SD, n=3), and an average value of the cell particle diameters of the thawed dental pulp-derived cell preparation was 16.98 $\pm$ 1.23 $\mu$m (average value $\pm$ SD, n=3) (Fig. 1).

[Example 19: Properties (Ability to Differentiate into Osteocytes) of Dental Pulp-Derived Cells]

The ability to differentiate into osteocytes was examined through the following method with reference to disclosure or the like of Pittenger MF., et al., Science. 284, 143-7 (1999) and Colter DC., et al., Proc Natl Acad Sci USA. 98, 7841-5 (2001).

The intermediate cell-frozen product prepared in Example 7 and the dental pulp-derived cell preparation prepared in Example 16 were each thawed and centrifuged (1,500 rpm, 5 minutes) to precipitate the cells. Subsequently, the cells were suspended in a DMEM (10% FBS) medium, seeded in a cell culture plate so as to have a density of 5,000 to 15,000 cells/cm$^2$, and cultured so as to become 90% to 100% confluent. The cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells was measured. The cells were seeded in a 24-well cell culture plate coated with collagen I at a cell density (15,000 to 25,000 cells/cm$^2$) in the DMEM (10% FBS) medium and were each cultured for 3 days. The cells were divided into 2 groups. The medium of one group was replaced with an osteocyte differentiation-inducing medium which was obtained by adding Supplements and Growth Factors for Differentiation into Osteocytes (LONZA KK.) containing dexamethasone, L-glutamine, ascorbate, penicillin/streptomycin, mesenchymal cell growth supplement (MCGS), and a $\beta$-glycerophosphate to Basal Medium for Differentiation into Osteocytes (LONZA KK.) to perform differentiation culture for 2 to 3 weeks while replacing the medium every 3 to 4 days. This group was regarded as an osteocyte differentiation-inducing group. The medium of the other group was replaced with a fresh DMEM (10% FBS) medium to perform culture for 2 to 3 weeks while replacing the medium every 3 to 4 days. This group was regarded as a control group. The cells of each of the osteocyte differentiation-inducing group and the control group were cultured, washed once with PBS, 0.4 mL of 10% formic acid was added to each well, and the wells were allowed to stand for 1 hour at room temperature to release calcium accumulated in the cells from the cells. The concentration of calcium released was quantitatively determined with Calcium E-Test Wako (Wako Pure Chemical Industries, Ltd.)

The measurement results are shown in Fig. 2. In both the cells contained in the intermediate cell-frozen product and the dental pulp-derived cell preparation, a more increase in the concentration of calcium in the cells was recognized in the osteocyte differentiation-inducing groups than in the control groups. These results show that both the cells contained in the intermediate cell-frozen product and the dental pulp-derived cell preparation have been differentiated into osteocytes and show that the dental pulp-derived cells of the present invention have an ability to differentiate into osteocytes.

[Example 20: Properties (Ability to Differentiate into Chondrocytes) of Dental Pulp-Derived Cells]

The ability to differentiate into chondrocytes was examined through the following method with reference to disclosure or the like of Kiani C., et al., Cell Res. 12 19-32 (2002) and Aung A., et al., Arthritis Rheum. 63 148-58 (2011).

The intermediate cell-frozen product prepared in Example 7 and the dental pulp-derived cell preparation prepared in Example 16 were each thawed and centrifuged (1,500 rpm, 5 minutes) to precipitate the cells. Subsequently, the cells were suspended in a DMEM (10% FBS) medium, seeded in a cell culture plate so as to have a density of 5,000 to 10,000 cells/cm$^2$, and cultured so as to become 90% to 100% confluent. The cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells was measured. The cells were divided into 2 groups. The cells of one group were suspended in a chondrocyte differentiation-inducing medium Stem MACS (registered trademark) ChondroDiff Medium (Miltenyi Biotec) at a concentration of $1 \times 10^6$ cells/mL, and 1 mL each of the suspension was seeded in a low adsorptive container (STEMFULL (registered trademark), Sumitomo Bakelite Co., Ltd.) to form spheroids. Differentiation culture was performed for 2 to 3 weeks while replacing the medium every 3 to 4 days. This group was regarded as a chondrocyte differentiation-inducing group. The cells of the other group were subcultured with a DMEM (10% FBS) medium, which was used as a control group.

After the culture, the cells were collected and treated with Proteinase K for 30 minutes in a warm bath at 55°C to lyse the cells. Total RNA was extracted from the lysed cells with RNeasy (Registered trademark) Plus Mini Kit (QIAGEN N.V.) to prepare a total RNA extract, and then the concentration of RNA contained in the total RNA extract was measured with an absorptiometer (DeNovix Inc.) After measuring the concentration of RNA, cDNA was synthesized using QuantiTect (Registered trademark) Reverse Transcription Kit (QIAGEN N.V.) Furthermore, a PCR reaction solution was prepared, 25 ng of cDNA of each group was used as a template, real-time RT-PCR was performed under PCR conditions of [(50°C / 2 minutes) $\times$ 1 cycle, (95°C / 10 minutes) $\times$ 1 cycle, and (95°C / 15 seconds, 60°C / 1 minute) $\times$ 40 cycles] to amplify the aggrecan gene and the $\beta$-actin gene. An aggrecan probe (Applied Biosystems / Assay ID: Hs00153936_m1) and a $\beta$-actin probe (Applied Biosystems / 4310881E) were respectively used as PCR primers.

The measurement results are shown in Fig. 3. In both the cells contained in the intermediate cell-frozen product and the dental pulp-derived cell preparation, it was found that the threshold cycle (Ct) values of aggrecan were higher in the chondrocyte differentiation-inducing groups than in the control groups. These results show that the expression level of aggrecan which is a main molecule constituting the extracellular matrix of chondrocytes has increased in the chondrocyte differentiation-inducing groups and show that both the cells contained in the intermediate cell-frozen product and the dental pulp-derived cell preparation have the ability to differentiate into chondrocytes.

[Example 21: Properties of Dental Pulp-Derived Cells (Measurement 1 of Surface Antigens)]

The dental pulp-derived cells had a substantially spindle shape similarly to human mesenchymal stem cells. Therefore, the presence or absence of expression of surface antigen markers CD73, CD90, CD105, and CD166 for which human mesenchymal stem cells are known to be positive and surface antigen markers CD34 and CD45 for which mesenchymal stem cells are known to be negative was examined using a flow cytometer.

Phosphate-buffered physiological saline at a pH of 7.4 which contains BSA and is powder (SIGMA-Aldrich) was dissolved in pure water and passed through a 0.45 $\mu$m filter to prepare PBS-B [0.01 M phosphate-buffered physiological saline (pH 7.4) containing 0.138 M sodium chloride, 0.0027 M potassium chloride, and 1% (w/v) bovine serum albumin.] IgG from Human (SIGMA-Aldrich) was dissolved in PBS (Life Technologies) and passed through a 0.45 $\mu$m filter to prepare a 20 mg/mL IgG solution. 2 mL of the 20 mg/mL IgG solution was added to 18 mL of PBS-B to prepare a blocking solution.

In addition, FITC-labeled anti-human CD34 antibody (anti-CD34-FITC, Nippon Becton Dickinson Company, Ltd.) was used as an anti-CD34 antibody, FITC-labeled anti-human CD45 antibody (anti-CD45-FITC, Beckman Coulter Inc.) was used as an anti-CD45 antibody, FITC-labeled anti-human CD73 antibody (anti-CD73-FITC, Nippon Becton Dickinson Company, Ltd.) was used as an anti-CD73 antibody, FITC-labeled anti-human CD90 antibody (anti-CD73-FITC, Nippon Becton Dickinson Company, Ltd.) was used as an anti-CD90 antibody, PE-labeled anti-human CD105 antibody (anti-CD105-R-PE, Nippon Becton Dickinson Company, Ltd.) was used as an anti-CD105 antibody, PE-labeled anti-human CD166 antibody (anti-CD 166-PE, Ancell Corporation) was used as an anti-CD166 antibody, FITC-labeled mouse IgG1 isotype control (anti-IgG1-FITC, Beckman Coulter Inc.) and PE-labeled mouse IgG1 isotype control (IgG1-PE, Beckman Coulter Inc.) were used as control antibodies.

(Method for Staining Cells)

The intermediate cell-frozen product prepared in Example 7 and the dental pulp-derived cell preparation prepared in Example 16 were each thawed and centrifuged (1,500 rpm, 5 minutes) to precipitate the cells. A supernatant was removed, 10 mL of a DMEM (10% FBS) medium was added thereto to suspend the cells, and the number of live cells was measured. ($1 \times 10^7$) Cells were collected in a 50 mL centrifuge tube, centrifuged (1,500 rpm, 5 minutes), and precipitated, and a supernatant was removed. PBS-B was added thereto to make a total volume of 10 mL and suspend the cells. Then, the cells were centrifuged (1,500 rpm, 5 minutes) again to precipitate the cells, and a supernatant was removed. Subsequently, the cells were suspended in 1 mL of the blocking solution and were allowed to stand on ice for 1 hour. Each antibody solution was added to nine 5-mL reaction tubes (numbers (1) to (9)) as shown in Table 15.

Subsequently, 100 μL of the suspension of the cells which have been suspended in the blocking solution was added to each tube, and the mixture was gently shaken and allowed to stand on ice for 20 minutes to bind antibodies contained in each antibody solution to surface antigen markers expressed on the surfaces of the cells. Subsequently, 3 mL of PBS-B was added to each tube and mixed, the cells were then centrifuged (1,500 rpm, 5 minutes) and precipitated, and a supernatant was removed to remove antibodies which had not been bound to the cells. This operation of removing antibodies was repeated 3 times. 400 μL of PBS-B was added to each tube to suspend the cells.

[Table 15]

Table 15 Type and Amount of Antibody Solution Added to Each Reaction Tube

| Tube number | Antibody solution | Addition amount (μL) |
|---|---|---|
| (1) | PBS-B | - |
| (2) | IgG1-FITC | 20 |
| (3) | Anti-CD34-FITC | 20 |
| (4) | Anti-CD45-FITC | 20 |
| (5) | Anti-CD73-FITC | 5 |
| (6) | Anti-CD90-FITC | 2 |
| (7) | IgG1-PE | 20 |
| (8) | Anti-CD105-FITC | 2 |
| (9) | Anti-CD 166-FITC | 20 |

(Measurement and Analysis)

Regarding the cells of the tube numbers (1) to (9), the amount of each fluorescent dye of FITC and PE was measured with BD FACSVerse (registered trademark) (Nippon Becton Dickinson Company, Ltd.) to obtain the amounts of fluorescent dyes bound to the surfaces of the cells through antibodies specifically bound to the surface antigens by comparison with the negative controls. The tube (2) is a negative control of the tubes (3) to (6), and the tube (7) is a negative control of the tubes (8) and (9). The tube (1) is unstained cells.

The measurement results are shown in Fig. 4. As for the cells of the tube numbers (5), (6), (8), and (9) (which were respectively CD73-, CD90-, CD105-, and CD166-stained cells), 85% or more of the cells were positive for these surface antigens in both the cells contained in the intermediate cell-frozen product and the dental pulp-derived cell preparation. As for the cells of the tube numbers (3), and (4) (which were respectively CD34- and CD45-stained cells), most of the cells were negative for these surface antigens in both the cells contained in the intermediate cell-frozen product and the dental pulp-derived cell preparation. These results show that the dental pulp-derived cells are positive for CD73, CD90, CD105, and CD166 and negative for CD34 and CD45.

[Example 22: Properties of Dental Pulp-Derived Cells (Measurement 2 of Surface Antigens)]

The intermediate cell-frozen product prepared in Example 7 and the dental pulp-derived cell preparation prepared in Example 16 were each thawed, and the total amount of each cell suspension was added to a DMEM medium. The cell suspension was centrifuged (300 × g, 5 minutes, room temperature), and a supernatant was removed. Thereafter, a DMEM medium was added thereto to suspend the cells. Subsequently, the cells were stained with antibodies against surface antigens using BD Lyoplate (Human Cell Screening Marker Screening Panel, Nippon Becton Dickinson Company, Ltd.) The staining with antibodies was carried out according to the protocol of Human Cell Screening Panel (Nippon Becton Dickinson Company, Ltd.) The outline of the method for measuring surface antigens using Human Cell Screening Panel will be described below. A cell suspension is dispensed into each well of a 96-well plate. Antibodies against various surface antigens are respectively added to the wells as primary antibodies and allowed to stand for 30 minutes to bind the surface antigens to the antibodies. The cell suspension is centrifuged to precipitate cells, and a supernatant is removed. After washing the cells, antibodies against the primary antibodies are respectively added to the wells as secondary antibodies and allowed to stand for 30 minutes to bind the surface antigens to the antibodies. The secondary antibodies are fluorescently labeled with Alexa Fluor 647. The cell suspension is centrifuged to precipitate the cells, and a supernatant is removed. The cells are resuspended. Regarding this cell suspension, cells to which the secondary antibodies are bound are fluorescently detected as positive cells through flow cytometry to obtain an abundance ratio of the positive cells of the surface antigens. The measurement was performed on 9 lots of the intermediate cells and 7 lots of the cells contained in the dental pulp-derived cell preparation to obtain the positive ratio for each antigen.

(Results)

Surface antigens for which the intermediate cells and the cells contained in the dental pulp-derived cell preparation were positive are respectively shown in Figs. 5 and 6.

In the intermediate cells, positive rates of 95% or higher were shown for CD47, CD81, CD90, CD147, and HLA-A, -B, and -C in all of the lots. In addition, positive rates of 90% or higher were shown for CD29, CD46, CD55, CD59, CD73, and CD140b in all of the lots. In addition, positive rates of 80% or higher, approximately 90% or higher, were shown for CD9, CD44, CD49b, CD49c, CD98, and EGF-R in all of the lots. In addition, positive rates of 70% or higher, approximately 90% or higher, were shown for CD49f and CD166 in all of the cells. In addition, positive rates of 60% or higher, approximately 90% or higher, were shown for CD10, CD13, CD58, CD63, CD151, and CD164 in all of the cells (Fig. 5). In addition to these, the intermediate cells are also positive for CD105 (Fig. 4).

On the other hand, in the cells contained in the dental pulp-derived cell preparation, positive rates of 95% or higher, approximately 98% or higher, were shown for CD29, CD59, CD44, and CD164 in all of the lots. In addition, positive rates of 90% or higher, approximately 95% or higher, were shown for CD9, CD13, CD46, CD47, CD58, CD63, CD73, CD81, CD90, CD98, CD147, EGF-R, and HLA-A, -B, and -C in all of the lots. In addition, positive rates of 80% or higher were shown for CD49b, CD49c, CD49e, CD55, CD95, CD151, and CD166 in all of the lots, and positive rates of approximately 90% or higher were shown therefor except for CD95, CD151, and CD166. In addition, positive rates of 70% or higher, approximately 80% or higher, were shown for CD10, CD49f, and CD140b in all of the lots (Fig. 6). In addition to these, the cells contained in the dental pulp-derived cell preparation are also positive for CD105 (Fig. 4).

Subsequently, surface antigens for which the intermediate cells and the cells contained in the dental pulp-derived cell preparation were negative are respectively shown in Tables 16 and 17.

In the intermediate cells, positive rates of 1% or lower were shown for CD120b, CD132, CD158a, CD161, CD184, CD195, CD206, CD210, CD212, CD226, CD244, CD267, CD278, CD279, CD282, CD294, NKB1, SSEA-1, TRA-1-60, TRA-1-81, Vβ23, SSEA-3, CLA, and integrin β7 in all of the lots. In addition, positive rates 2% or lower were shown for CD8b, CD11b, CD15s, CD16, CD19, CD24, CD31, CD32, CD62E, CD62P, CD66f, CD86, CD88, CD94, CD100, CD103, CD104, CD114, CD117, CD118, CD121b, CD122, CD123, CD124, CD126, CD127, CD128b, CD135, CD137, CD137ligand, CD150, CD163, CD172b, CD177, CD178, CD180, CD197, CD220, CD229, CD231, CD255, CD268, CD305, CD314, CD321, CDw327, CDw328, CD329, CD335, CD336, BLTR-1, CLIP, CMRF-44, CMRF-56, fMLP-R, Vβ8, Invariant NKT, and yδ TCR in all of the lots, and the approximate positive rates were 1% or lower. In addition, positive rates of 5% or lower were shown for CD1a, CD1b, CD1d, CD2, CD3, CD5, CD6, CD7, CD8a, CD11c, CD15, CD18, CD21, CD22, CD23, CD25, CD27, CD28, CD33, CD35, CD37, CD38, CD41a, CD41b, CD42b, CD45, CD45RB, CD45RO, CD48, CD50, CD53, CD62L, CD64, CD66 (a, c, d, e), CD69, CD70, CD72, CD74, CD84, CD85, CD87, CD89, CDw93, CD97, CD134, CD138, CD141, CD144, CD154, CD158b, CD162, CD183, CD205, CD235a, CD309, CD326, CD337, and αβ TCR in all of the cells. In addition, although positive rates of more than 5% were shown for CD26, CD106, and CD271 in some lots, the average value of the positive rates was 5% or lower (Table 16A to Table 16D). In addition to these, the intermediate cells are also negative for CD34, CD40, CD45, CD80, CD86, and MHC-class II antigen (Figs. 4 and 11A).

In the cells contained in the dental pulp-derived cell preparation, positive rates of 1% or lower were shown for CD1a, CD1d, CD2, CD3, CD4, CD5, CD7, CD8a, CD8b, CD11b, CD11c, CD15, CD15s, CD16, CD18, CD19, CD20, CD21, CD22, CD23, CD24, CD25, CD28, CD30, CD31, CD32, CD33, CD34, CD35, CD37, CD38, CD41a, CD86, CD87, CD88, CD89, CDw93, CD94, CD100, CD102, CD103, CD114, CD117, CD118, CD120b, CD121b, CD122, CD123, CD124, CD126, CD127, CD128b, CD132, CD134, CD135, CD137, CD137ligand, CD138, CD144, CD150, CD153, CD154, CD158a, CD158b, CD161, CD162, CD163, CD172b, CD177, CD178, CD180, CD184, CD195, CD196, CD197, CD205, CD206, CD210, CD212, CD220, CD226, CD229, CD231, CD235a, CD244, CD255, CD267, CD268, CD278, CD279, CD282, CD294, CD305, CD309, CD314, CD321, CDw327, CDw328, CD329, CD335, CD336, CD337, BLTR-1, CLIP, CMRF-44, CMRF-56, fMLP-R, SSEA-1, TRA-1-60, CLA, integrin β7, and Invariant NKT in all of the lots. In addition, positive rates of 2% or lower were shown for CD1b, CD6, CD27, CD41b, CD42a, CD42b, CD43, CD45, CD45RB, CD48, CD50, CD53, CD57, CD62E, CD62L, CD62P, CD64, CD66b, CD66f, CD69, CD70, CD72, CD75, CD84, CD85, CD97, CD99R, CD183, CD193, SSEA-3, and γδ TCR in all of the lots, and the approximate positive rates were 1% or lower. In addition, positive rates of 5% or lower were shown for CD4v4, CD14, CD36, CD45RA, CD45RO, CD66 (a, c, d, e), CD79b, CD83, CD152, CD209, CD275, CD326, MIC A/B, and αβ TCR in all of the lots, and the approximate positive rates were 2% or lower. In addition, although positive rates of more than 5% were shown forCD106, and CD271 in some lots, the average value of the positive rates was 5% or lower (Table 17A to Table 17E). In addition to these, the cells contained in the dental pulp-derived cell preparation are also negative for CD34, CD40, CD45, CD80, CD86, and MHC-class II antigen (Figs. 4 and 11B).

[Table 16A]

Table 16A Surface antigens for which intermediate cells were negative

| Surface antigen | Average of intermediates | SE |
|---|---|---|
| CD1a | 0.49 | 0.32 |
| CD1b | 1.61 | 0.57 |
| CD1d | 0.89 | 0.32 |
| CD2 | 0.79 | 0.33 |
| CD3 | 0.00 | 0.47 |
| CD5 | 0.00 | 0.49 |
| CD6 | 1.15 | 0.44 |
| CD7 | 0.64 | 0.30 |
| CD8a | 0.74 | 0.34 |
| CD8b | 0.00 | 0.55 |
| CD11b | 0.00 | 0.32 |
| CD11c | 0.81 | 0.29 |
| CD15 | 1.04 | 0.46 |
| CD15s | 0.58 | 0.18 |
| CD16 | 0.44 | 0.25 |
| CD18 | 0.67 | 0.37 |
| CD19 | 0.25 | 0.32 |
| CD21 | 0.65 | 0.38 |
| CD22 | 0.67 | 0.32 |
| CD23 | 0.70 | 0.29 |
| CD24 | 0.00 | 0.36 |
| CD25 | 0.86 | 0.33 |
| CD26 | 3.49 | 1.18 |
| CD27 | 0.90 | 0.48 |
| CD28 | 0.88 | 0.44 |
| CD31 | 0.25 | 0.32 |
| CD32 | 0.17 | 0.37 |
| CD33 | 0.57 | 0.37 |
| CD35 | 0.61 | 0.39 |
| CD37 | 0.58 | 0.32 |
| CD38 | 0.91 | 0.41 |
| CD41a | 0.40 | 0.35 |
| CD41b | 0.44 | 0.32 |

(continued)

| Surface antigen | Average of intermediates | SE |
|---|---|---|
| CD42b | 0.39 | 0.32 |
| CD45 | 0.67 | 0.34 |
| CD45RB | 0.59 | 0.29 |

[Table 16B]

Table 16B Surface antigens for which intermediate cells were negative

| Surface antigen | Average of intermediates | SE |
|---|---|---|
| CD45RO | 0.94 | 0.37 |
| CD48 | 0.64 | 0.30 |
| CD50 | 0.84 | 0.50 |
| CD53 | 0.56 | 0.33 |
| CD62E | 0.14 | 0.33 |
| CD62L | 0.33 | 0.31 |
| CD62P | 0.14 | 0.19 |
| CD64 | 0.27 | 0.33 |
| CD66 (a, c, d, e) | 0.06 | 0.40 |
| CD66f | 0.17 | 0.32 |
| CD69 | 0.40 | 0.45 |
| CD70 | 0.17 | 0.33 |
| CD72 | 0.17 | 0.38 |
| CD74 | 0.00 | 0.66 |
| CD84 | 0.42 | 0.39 |
| CD85 | 0.02 | 0.45 |
| CD86 | 0.23 | 0.21 |
| CD87 | 0.66 | 0.46 |
| CD88 | 0.30 | 0.19 |
| CD89 | 0.56 | 0.32 |
| CDw93 | 0.42 | 0.40 |
| CD94 | 0.38 | 0.23 |
| CD97 | 0.67 | 0.27 |
| CD100 | 0.28 | 0.24 |
| CD103 | 0.24 | 0.19 |
| CD104 | 0.22 | 0.19 |
| CD106 | 4.82 | 1.68 |
| CD114 | 0.12 | 0.18 |
| CD117 | 0.38 | 0.23 |

(continued)

| Surface antigen | Average of intermediates | SE |
|---|---|---|
| CD118 | 0.25 | 0.17 |
| CD120b | 0.04 | 0.05 |
| CD121b | 0.13 | 0.19 |
| CD122 | 0.20 | 0.27 |
| CD123 | 0.21 | 0.18 |
| CD124 | 0.21 | 0.25 |
| CD126 | 0.12 | 0.20 |

[Table 16C]

Table 16C Surface antigens for which intermediate cells were negative

| Surface antigen | Average of intermediates | SE |
|---|---|---|
| CD127 | 0.23 | 0.26 |
| CD128b | 0.09 | 0.19 |
| CD132 | 0.19 | 0.05 |
| CD134 | 0.26 | 0.33 |
| CD135 | 0.38 | 0.23 |
| CD137 | 0.22 | 0.26 |
| CD137Ligand | 0.34 | 0.25 |
| CD138 | 0.54 | 0.38 |
| CD141 | 0.81 | 0.37 |
| CD144 | 0.29 | 0.42 |
| CD150 | 0.18 | 0.18 |
| CD154 | 0.26 | 0.26 |
| CD158a | 0.00 | 0.09 |
| CD158b | 0.02 | 0.32 |
| CD161 | 0.02 | 0.20 |
| CD162 | 0.20 | 0.32 |
| CD163 | 0.00 | 0.19 |
| CD172b | 0.21 | 0.25 |
| CD177 | 0.03 | 0.21 |
| CD178 | 0.06 | 0.23 |
| CD180 | 0.16 | 0.27 |
| CD183 | 1.24 | 0.51 |
| CD184 | 0.00 | 0.27 |
| CD195 | 0.00 | 0.27 |
| CD197 | 0.36 | 0.17 |

(continued)

| Surface antigen | Average of intermediates | SE |
|---|---|---|
| CD205 | 0.76 | 0.53 |
| CD206 | 0.00 | 0.17 |
| CD210 | 0.18 | 0.03 |
| CD212 | 0.31 | 0.08 |
| CD220 | 0.03 | 0.19 |
| CD226 | 0.00 | 0.16 |
| CD229 | 0.12 | 0.20 |
| CD231 | 0.00 | 0.20 |
| CD235a | 0.77 | 0.50 |
| CD244 | 0.00 | 0.43 |
| CD255 | 0.16 | 0.18 |

[Table 16D]

Table 16D Surface antigens for which intermediate cells were negative

| Surface antigen | Average of intermediates | SE |
|---|---|---|
| CD267 | 0.20 | 0.05 |
| CD268 | 0.19 | 0.18 |
| CD271 | 3.62 | 0.74 |
| CD278 | 0.00 | 0.10 |
| CD279 | 0.17 | 0.17 |
| CD282 | 0.19 | 0.15 |
| CD294 | 0.06 | 0.05 |
| CD305 | 0.33 | 0.21 |
| CD309 | 0.39 | 0.26 |
| CD314 | 0.35 | 0.21 |
| CD321 | 0.40 | 0.25 |
| CD326 | 2.37 | 0.41 |
| CDw327 | 0.34 | 0.22 |
| CDw328 | 0.42 | 0.22 |
| CD329 | 0.37 | 0.18 |
| CD335 | 0.31 | 0.18 |
| CD336 | 0.20 | 0.18 |
| CD337 | 0.84 | 0.42 |
| $\alpha\beta$ TCR | 1.44 | 0.27 |
| BLTR-1 | 0.33 | 0.22 |
| CLIP | 0.25 | 0.23 |

(continued)

| Surface antigen | Average of intermediates | SE |
|---|---|---|
| CMRF-44 | 0.48 | 0.23 |
| CMRF-56 | 0.26 | 0.23 |
| fMLP-R | 0.44 | 0.20 |
| γδ TCP | 0.48 | 0.19 |
| Invariant NKT | 0.08 | 0.15 |
| NKB1 | 0.22 | 0.12 |
| SSEA-1 | 0.00 | 0.15 |
| TRA-1-60 | 0.00 | 0.12 |
| TRA-1-81 | 0.00 | 0.10 |
| Vβ23 | 0.21 | 0.13 |
| Vβ8 | 0.44 | 0.15 |
| SSEA-3 | 0.11 | 0.05 |
| CLA | 0.07 | 0.05 |
| Integrin β7 | 0.06 | 0.04 |

[Table 17A]

Table 17A Surface antigens for which cells contained in preparation were negative

| Surface antigen | Average of product | SE |
|---|---|---|
| CD1a | 0.00 | 0.85 |
| CD1b | 0.00 | 0.80 |
| CD1d | 0.00 | 0.83 |
| CD2 | 0.00 | 0.73 |
| CD3 | 0.00 | 0.22 |
| CD4 | 0.00 | 0.76 |
| CD4v4 | 0.52 | 0.84 |
| CD5 | 0.00 | 0.24 |
| CD6 | 0.00 | 0.78 |
| CD7 | 0.00 | 0.77 |
| CD8a | 0.00 | 0.85 |
| CD8b | 0.00 | 0.17 |
| CD11b | 0.00 | 0.23 |
| CD11c | 0.00 | 0.80 |
| CD14 | 0.71 | 0.36 |
| CD15 | 0.05 | 0.21 |
| CD15s | 0.00 | 0.21 |
| CD16 | 0.00 | 0.84 |

(continued)

| Surface antigen | Average of product | SE |
|---|---|---|
| CD18 | 0.00 | 0.82 |
| CD19 | 0.00 | 0.75 |
| CD20 | 0.00 | 0.22 |
| CD21 | 0.00 | 0.75 |
| CD22 | 0.00 | 0.76 |
| CD23 | 0.00 | 0.80 |
| CD24 | 0.00 | 0.24 |
| CD25 | 0.00 | 0.78 |
| CD26 | 9.03 | 1.86 |
| CD27 | 0.00 | 0.78 |
| CD28 | 0.00 | 0.81 |
| CD30 | 0.00 | 0.74 |
| CD31 | 0.00 | 0.69 |
| CD32 | 0.00 | 0.21 |
| CD33 | 0.00 | 0.71 |

[Table 17B]

Table 17B Surface antigens for which cells contained in preparation were negative

| Surface antigen | Average of product | SE |
|---|---|---|
| CD34 | 0.00 | 0.58 |
| CD35 | 0.00 | 0.75 |
| CD36 | 0.71 | 0.28 |
| CD37 | 0.00 | 0.75 |
| CD38 | 0.00 | 0.74 |
| CD41a | 0.00 | 0.75 |
| CD41b | 0.00 | 0.29 |
| CD42a | 0.00 | 0.69 |
| CD42b | 0.00 | 0.83 |
| CD43 | 0.00 | 0.78 |
| CD45 | 0.00 | 0.83 |
| CD45RA | 1.10 | 0.44 |
| CD45RB | 0.00 | 0.84 |
| CD45RO | 0.74 | 0.43 |
| CD48 | 0.19 | 0.29 |
| CD50 | 0.29 | 0.38 |
| CD53 | 0.00 | 0.73 |

(continued)

| Surface antigen | Average of product | SE |
|---|---|---|
| CD56 | 2.67 | 1.53 |
| CD57 | 0.13 | 0.29 |
| CD62E | 0.00 | 0.75 |
| CD62L | 0.00 | 0.74 |
| CD62P | 0.00 | 0.79 |
| CD64 | 0.00 | 0.85 |
| CD66 (a, c, d, e) | 0.14 | 0.37 |
| CD66b | 0.43 | 0.33 |
| CD66f | 0.00 | 0.84 |
| CD69 | 0.00 | 0.84 |
| CD70 | 0.00 | 0.31 |
| CD72 | 0.00 | 0.29 |
| CD75 | 0.01 | 0.33 |
| CD79b | 0.91 | 0.93 |
| CD83 | 0.51 | 0.86 |
| CD84 | 0.00 | 0.73 |

[Table 17C]

Table 17C Surface antigens for which cells contained in preparation were negative

| | | |
|---|---|---|
| CD85 | 0.00 | 0.35 |
| CD86 | 0.00 | 0.77 |
| CD87 | 0.00 | 0.79 |
| CD88 | 0.00 | 0.82 |
| CD89 | 0.00 | 0.78 |
| CDw93 | 0.00 | 0.24 |
| CD94 | 0.00 | 0.76 |
| CD97 | 0.00 | 0.90 |
| CD99R | 0.00 | 0.30 |
| CD100 | 0.00 | 0.77 |
| CD102 | 0.00 | 0.20 |
| CD103 | 0.00 | 0.80 |
| CD106 | 4.43 | 0.79 |
| CD114 | 0.00 | 0.77 |
| CD117 | 0.00 | 0.72 |
| CD118 | 0.00 | 0.82 |
| CD120b | 0.00 | 0.07 |

(continued)

| | | |
|---|---|---|
| CD121b | 0.00 | 0.78 |
| CD122 | 0.00 | 0.79 |
| CD123 | 0.00 | 0.78 |
| CD124 | 0.00 | 0.77 |
| CD126 | 0.00 | 0.77 |
| CD127 | 0.00 | 0.78 |
| CD128b | 0.00 | 0.81 |
| CD132 | 0.26 | 0.06 |
| CD134 | 0.00 | 0.80 |
| CD135 | 0.00 | 0.76 |
| CD137 | 0.00 | 0.76 |
| CD137Ligand | 0.00 | 0.79 |
| CD138 | 0.00 | 0.76 |
| CD144 | 0.00 | 0.79 |
| CD146 | 9.80 | 3.52 |
| CD150 | 0.00 | 0.80 |

[Table 17D]

Table 17D Surface antigens for which cells contained in preparation were negative

| Surface antigen | Average of product | SE |
|---|---|---|
| CD152 | 0.35 | 0.50 |
| CD153 | 0.00 | 0.74 |
| CD154 | 0.00 | 0.73 |
| CD158a | 0.00 | 0.14 |
| CD158b | 0.00 | 0.23 |
| CD161 | 0.00 | 0.77 |
| CD162 | 0.00 | 0.74 |
| CD163 | 0.00 | 0.78 |
| CD172b | 0.00 | 0.63 |
| CD177 | 0.00 | 0.77 |
| CD178 | 0.00 | 0.77 |
| CD180 | 0.00 | 0.79 |
| CD183 | 0.00 | 0.79 |
| CD184 | 0.00 | 0.14 |
| CD193 | 0.12 | 0.33 |
| CD195 | 0.00 | 0.16 |
| CD196 | 0.00 | 0.71 |

(continued)

| Surface antigen | Average of product | SE |
|---|---|---|
| CD197 | 0.00 | 0.18 |
| CD205 | 0.00 | 0.22 |
| CD206 | 0.00 | 0.79 |
| CD209 | 1.62 | 0.47 |
| CD210 | 0.00 | 2.02 |
| CD212 | 0.00 | 2.09 |
| CD220 | 0.00 | 0.80 |
| CD226 | 0.00 | 0.78 |
| CD229 | 0.00 | 0.79 |
| CD231 | 0.00 | 0.78 |
| CD235a | 0.00 | 0.22 |
| CD244 | 0.00 | 0.11 |
| CD255 | 0.00 | 0.19 |
| CD267 | 0.00 | 2.11 |
| CD268 | 0.00 | 0.67 |
| CD271 | 4.62 | 0.78 |

[Table 17E]

Table 17E Surface antigens for which cells contained in preparation were negative

| | | |
|---|---|---|
| CD275 | 0.74 | 0.44 |
| CD278 | 0.00 | 0.76 |
| CD279 | 0.00 | 0.73 |
| CD282 | 0.00 | 0.82 |
| CD294 | 0.00 | 2.05 |
| CD305 | 0.00 | 0.78 |
| CD309 | 0.00 | 0.83 |
| CD314 | 0.00 | 0.81 |
| CD321 | 0.00 | 0.75 |
| CD326 | 0.99 | 0.74 |
| CDw327 | 0.00 | 0.77 |
| CDw328 | 0.00 | 0.82 |
| CD329 | 0.00 | 0.83 |
| CD335 | 0.00 | 0.81 |
| CD336 | 0.00 | 0.81 |
| CD337 | 0.00 | 0.74 |
| αβ TCR | 0.95 | 0.27 |

(continued)

| | | |
|---|---|---|
| BLTR-1 | 0.00 | 0.79 |
| CLIP | 0.00 | 0.74 |
| CMRF-44 | 0.27 | 0.18 |
| CMRF-56 | 0.00 | 0.85 |
| fMLP-R | 0.00 | 0.82 |
| γδ TCR | 0.00 | 0.80 |
| Invariant NKT | 0.00 | 0.79 |
| SSEA-1 | 0.00 | 0.12 |
| TRA-1-60 | 0.00 | 0.13 |
| SSEA-3 | 0.96 | 0.21 |
| CLA | 0.14 | 0.13 |
| Integrin β7 | 0.00 | 2.03 |
| MIC A/B | 0.45 | 0.41 |

Surface antigens which had a positive rate difference of at least 10% between the intermediate cells and the cells contained in the dental pulp-derived cell preparation are shown in Fig. 7. In the cells contained in the dental pulp-derived cell preparation obtained by further culturing the intermediate cells through the methods described in Examples 9 to 15, the positive rates of CD39, CD49a, CD61, CD107a, CD107b, and CD143 increased by 20% or more, but the positive rate of CD146 decreased by 60% compared to the intermediate cells. These results show that the cells contained in the dental pulp-derived cell preparation have properties different from the intermediate cells. That is, these results show that cells contained in a dental pulp-derived cell preparation can be obtained as new cells different from the intermediate cells by performing the culture through the methods described in Examples 9 to 15.

[Example 23: Properties of Dental Pulp-Derived Cells (Vascular Endothelial Cell Growth Factor (VEGF) Secretory Ability Test)]

The intermediate cell-frozen product prepared in Example 7 and the dental pulp-derived cell preparation prepared in Example 16 were each thawed and centrifuged (1,500 rpm, 5 minutes) to precipitate the cells. Subsequently, the cells were suspended in a DMEM (10% FBS) medium, seeded in a cell culture plate so as to have a density of 5,000 to 15,000 cells/cm$^2$, and cultured so as to become 90% to 100% confluent. The cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells was measured. After suspending the cells in the DMEM (10% FBS) medium at a concentration of $0.7 \times 10^5$ cells/mL, 10 mL of each suspension was seeded in a T25 cell culture plate and cultured for 5 days. After the culture, the total amount of culture supernatant was collected, the weight of the collected culture supernatant was measured with an electronic balance (Shimadzu Corporation), and the culture supernatant was cryopreserved at -80°C until measurement. After collecting and removing the supernatant, the cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells was measured.

The above-described cryopreserved culture supernatant was thawed, VEGF contained in the culture supernatant was labeled with Quantikine ELISA Human VEGF Kit (R&D Systems) and then detected with a microplate reader (Molecular Devices, LLC.) The obtained detection value was interpolated into a calibration curve created with VEGF at a well-known concentration, the concentration of VEGF contained in the culture supernatant was obtained. The amount of VEGF secreted was calculated as a secretion amount per cell number according to the following equation.

$$[\text{VEGF secretion amount} = \text{VEGF concentration measurement value} \times \text{volume of culture supernatant} / \text{number of live cells } (1 \times 10^5 \text{ cells})]$$

Fig. 8 is an example of the measurement results. These results show that both the intermediate cells and the cells contained in the dental pulp-derived cell preparation have a VEGF secretory ability, but the ability is enhanced in the process of further culturing the intermediate cells to obtain the dental pulp-derived cell preparation. Since VEGF is a substance having an angiogenic effect, the dental pulp-derived cell preparation can promote formation of blood vessels in the body using VEGF by administering this dental pulp-derived cell preparation to humans. In addition, since the VEGF secretory ability is enhanced in the cells contained in the dental pulp-derived cell preparation produced through the intermediate cells, it is thought that the dental pulp-derived cell preparation produced through the production method is effective as a therapeutic agent for diseases for which an angiogenic effect needs to be exerted.

[Example 24: Properties of Dental Pulp-Derived Cells (Prostaglandin $E_2$ ($PGE_2$) Secretory Ability Test)]

The intermediate cell-frozen product prepared in Example 7 and the dental pulp-derived cell preparation prepared in Example 16 were each thawed and centrifuged (1,500 rpm, 5 minutes) to precipitate the cells. Subsequently, the cells were suspended in a DMEM (10% FBS) medium, seeded in a cell culture plate so as to have a density of 5,000 to 15,000 cells/cm$^2$, and cultured so as to become 90% to 100% confluent. The cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells was measured. After suspending the cells in the DMEM (10% FBS) medium at a concentration of $1 \times 10^5$ cells/mL, 1 mL of each cell diluent was seeded in 6 wells in a 12-well cell culture plate. Furthermore, 1 mL of a DMEM (10% FBS) medium containing 40 ng/mL TNF-$\alpha$ (R&D Systems) was added to each of the 3 wells, and 1 mL of a DMEM (10% FBS) medium was added to each of the remaining 3 wells. The former was cultured as a TNF-$\alpha$-stimulated group and the latter was cultured as a non-stimulated group (control group) for about 24 hours. After the culture, the total amount of culture supernatants was collected, the weight of the collected supernatants was measured with an electronic balance (Shimadzu Corporation), and the supernatants were cryopreserved at -80°C until measurement. After collecting and removing the supernatants, the cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells was measured.

The above-described cryopreserved culture supernatant was thawed, prostaglandin $E_2$ ($PGE_2$) contained in the culture supernatant was labeled with Prostaglandin E2 Express EIA Kit (Cayman Chemical) and then detected with a microplate reader (Molecular Devices, LLC.) The obtained detection value was interpolated into a calibration curve created with $PGE_2$ at a well-known concentration, the concentration of $PGE_2$ contained in the culture supernatant was obtained. The amount of $PGE_2$ secreted was calculated as a secretion amount per cell number $1 \times 10^5$ according to the following equation.

$$[\text{PGE}_2 \text{ secretion amount} = \text{PGE}_2 \text{ concentration measurement value} \times \text{volume of culture supernatant} / \text{number of live cells } (1 \times 10^5 \text{ cells})]$$

The measurement results are shown in Fig. 9. These results show that both the intermediate cells and the cells contained in the dental pulp-derived cell preparation have a TNF$\alpha$-reactive $PGE_2$ secretory ability, but the ability is enhanced in the process of further culturing the intermediate cells to obtain the dental pulp-derived cell preparation. Since $PGE_2$ has a strong anti-inflammatory effect, the dental pulp-derived cell preparation acts on an inflammatory site in the body through $PGE_2$ and can suppress tissue destruction accompanied by inflammation by administering the cells to humans. In addition, since the $PGE_2$ secretory ability is enhanced in the cells contained in the dental pulp-derived cell preparation produced through the intermediate cells, it is thought that the dental pulp-derived cell preparation produced through the production method is effective as a therapeutic agent for diseases for which an anti-inflammatory effect needs to be exerted.

[Example 25: Kynurenine Secretion Test]

(Test Method)

The intermediate cell-frozen product prepared in Example 7 and the dental pulp-derived cell preparation prepared in Example 16 were thawed and then seeded in a cell culture flask so as to have a density of 5,000 to 15,000 cells/cm$^2$, and cultured for 4 days so that the cells became 90% to 100% confluent. The cells were washed with PBS, Trypsin-EDTA (Thermo Fisher Scientific Inc.) was added thereto, and each mixture was allowed to stand for 5 to 10 minutes at 37°C to peel off the cells. A DMEM (10% FBS) medium was added thereto to suspend the cells, and the number of live cells was measured. The cells were seeded in two cell culture flasks so as to have a density of 15,000 to 25,000 cells/cm$^2$. A DMEM (10% FBS) medium containing 100 U/mL IFN-γ (SHIONOGI Co., Ltd.) was added to one of the cell culture flasks, and a DMEM (10% FBS) medium was added to the other cell culture flask. The former was cultured as an IFN-γ-stimulated group and the latter was cultured as a non-stimulated group (control group) for 3 days. After the culture, the total amount of cell culture supernatants was collected. The weight of the collected cell culture supernatants was measured with an electronic balance (Shimadzu Corporation), and the supernatants were cryopreserved at -80°C until measurement. After collecting and removing the supernatants, the cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells of each of the IFN-γ-stimulated group and the non-stimulated group was measured. The above-described cryopreserved culture supernatant was thawed, 30% trichloroacetic acid (NACALAI TESQUE, INC.) was added thereto to perform centrifugation (10,000 g, 10 minutes). Thereafter, kynurenine contained in the culture supernatant was detected with HPLC (Shimadzu Corporation). The obtained detection value was interpolated into a calibration curve created with kynurenine at a well-known concentration, the concentration of kynurenine (molar concentration) contained in the culture supernatant was obtained. The amount of kynurenine secreted was calculated as a secretion amount per cell number according to the following equation.

$$[\text{Kynurenine secretion amount} = \text{kynurenine concentration measurement value} \times 208.21 \times \text{volume of culture supernatant} / \text{number of live cells } (1 \times 10^5 \text{ cells})]$$

(Results)

The results are shown in Table 10. By culturing both the intermediate cells and the dental pulp-derived cell preparation in the presence of IFN-γ, the amount of kynurenine secreted significantly increases. Kynurenine can suppress proliferation of T cells and can differentiate monocytes into anti-inflammatory M2 macrophages. Accordingly, it is thought that the pluripotent stem cell-enriched dental pulp-derived cells can exert an anti-inflammatory effect using kynurenine by being administered to humans.

[Example 26: Properties of Dental Pulp-Derived Cells (Low Immunogenicity Test)]

The intermediate cell-frozen product prepared in Example 7 and the dental pulp-derived cell preparation prepared in Example 16 were each thawed and centrifuged (1,500 rpm, 5 minutes) to precipitate the cells. Subsequently, the cells were suspended in a DMEM (10% FBS) medium, seeded in a cell culture plate so as to have a density of 5,000 to 15,000 cells/cm$^2$, and cultured so as to become 90% to 100% confluent. The cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells was measured. The cells were seeded in two cell culture plates so as to have a density of 15,000 to 25,000 cells/cm$^2$. A DMEM (10% FBS) medium containing 100 U/mL IFN-γ (SHIONOGI Co., Ltd.) was added to one of the cell culture plates, and a DMEM (10% FBS) medium was added to the other cell culture plate. The former was cultured as an IFN-γ-stimulated group and the latter was cultured as a non-stimulated group (control group) for 3 days. After the culture, the cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells of each of the IFN-γ-stimulated group and the non-stimulated group was measured. Subsequently, the cell suspension of each group was centrifuged (1,500 rpm, 5 minutes), and a supernatant was removed. Then, a blocking solution (described in Example 21) was added thereto to adjust the cell concentration to $1 \times 10^7$ cells/mL and allowed to stand on ice for 1 hour.

FITC-labeled anti-human MHC-class I antigen antibody (Ancell Corporation), FITC-labeled anti-human MHC-class II antigen antibody (Ancell Corporation), FITC-labeled anti-human CD40 antibody (BD Biosciences), FITC-labeled anti-human CD80 antibody (BD Biosciences), and FITC-labeled anti-human CD86 antibody (BD Biosciences) were respectively used as antibodies against MHC-class I antigen, MHC-class II antigen, CD40, CD80, and CD86. In addition, FITC-labeled mouse IgG1 isotype control (Beckman Coulter Inc.) and FITC-labeled mouse IgG2a isotype control (BD Biosciences) were used as control antibodies.

Each antibody solution was added to 5-mL reaction tubes (numbers (1) to (16)) as shown in Table 16. Subsequently, 100 μL of the cell suspension of the IFN-γ-stimulated group was added to each of the 5 mL reaction tubes (1) to (8) and 100 μL of the cell suspension of the IFN-γ-stimulated group was added to each of the 5 mL reaction tubes (9) to (16), and the mixture was gently shaken and allowed to stand on ice for 20 minutes to bind antibodies contained in each antibody solution to surface antigen markers expressed on the surfaces of the cells. Subsequently, 3 mL of PBS-B was added to each tube and mixed, the cells were then centrifuged (1,500 rpm, 5 minutes) and precipitated, and a supernatant was removed to remove antibodies which had not been bound to the cells. This operation of removing antibodies was repeated 3 times. Subsequently, 400 μL of PBS-B was added to each tube to suspend the cells.

[Table 18]

Table 18 Type and Amount of Antibody Solution Added to Each Reaction Tube

| Tube number | Antibody solution | Addition amount |
|---|---|---|
| (1), (9) | - | - |
| (2), (10) | IgG1-FITC | 20 |
| (3), (11) | IgG2a-FITC | 20 |
| (4), (12) | Anti-MHC-Class I | 2 |
| (5), (13) | Anti-MHC-Class II | 2 |
| (6), (14) | Anti-CD40-FITC | 20 |
| (7), (15) | Anti-CD80-FITC | 20 |
| (8), (16) | Anti-CD86-FITC | 20 |

Regarding the cells of the tube numbers (1) to (16), the amount of fluorescent dye of FITC was measured with BD FACSVerse (Nippon Becton Dickinson Company, Ltd.) to obtain the amount of fluorescent dye bound to the surfaces of the cells through antibodies specifically bound to the surface antigens by comparison with the negative controls. The tube (2) is a negative control of the tubes (4) and (6) to (8), the tube (3) is a negative control of the tube (5), the tube (10) is a negative control of the tubes (12) and (14) to (16), and the tube (11) is a negative control of the tube (13). The tubes (1) and (9) are unstained cells.

(Results)

The measurement results are shown in Figs. 11A and 11B. As for the measurement results of MHC-class I antigen, approximately all of the cells in the intermediate cells and the dental pulp-derived cell preparation were positive regardless of the presence or absence of IFN-γ stimulation. As for the measurement results of MHC-class II antigen, all of the cells were substantially negative through IFN-γ non-stimulation, but most cells were positive through IFN-γ stimulation in the intermediate cells and the dental pulp-derived cell preparation. As for the measurement results of CD40, CD80, and CD86, approximately all of the cells in the intermediate cells and the dental pulp-derived cell preparation were negative regardless of the presence or absence of IFN-γ stimulation.

MHC-class I antigen and MHC-class II antigen are cell surface antigens having a function of presenting antigens to immune cells. In addition, it is known that MHC-class I antigen is expressed in almost all cells and that expression of MHC-class II antigen is induced in many cells through IFN-γ stimulation. On the other hand, CD40, CD80, and CD86 are surface antigens involved in activation of immune system cells. That is, it is thought that since the intermediate cell-frozen product and the dental pulp-derived cell preparation do not express CD40, CD80, and CD86, these do not actively activate immune cells. That is, these results show that the cells contained in both the intermediate cell-frozen product and the dental pulp-derived cell preparation do not have immunogenicity, and therefore, have properties of not causing immunogenicity even with IFN-γ stimulation.

[Example 27: Measurement of Secretory Ability of Various Factors Containing Cytokine of Intermediate Cells and Cells Contained in Dental Pulp-Derived Cell Preparation]

(Preculture)

The intermediate cell-frozen product prepared in Example 7 and the dental pulp-derived cell preparation prepared in Example 16 were each thawed, and the total amount of each thawed cell suspension was added to a DMEM medium. Centrifugation (300 × g, 5 minutes, room temperature) was performed and a supernatant was removed. Thereafter, a DMEM medium was added thereto to suspend the cells, and the number of live cells and the number of all cells in the cell suspension were measured with Muse Cell Analyzer (Millipore Sigma) to calculate the viability. The cells were seeded in a T225 flask so that the density of live cells became 7,000 cells/cm$^2$ for the cells in the intermediate cell-frozen product and 12,000 cells/cm$^2$ for the cells in the dental pulp-derived cell preparation, and were cultured in a $CO_2$ incubator (37°C, 5% $CO_2$) for 4 days. After the culture, the medium was removed from the T225 flask. Then, the cells were washed with DPBS, 0.25% Trypsin-EDTA was added thereto, and each mixture was allowed to stand in the $CO_2$ incubator for 5 minutes. After confirming the peeling-off of the cells, a DMEM medium was added thereto to suspend the cells, and the total amount of each of the cell suspensions was collected. Centrifugation (300 × g, 5 minutes, room temperature) was performed to precipitate the cells. After removing each supernatant, a DMEM medium was added thereto to suspend the cells, and the number of live cells and the number of all cells in each cell suspension were measured with Muse Cell Analyzer (Millipore Sigma) to calculate the viability.

(Collection of Culture Supernatant)

The cells collected in the preculture were seeded in T25 flasks so that the number of live cells became 28,000 cells per flask and distributed to a non-stimulated group, a TNF-α-stimulated group, and an IFN-γ-stimulated group to start culture. The amount of medium of each group was 10 mL per flask, and the final concentration of TNF-α was adjusted to 10 ng/mL (with a 2 μg/mL stock solution) and the final concentration of IFN-γ was adjusted to 100 U/mL (with 1 × 10$^6$ U/mL). On day 3 after the start of the culture, 0.5 mL of each culture supernatant was collected in a cryotube and cryopreserved. After the collection of the culture supernatants, the cells were washed with DPBS, 0.25% Trypsin-EDTA was added thereto, and each mixture was allowed to stand in the $CO_2$ incubator for 5 minutes. After confirming the peeling-off of the cells, a DMEM medium was added thereto, and the total amount of each of the cell suspensions was collected. Centrifugation (300 × g, 5 minutes, room temperature) was performed and each supernatant was removed. Thereafter, a DMEM medium was added thereto to suspend the cells, and the number of live cells and the number of all cells in each cell suspension were measured with Muse Cell Analyzer (Millipore Sigma) to calculate the viability.

(Measurement of Cytokine)

The concentrations of various factors containing a cytokine contained in the collected culture supernatants were measured with LEGENDplex (BioLegend, Inc.) The measurement principle of LEGENDplex will be outlined below. A primary antibody which is bound to beads and differs for each antigen (type of cytokine) is added to a sample containing a substance to be measured to bind the primary antibody to the substance. Subsequently, a biotinylated secondary antibody was added thereto and bound to the substance bound to the primary antibody, and phycoerythrin (PE)-labeled streptavidin was further bound thereto. Since avidin is specifically bound to biotin, this can be measured through flow cytometry to quantitatively determine phycoerythrin (PE)-labeled streptavidin bound to the beads according to fluorescence intensity thereof. Since this quantitative value is proportional to the amount of substance to be measured contained in the sample, the substance can be quantitatively determined.

(Results: Expression Level of Various Factors in Non-Stimulated groups)

The concentrations of various factors contained in the culture supernatants in the non-stimulated groups are shown in Fig. 12. Both the intermediate cells and the cells contained in the dental pulp-derived cell preparation express MMP-2, IGFBP-4, and cystatin C at a high level (Fig. 12).

In addition, both the intermediate cells and the cells contained in the dental pulp-derived cell preparation express IL-6, IL-11, MCP-1, IL-8, GROα, HGF, VEGF, VCAM-1, TIMP-3, TIMP-2, and TIMP-1 (Fig. 13).

Furthermore, the amount of the intermediate cells and the cells contained in the dental pulp-derived cell preparation is small, but both the the intermediate cells and the cells contained in the dental pulp-derived cell preparation express IL-23, TNF-α, IL-18, IL-33, IL-27, TARC, ENA-78, MIP-3α, MIP-1β, IP-10, SCF, and ICAM-1 (Fig. 14). In addition, both the intermediate cells and the cells contained in the dental pulp-derived cell preparation do not or hardly express IL-21, Exotaxin, MIP-1α, MIG, I-TAC, and GM-CSF. Expression of IFN-α is not detected in the intermediate cells, but a trace amount of IFN-α is expressed in the cells contained in the dental pulp-derived cell preparation. In addition, although not shown in the table, both the intermediate cells and the cells contained in the dental pulp-derived cell preparation do not or hardly express IL-2, IL-4, IL-5, IL-9, and IL-13 which are inflammatory cytokines.

(Results: Difference in Expression Levels of Various Factors in Non-Stimulated Groups between Intermediate Cells and Cells Contained in Dental Pulp-Derived Cell Preparation)

Concentration ratios of factors of which expression levels are relatively different between the intermediate cells and the cells contained in the dental pulp-derived cell preparation among various factors contained in the culture supernatants are shown in Fig. 15. The expression levels of these factors in the cells contained in the dental pulp-derived cell preparation are high compared to the intermediate cells. In particular, the expression levels of IL-6, IL-11, HGF, IGFBP-4, TIMP-3, and TIMP-1 in the cells contained in the dental pulp-derived cell preparation are 1.5 times or more of the expression levels thereof in the intermediate cells.

(Results: Change in Expression Level of IL-6 Due to Stimulation with TNF-α and IFN-γ)

The concentrations of IL-6 contained in the culture supernatants when stimulated with TNF-α and IFN-γ will be shown in Fig. 16. In the intermediate cells, the expression level of IL-6 increases due to stimulation with both TNF-α and IFN-γ compared to the case of no stimulation. The same applies to the cells contained in the dental pulp-derived cell preparation.

(Results: Change in Expression Level of IL-11 Due to Stimulation with TNF-α and IFN-γ)

The concentrations of IL-11 contained in the culture supernatants when stimulated with TNF-α and IFN-γ will be shown in Fig. 17. In the intermediate cells, the expression level of IL-11 increases due to stimulation with both TNF-α and IFN-γ compared to the case of no stimulation. The same applies to the cells contained in the dental pulp-derived cell preparation.

(Results: Change in Expression Level of IP-10 Due to Stimulation with TNF-α and IFN-γ)

The concentrations of IP-10 contained in the culture supernatants when stimulated with TNF-α and IFN-γ will be shown in Fig. 18. In the intermediate cells, the expression level of IP-10 increases due to stimulation with both TNF-α and IFN-γ compared to the case of no stimulation. Although the concentration of IP-10 in the case of no stimulation does not appear in Fig. 18, this is because the concentration of IP-10 is low, and as shown in Fig. 14, the IP-10 is expressed in the intermediate cells even in the case of no stimulation. The same applies to the cells contained in the dental pulp-derived cell preparation.

(Results: Change in Expression Level of MCP-1 Due to Stimulation with TNF-α and IFN-γ)

The concentrations of MCP-1 contained in the culture supernatants when stimulated with TNF-α and IFN-γ will be shown in Fig. 19. In the intermediate cells, the expression level of MCP-1 increases due to stimulation with both TNF-α and IFN-γ compared to the case of no stimulation. The same applies to the cells contained in the dental pulp-derived cell preparation.

(Results: Change in Expression Level of GM-CSF Due to Stimulation with TNF-α and IFN-γ)

The concentrations of GM-CSF contained in the culture supernatants when stimulated with TNF-α and IFN-γ will be shown in Fig. 20. In the case of no stimulation, expression of GM-CSF is hardly recognized in both the intermediate cells and the cells contained in the dental pulp-derived cell preparation. In the intermediate cells, expression of GM-CSF is induced by stimulation with TNF-α, but is not induced by stimulation with IFN-γ. The same applies to the cells contained in the dental pulp-derived cell preparation.

(Results: Change in Expression Level of HGF Due to Stimulation with TNF-α and IFN-γ)

The concentrations of HGF contained in the culture supernatants when stimulated with TNF-α and IFN-γ will be shown in Fig. 21. In the intermediate cells, the expression level of HGF decreases due to stimulation with TNF-α but increases due to stimulation with IFN-γ compared to the case of no stimulation. The same applies to the cells contained in the dental pulp-derived cell preparation.

(Results: Change in Expression Level of IL-8 Due to Stimulation with TNF-α and IFN-γ)

The concentrations of IL-8 contained in the culture supernatants when stimulated with TNF-α and IFN-γ will be shown in Fig. 22. In the intermediate cells, the expression level of IL-8 increases due to stimulation with TNF-$\alpha$ but does not change with stimulation with IFN-$\gamma$ compared to the case of no stimulation. The same applies to the cells contained in the dental pulp-derived cell preparation.

[Example 28: Properties (Cell Division Ability) of Dental Pulp-Derived Cells]

The intermediate cell-frozen product prepared in Example 7 and the dental pulp-derived cell preparation prepared in Example 16 were each thawed and centrifuged (1,500 rpm, 5 minutes) to precipitate the cells. Subsequently, the cells were suspended in a DMEM (10% FBS) medium, seeded in a cell culture plate so as to have a density of 5,000 to 15,000 cells/cm$^2$, and cultured so as to become 90% to 100% confluent. The cells were washed with PBS, trypsin-EDTA was added thereto, and each mixture was allowed to stand for 5 to 10 minutes at 37°C to peel off the cells. A DMEM (10% FBS) medium was added thereto to suspend the cells, and the number of live cells was measured. Subsequently, the cells were seeded in a cell culture plate so as to have a density of 5,000 to 15,000 cells/cm$^2$, and cultured so as to become 90% to 100% confluent. The subculture of the cells was repeated, and the number of live cells was measured every time the subculture was completed to calculate the number of cell divisions. The number of cell divisions was calculated from the number of live cells at the start of each subculture and the number of live cells at the completion of the culture according to the following equation.

$$\text{Number of cell divisions} = \log_2 (\text{number of live cells at completion of culture} / \text{number of live cells at start of culture})$$

(Results)

The cells contained in the intermediate cell-frozen product had an ability of undergoing at least 14 divisions even after thawing, and the doubling time at that time was within 3 days (within 72 hours). In addition, the cells contained in the dental pulp-derived cell preparation had an ability of undergoing at least 4 divisions even after thawing, and the doubling time at that time was within 3 days (within 72 hours).

[Example 29: Usage Example 1 of Dental Pulp-Derived Cell Preparation]

The dental pulp-derived cell preparation is administered to patients with autoimmune diseases such as rheumatoid arthritis and collagen disease by means such as intravenous drip infusion and local injection as medicines for ameliorating various symptoms associated with the diseases.

[Example 30: Usage Example 2 of Dental Pulp-Derived Cell Preparation]

The dental pulp-derived cell preparation is thawed when in use. At the time of use, the dental pulp-derived cell preparation is taken out of a liquid nitrogen storage container and thawed by heating in a water bath at 36.5°C to 37.5°C. The thawed dental pulp-derived cell preparation can be administered to humans as pharmaceutical products through means such as intravenous drip infusion or local injection. In the case of intravenous drip infusion, the dental pulp-derived cell preparation is transferred to a dialysis bag from a cell cryopreservation container and then administered to a patient through intravenous drip infusion. In the case of local injection, the dental pulp-derived cell preparation is transferred to a syringe from a cell cryopreservation container and then injected locally into a patient.

[Example 31: Usage Example 3 of Dental Pulp-Derived Cell Preparation]

Dental pulp-derived cell preparations are thawed and used in medical institutions before use. Accordingly, dental pulp-derived cell preparations which have been cryopreserved in storages of their manufacturers, distributors, or the like are shipped in a frozen state in response to requests from medical institutions and transported to the medical institutions. The cells which have been carried in a frozen state are thawed immediately before administration to patients, and are then administered to patients by means of intravenous drip infusion or the like in the medical institutions.

## Industrial Applicability

The present invention is useful, for example, for providing pharmaceutical products which can be administered to humans and contains dental pulp-derived cells as active components.

## Claims

1. A method for producing dental pulp-derived cells enriched with pluripotent stem cells, the method comprising:

    a step (a) of digesting dental pulp with a protease to prepare a dental pulp suspension;
    a step (b) of culturing the suspension to proliferate pluripotent stem cells contained in the suspension;
    a step (c) of freezing the proliferated pluripotent stem cells in a state in which the pluripotent stem cells are suspended in a first cryopreservation liquid;
    a step (d) of thawing the frozen pluripotent stem cells;
    a step (e) of culturing the thawed pluripotent stem cells in a state in which the pluripotent stem cells are adhered to surfaces of carrier particles to proliferate the pluripotent stem cells on the surfaces of the carrier particles;
    a step (f) of bringing the carrier particles into contact with a protease to separate the pluripotent stem cells adhered to the surfaces of the carrier particles from the carrier particles; and
    a step (g) of preparing a suspension of the separated pluripotent stem cells.

2. The production method according to claim 1,
wherein quality tests of the pluripotent stem cells are performed in the step (c) or (d).

3. The production method according to claim 2,
wherein the quality tests are performed on cells fractionated from cells before suspension in the first cryopreservation liquid, subcultured cells which have been fractionated from the cells before suspension in the first cryopreservation liquid, pre-freezing cells which have been suspended in the first cryopreservation liquid and fractionated, cells immediately after thawing, which have been fractionated and frozen, and/or cells obtained by fractionation, freezing, thawing, and culturing.

4. The production method according to claim 3,
wherein the quality tests are performed for one or more of the following Quality Tests a to j
Quality Test a which verifies that a proportion of the number of cells showing a substantially spindle-shaped form in all cells when observed under an optical microscope is greater than or equal to 99%, greater than or equal to 99.5%, greater than or equal to 99.9%, or greater than or equal to 99.95%,
Quality Test b which verifies that a cell viability is greater than or equal to 50%, greater than or equal to 60%, greater than or equal to 70%, greater than or equal to 80%, greater than or equal to 90%, or greater than or equal to 95%,
Quality Test c which verifies that cells are positive for CD73, CD90, CD105, and CD166 and negative for CD34 and CD45 in expression patterns of the cell surface antigen markers, or verifies that cells are positive for at least one of CD73 and CD90 and negative for CD34 in expression patterns of the cell surface antigen markers,
Quality Test d which verifies that cells are negative for CD40, CD80, CD86, and MHC-class II antigen in expression patterns of the cell surface antigen markers, or verifies that cells are negative for at least one of CD40, CD80, CD86, and MHC-class II antigen in expression patterns of the cell surface antigen markers,
Quality Test e which verifies that cells remain negative for CD40, CD80, and CD86 and become positive for MHC-class II antigen in expression patterns of the cell surface antigen markers when the cells are stimulated with interferon-y, or verifies at least any one of cells being negative for CD40, negative for CD80, negative for CD86, or positive for MHC-class II antigen in expression patterns of the cell surface antigen markers when the cells are stimulated with interferon-y,
Quality Test f which verifies that cells express prostaglandin $E_2$ and/or vascular endothelial growth factor (VEGF) and an expression level of prostaglandin $E_2$ is increased by stimulating the cells with TNF-$\alpha$,
Quality Test g which verifies that an expression level of aggrecan increases when cells are cultured in a medium containing a substance that induces differentiation into chondrocytes,
Quality Test h which verifies that an amount of calcium accumulated in cells increases when the cells are cultured in a medium containing a substance that induces differentiation into osteocytes,
Quality Test i which verifies that cells have an ability of dividing at least 10, 14, or 15 times on a cell culture plate, and
Quality Test j which verifies that an average doubling time of cells on a cell culture plate is within 96 hours, 84 hours, 72 hours, 48 hours, or 36 hours during a period of Quality Test i.

5. The production method according to any one of claims 1 to 4, further comprising:
loading the suspension on a filtration membrane to collect cells which have been passed through in the step (g).

6. The production method according to claim 5,
wherein the filtration membrane has a pore diameter of 20 $\mu$m to 80 $\mu$m.

**7.** The production method according to any one of claims 1 to 6, wherein the dental pulp is obtained from human permanent teeth or deciduous teeth.

**8.** The production method according to any one of claims 1 to 7, wherein the protease used in the step (a) comprises a serine protease, a metalloprotease, or a mixture thereof.

**9.** The production method according to any one of claims 1 to 7, wherein the protease used in the step (a) comprises a metalloprotease.

**10.** The production method according to any one of claims 1 to 7, wherein the protease used in the step (a) comprises a matrix metalloprotease, a neutral metalloprotease, or a mixture thereof.

**11.** The production method according to any one of claims 1 to 7, wherein the protease used in the step (a) comprises a collagenase and a neutral metalloprotease.

**12.** The production method according to claim 11, wherein the collagenase comprises collagenase I, collagenase II, or a mixture thereof.

**13.** The production method according to claim 10 or 11, wherein the neutral metalloprotease comprises thermolysin, Dispase, or a mixture thereof.

**14.** The production method according to any one of claims 1 to 13, wherein the first cryopreservation liquid used in the step (c) includes a bicarbonate Ringer's solution, human serum albumin, and DMSO.

**15.** The production method according to any one of claims 1 to 14, wherein the carrier particles used in the step (e) have a substantially spherical shape having a diameter of 80 to 300 $\mu$m in a swollen state.

**16.** The production method according to claim 15, wherein the carrier particles are porous carrier particles having pores of 3 to 40 $\mu$m in diameter which open to the surfaces of the carrier particles in the swollen state.

**17.** The production method according to any one of claims 1 to 16, wherein the carrier particles contain gelatin.

**18.** The production method according to any one of claims 1 to 17, wherein the protease used in the step (f) comprises a serine protease, a metalloprotease, or a mixture thereof.

**19.** The production method according to any one of claims 1 to 17, wherein the protease used in the step (f) comprises trypsin.

**20.** The production method according to any one of claims 1 to 19, further comprising: a step (h) of freezing the suspension obtained in the step (g) in a state in which the suspension is suspended in a second cryopreservation liquid.

**21.** The production method according to claim 20, wherein the second cryopreservation liquid used in the step (h) includes a bicarbonate Ringer's solution, human serum albumin, and DMSO.

**22.** The production method according to any one of claims 1 to 19, wherein quality tests of the pluripotent stem cells are performed in the step (g).

**23.** The production method according to claim 20 or 21, wherein quality tests of the pluripotent stem cells are performed in the step (g) or (h).

**24.** The production method according to claim 22 or 23, wherein the quality tests are performed on cells fractionated from cells before suspension in the second cryopreservation liquid, subcultured cells which have been fractionated from the cells before suspension in the second cryopreservation liquid, pre-freezing cells which have been suspended in the second cryopreservation liquid and fractionated, cells immediately after thawing, which have been fractionated and frozen, and/or cells obtained by fractionation, freezing, thawing, and culturing.

**25.** The production method according to claim 22 or 24,
wherein the quality tests are performed for one or more of the following Quality Tests a' to j'
Quality Test a' which verifies that a proportion of the number of cells showing a substantially spindle-shaped form in all cells when observed under an optical microscope is greater than or equal to 99%, greater than or equal to 99.5%, greater than or equal to 99.9%, or greater than or equal to 99.95%,
Quality Test b' which verifies that a cell viability is greater than or equal to 50%, greater than or equal to 60%, greater than or equal to 70%, greater than or equal to 80%, greater than or equal to 90%, or greater than or equal to 95%,
Quality Test c' which verifies that cells are positive for CD73, CD90, CD105, and CD166 and negative for CD34 and CD45 in expression patterns of the cell surface antigen markers, or verifies that cells are positive for at least one of CD73 and CD90 and negative for CD34 in expression patterns of the cell surface antigen markers,
Quality Test d' which verifies that cells are negative for CD40, CD80, CD86, and MHC-class II antigen in expression patterns of the cell surface antigen markers, or verifies that cells are negative for at least one of CD40, CD80, CD86, and MHC-class II antigen in expression patterns of the cell surface antigen markers,
Quality Test e' which verifies that cells remain negative for CD40, CD80, and CD86 and become positive for MHC-class II antigen in expression patterns of the cell surface antigen markers when the cells are stimulated with interferon-y, or verifies at least any one of cells being negative for CD40, negative for CD80, negative for CD86, or positive for MHC-class II antigen in expression patterns of the cell surface antigen markers when the cells are stimulated with interferon-y,
Quality Test f' which verifies that cells express prostaglandin $E_2$ and/or vascular endothelial growth factor (VEGF) and an expression level of prostaglandin $E_2$ is increased by stimulating the cells with TNF-$\alpha$,
Quality Test g' which verifies that an expression level of aggrecan increases when cells are cultured in a medium containing a substance that induces differentiation into chondrocytes,
Quality Test h' which verifies that an amount of calcium accumulated in cells increases when the cells are cultured in a medium containing a substance that induces differentiation into osteocytes,
Quality Test i' which verifies that cells have an ability of dividing at least 3, 4, or 5 times on a cell culture plate, and
Quality Test j' which verifies that an average doubling time of cells on a cell culture plate is within 96 hours, 84 hours, 72 hours, 48 hours, or 36 hours during a period of Quality Test i'.

**26.** The production method according to any one of claims 22 to 25,
wherein a positive rate of CD107b in the pluripotent stem cells used in the quality tests in the step (g) or (h) is higher than the corresponding positive rate of the pluripotent stem cells used in the quality tests in the step (c) or (d).

**27.** The production method according to any one of claims 22 to 26,
wherein a positive rate of at least one of CD39, CD49a, CD61, CD107a, CD107b, and CD143 in the pluripotent stem cells used in the quality tests in the step (g) or (h) is higher than the corresponding positive rate of the pluripotent stem cells used in the quality tests in the step (c) or (d), and
wherein a positive rate of CD146 in the pluripotent stem cells used in the quality tests in the step (g) or (h) is lower than the corresponding positive rate of the pluripotent stem cells used in the quality tests in the step (c) or (d).

**28.** The production method according to any one of claims 22 to 27,
wherein an expression level of at least one of IL-6, HGF, IGFBP-4, IL-11, TIMP-3, and TIMP-2 in the pluripotent stem cells used in the quality tests in the step (g) or (h) is higher than the corresponding expression level in the pluripotent stem cells used in the quality tests in the step (c) or (d).

**29.** Cells obtained through the production method according to any one of claims 1 to 28.

**30.** The cells according to claim 29, which are positive for CD73, CD90, CD105, and CD166 and negative for CD34 and CD45.

**31.** The cells according to claim 30, which are negative for CD40, CD80, CD86, and MHC-class II antigen.

**32.** The cells according to claim 31, which become positive for the MHC-class II antigen when the cells are stimulated with interferon-y.

**33.** Dental pulp-derived pluripotent stem cells having at least one characteristic shown in (1) to (4) below

(1) the cells are positive for CD73, CD90, CD105, and CD166 and negative for CD34, CD40, CD45, CD80, CD86, and MHC-class II antigen, become positive for the MHC-class II antigen when the cells are stimulated with interferon-y, and express prostaglandin $E_2$ and/or vascular endothelial growth factor, and an expression level of prostaglandin $E_2$ increases when the cells are stimulated with TNF-$\alpha$,
(2) the cells are positive for at least one of CD73, CD90, CD105, and CD166 and negative for CD34 and CD45,
(3) the cells are positive for at least one of CD47, CD81, and CD147, and negative for at least one of CD19, CD34, and CD206, and
(4) the celsl are positive for at least one of CD47, CD81, and CD147 and negative for at least one of CD19, CD31, CD33, CD34, CD38, CD45, CD206, CD235a, and SSEA-1.

**34.** The cells according to claim 33, which have an ability to differentiate into osteocyte and chondrocyte.

**35.** The cells according to claim 33 or 34,
wherein an average diameter of the cells in a state in which the cells are made to float in a medium is 16 to 20 $\mu$m.

**36.** The cells according to any one of claims 33 to 35, which have an ability of dividing at least 3 times in an in vitro environment,
wherein an average doubling time is within 96 hours.

**37.** The dental pulp-derived pluripotent stem cells according to any one of claims 33 to 35, which have the following characteristics that
the cells have divided at least 10, 15, 16, or 17 times in an in vitro environment, and an average time of the cell divisions is within 48 hours, and
the cells have an ability of dividing at least 10, 14, or 15 times in an in vitro environment, and an average time of the cell divisions is within 96 hours, 84 hours, 72 hours, 48 hours, or 36 hours.

**38.** The dental pulp-derived pluripotent stem cells according to any one of claims 33 to 35, which have the following characteristics that
the cells have divided at least 16, 21, 22, or 23 times in an in vitro environment, and
the cells have an ability of dividing at least 3, 4, or 5 times in an in vitro environment, and an average time of the cell divisions is within 96 hours, 84 hours, 72 hours, 48 hours, or 36 hours.

**39.** The dental pulp-derived pluripotent stem cells according to any one of claims 33 to 38, which are negative for at least one of CD19, CD26, CD106, CD117, and CD271.

**40.** The dental pulp-derived pluripotent stem cells according to any one of claims 33 to 39, which are positive for at least one of CD140b and HLA-A, -B, and -C.

**41.** The dental pulp-derived pluripotent stem cells according to any one of claims 33 to 40, which are negative for at least one of CD56 and CD146.

**42.** The dental pulp-derived pluripotent stem cells according to any one of claims 33 to 41, which are positive for at least one of CD49e and CD95.

**43.** The dental pulp-derived pluripotent stem cells according to any one of claims 33 to 42, which are positive for at least one of CD10, CD46, CD47, CD55, CD58, and CD59.

**44.** The dental pulp-derived pluripotent stem cells according to any one of claims 33 to 43, which express at least one of MMP-2, IGFBP-4, cystatin C, IL-6, IL-11, MCP-1, IL-8, HGF, VEGF, TIMP-1, TIMP-2, and TIMP-3.

**45.** The dental pulp-derived pluripotent stem cells according to any one of claims 33 to 44, which express at least one of GRO$\alpha$, VCAM-I, and IP-10.

**46.** The dental pulp-derived pluripotent stem cells according to any one of claims 33 to 45, which express IL-6 and in which an expression level thereof is increased by TNF-$\alpha$ stimulation and interferon-y stimulation.

**47.** The dental pulp-derived pluripotent stem cells according to any one of claims 33 to 46, which express IL-11 and in which an expression level thereof is increased by TNF-α stimulation and interferon-y stimulation.

**48.** The dental pulp-derived pluripotent stem cells according to any one of claims 33 to 47, which express IP-10 and in which an expression level thereof is increased by TNF-α stimulation and interferon-y stimulation.

**49.** The dental pulp-derived pluripotent stem cells according to any one of claims 33 to 48, which express MCP-1 and in which an expression level thereof is increased by TNF-α stimulation and interferon-y stimulation.

**50.** The dental pulp-derived pluripotent stem cells according to any one of claims 33 to 49,
wherein expression of GM-CSF is induced by TNF-α stimulation.

**51.** The dental pulp-derived pluripotent stem cells according to any one of claims 33 to 50, which express HGF and in which an expression level thereof is decreased by TNF-α stimulation and increases by interferon-y stimulation.

**52.** The dental pulp-derived pluripotent stem cells according to any one of claims 33 to 51, which express IL-8 and in which an expression level thereof is increased by TNF-α stimulation.

**53.** The dental pulp-derived pluripotent stem cells according to any one of claims 33 to 52,
wherein the dental pulp is obtained from human permanent teeth or deciduous teeth.

**54.** A composition comprising:
the dental pulp-derived pluripotent stem cells according to any one of claims 33 to 53 suspended in a bicarbonate Ringer's solution containing human serum albumin and dimethyl sulfoxide.

**55.** A composition comprising:
the dental pulp-derived pluripotent stem cells according to any one of claims 33 to 53 suspended in a solution containing sodium ions, potassium ions, calcium ions, magnesium ions, hydrogen carbonate ions, citrate ions, human serum albumin, and dimethyl sulfoxide.

**56.** The composition according to claim 55,
wherein sodium ions, potassium ions, calcium ions, magnesium ions, hydrogen carbonate ions, citrate ions, human serum albumin, and dimethyl sulfoxide are respectively contained at concentrations of 91 to 113 mM, 2.52 to 3.08 mM, 0.95 to 1.16 mM, 0.315 to 0.385 mM, 15.6 to 19.2 mM, 1.04 to 1.28 mM, 46 to 56 g/L, and 9% to 11% (v/v).

**57.** The composition according to any one of claims 54 to 56, further comprising:

acetyltryptophan or a salt thereof; and
caprylic acid or a salt thereof.

**58.** The composition according to any one of claims 54 to 57,
wherein the dental pulp-derived pluripotent stem cells are contained at a density of 5 × 10° to 8 × $10^7$ cells/mL.

**59.** The composition according to any one of claims 54 to 58, which are encapsulated in a container in an amount of 1 to 20 mL.

**60.** The composition according to claim 59,
wherein the container is made of glass or plastic.

**61.** The composition according to any one of claims 54 to 60, which is in a frozen state.

**62.** A pharmaceutical composition comprising:
the composition according to any one of claims 54 to 61.

**63.** The pharmaceutical composition according to claim 62, which is a therapeutic agent for a disease selected from the group consisting of an autoimmune disease, an inflammatory disease, rheumatoid arthritis, Crohn's disease, chronic inflammatory bowel disease, myocardial infarction, cerebral infarction (including chronic cerebral infarction and acute cerebral infarction), chronic inflammatory demyelinating polyneuritis, multiple sclerosis, systemic lupus erythematosus, liver cirrhosis (including decompensated liver cirrhosis), sepsis, osteoarthritis, psoriasis, and organ graft rejection.

**64.** The pharmaceutical composition according to claim 62, for use in treating a disease selected from the group consisting of an autoimmune disease, an inflammatory disease, rheumatoid arthritis, Crohn's disease, chronic inflammatory bowel disease, myocardial infarction, cerebral infarction (including chronic cerebral infarction and acute cerebral infarction), chronic inflammatory demyelinating polyneuritis, multiple sclerosis, systemic lupus erythematosus, liver cirrhosis (including decompensated liver cirrhosis), sepsis, osteoarthritis, psoriasis, and organ graft rejection.

Fig.1

AVERAGE PARTICLE DIAMETER (μm)
20.00
16.00
12.00
8.00
4.00
0.00
INTERMEDIATE CELLS
CELLS CONTAINED IN DENTAL PULP-DERIVED CELL PREPARATION

Fig.2

CALCIUM CONCENTRATION (μg/mL)
25.0
20.0
15.0
10.0
5.0
0.0
OSTEOCYTE DIFFERENTIATION-INDUCING GROUP
CONTROL GROUP
INTERMEDIATE CELLS
CELLS CONTAINED IN DENTAL PULP-DERIVED CELL PREPARATION

# Fig.3

25.0
27.0
29.0
31.0
33.0
35.0
37.0
39.0
Ct VALUE (ACAN)
CHONDROCYTE DIFFERENTIATION-INDUCING GROUP
CONTROL GROUP
INTERMEDIATE CELLS
CELLS CONTAINED IN DENTAL PULP-DERIVED CELL PREPARATION

*Fig.4*

(%)
100
80
60
40
20
0
PROPORTION OF POSITIVE CELLS
CD34
CD45
CD73
CD90
CD105
CD166
INTERMEDIATE CELLS
CELLS CONTAINED IN DENTAL PULP-DERIVED CELL PREPARATION

Fig.5

PROPORTION OF POSITIVE CELLS (%)

0 20 40 60 80 100

CD9, CD10, CD13, CD29, CD44, CD46, CD47, CD49b, CD49c, CD49f, CD55, CD58, CD59, CD63, CD73, CD81, CD90, CD98, CD140b, CD147, CD151, CD164, CD166, EGF-R, HLA-A,B,C

HLA-A,B,C
EGF-R
CD166
CD164
CD151
CD147
CD140b
CD98
CD95
CD90
CD81
CD73
CD63
CD59
CD58
CD55
CD49f
CD49e
CD49c
CD49b
CD47
CD46
CD44
CD29
CD13
CD10
CD9
0
20
40
60
80
100
PROPORTION OF POSITIVE CELLS (%)

Fig.6

# Fig.7

80
60
40
20
0
(%)
-20
-40
-60
-80
CD39
CD49a
CD61
CD107a
CD107b
CD143
CD146

*Fig.8*

AMOUNT OF VEGF SECRETED
(pg/10^5 CELLS)
1200
1000
800
600
400
200
0
INTERMEDIATE CELLS
CELLS CONTAINED IN DENTAL PULP-DERIVED CELL PREPARATION

# Fig.9

TNF-α-STIMULATED GROUP
TNF-α-NON-STIMULATED GROUP
25000
20000
15000
10000
5000
0
AMOUNT OF PGE2 SECRETED (pg/10^5 CELLS)
INTERMEDIATE CELLS
CELLS CONTAINED IN DENTAL PULP-DERIVED CELL PREPARATION

*Fig.10*

AMOUNT OF KYNURENINE SECRETED
(ng/10^5 CELLS)
7000
6000
5000
4000
3000
2000
1000
0
■ IFN-γ(+)
□ IFN-γ(-)
INTERMEDIATE CELLS
CELLS CONTAINED IN DENTAL PULP-DERIVED CELL PREPARATION

# Fig.11A

PROPORTION OF POSITIVE CELLS (%)
100
80
60
40
20
0
IFN-γ- NON-STIMULATED GROUP
IFN-γ- STIMULATED GROUP
MHC class I
MHC class II
CD40
CD80
CD86

# Fig.11B

PROPORTION OF POSITIVE CELLS (%)
100
80
60
40
20
0
IFN-γ- NON-STIMULATED GROUP
IFN-γ- STIMULATED GROUP
MHC class I
MHC class II
CD40
CD80
CD86

# Fig.12

CONCENTRATIONS OF VARIOUS FACTORS
(pg/10^5 CELLS)
20,000
18,000
16,000
14,000
12,000
10,000
8,000
6,000
4,000
2,000
0
MMP-2
IGFBP-4
CystatinC

Fig.13

CONCENTRATIONS OF VARIOUS FACTORS (pg/10⁵ CELLS)

0
200
400
600
800
1,000
1,200
1,400
1,600

IL-6
IL-11
MCP-1
IL-8
GROα
HGF
VEGF
VCAM-1
TIMP-3
TIMP-2
TIMP-1

CONCENTRATIONS OF VARIOUS FACTORS
(pg/10^5 CELLS)
0
1
2
3
4
5
6
7
8
IL-23
IL-21
IFN-α
TNF-α
IL-18
IL-33
IL-27
TARC
ENA-78
MIP-3α
MIP-1β
Eotaxin
IP-10
MIP-1α
MIG
I-TAC
SCF
GM-CSF
ICAM-1


Fig.14

# *Fig.15*

CONCENTRATION RATIOS OF VARIOUS FACTORS
2.5
2.0
1.5
1.0
0.5
0.0
IL-6
IL-23
IL-11
MCP-1
ENA-78
HGF
VEGF
MMP-2
IGFBP-4
CystatinC
TIMP-3
TIMP-2
TIMP-1

# Fig.16

8,000
6,000
4,000
2,000
0
IL-6 (pg/10^5 CELLS)
NO STIMULATION
STIMULATION WITH TNF-α
STIMULATION WITH IFN-γ

# Fig.17

2,000
1,600
1,200
800
400
0
IL-11 (pg/10⁵ CELLS)
NO STIMULATION
STIMULATION WITH TNF-α
STIMULATION WITH IFN-γ

# Fig.18

3,000
2,000
1,000
0
IP-10 (pg/10^5 CELLS)
NO STIMULATION
STIMULATION WITH TNF-α
STIMULATION WITH IFN-γ

# Fig.19

6,000
4,000
2,000
0
MCP-1 (pg/10^5 CELLS)
NO STIMULATION
STIMULATION WITH TNF-α
STIMULATION WITH IFN-γ

# Fig.20

30
20
10
0
GM-CSF (pg/10^5 CELLS)
NO STIMULATION
STIMULATION WITH TNF-α
STIMULATION WITH IFN-γ

## Fig.21

600
400
200
0
HGF (pg/10^5 CELLS)
NO STIMULATION
STIMULATION WITH TNF-α
STIMULATION WITH IFN-γ

# Fig.22

2,000
1,600
1,200
800
400
0
IL-8 (pg/10⁵ CELLS)
NO
STIMULATION
STIMULATION
WITH TNF-α
STIMULATION
WITH IFN-γ

**INTERNATIONAL SEARCH REPORT**

International application No.
PCT/JP2019/029913

A. CLASSIFICATION OF SUBJECT MATTER
Int.Cl. C12N5/0775(2010.01)i, A61K35/32(2015.01)i, A61K35/545(2015.01)i, A61P19/02(2006.01)i, A61P19/04(2006.01)i, A61P29/00(2006.01)i, A61P37/06(2006.01)i

According to International Patent Classification (IPC) or to both national classification and IPC

B. FIELDS SEARCHED

Minimum documentation searched (classification system followed by classification symbols)
Int.Cl. C12N5/0775, A61K35/32, A61K35/545, A61P19/02, A61Pl9/04, A61P29/00, A61P37/06

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), PubMed, CiNii, CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | HADAEGH, Y. et al., "Characterization of stem cells from the pulp of unerupted third molar tooth.", Indian Journal of Dental Research, 2014, 25(1), pp. 14-21, abstract, page 15, left column, paragraph [0003] to page 16, left column, the bottom line, page 17, left column, paragraph [0004] to right column, the bottom line, page 20, left column, paragraph [0003], fig. 3-5 | 29-64<br>1-64 |
| X<br>Y | KERKIS, I. et al., "Stem Cells in Dental Pulp of Deciduous Teeth.", TISSUE ENGINEERING: Part B, 2012, vol. 18 no. 2, pp. 129-138, abstract, fig. 1, 2, table 1, pp. 133-134, section "Osteogenic Potential of DTSCs" | 29-64<br>1-64 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 October 2019 (07.10.2019) | 15 October 2019 (15.10.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2019/029913

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | DASTGUROI, M. E. et al., "Comparison of two digestion strategies on characteristics and differentiation potential of human dental pulp stem cells.", Archives of Oral Biology, 19 May 2018, 93, pp. 74-79, abstract, page 75, section "2.1. Primary cell culture", fig. 1, 2 | 29-64<br>1-64 |
| X<br>Y | VASILI, A. et al., "Endothelian transdifferentiation of human dental tissue-derived mesenchymal stem cells under the influence of erythropoetin (EPO), proangiogenic factor VEGF and serum-free conditions.", Regenerative Medicine, 2013, vol. 8, no. 6, Supply 1, page 130 OP-117, entire text | 29, 31-64<br>1-64 |
| X<br>Y | LIU, H. et al., "Multilineage potential of pulp stem cells from human young permanent teeth in vitro.", JOURNAL OF PEKING UNIVERSITY (HEALTH SCIENCES), 2007, 39 (1), pp. 41-45, abstract | 29-33, 35-64<br>1-64 |
| Y | CN 103849595 A (SHANGHAI KUNAI BIO TECHNOLOGY CO., LTD.) 11 June 2014, abstract, paragraphs [0015], [0021]-[0024], [0064], [0065], examples 1-7 (Family: none) | 1-64 |
| Y | WO 2014/185470 A1 (ADVANCED CENTER FOR TISSUE ENGINEERING LTD.) 20 November 2014, paragraph [0027] & US 2016/0296669 A1 paragraph [0080] & EP 2998390 A1 | 1-64 |

Form PCT/ISA/210 (continuation of second sheet) (January 2015)

**INTERNATIONAL SEARCH REPORT**

International application No.
PCT/JP2019/029913

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No. PCT/JP2019/029913

<Continuation of Box No. III>

Claims are classified into the following five inventions.

(Invention 1) Claims 1-32

Claims 1-32 have the special technical feature of the invention regarding a method which is for producing cells derived from dental pulp in which pluripotent stem cells are enriched and which includes steps of (a)-(g) described in claim 1, and thus are classified as invention 1.

(Invention 2)-(invention 5) (1)-(4) of claim 33 and claims 34-64 referring to (1)-(4) of claim 33, respectively

The invention in (1)-(4) of claim 33 and claim 1 classified as invention 1 share the common technical feature of pluripotent stem cells derived from dental pulp, respectively. However, since pluripotent stem cells derived from dental pulp are well known to the art, and said technical feature does not make a contribution over the prior art, this cannot be considered a special technical feature. Furthermore, there are no other identical or corresponding technical features between the invention in (1)-(4) of claim 33 and the invention in claim 1.

Also, the invention in (1)-(4) of claims 33 is not dependent on claim 1. Further, the invention in (1)-(4) of claims 33 is not substantially identical or equivalent to the claims classified as invention 1.

Therefore, the invention in (1)-(4) of claims 33 cannot be classified as invention 1, and thus are classified as inventions 2-5, respectively. This also applies to claims 34-64 referring to (1)-(4) of claim 33.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5486359 A
- US 5827740 A
- US 6835377 B
- US 6387369 B
- JP 2004129549 A
- JP 2004210713 A
- JP 2010252778 A
- WO 2002007679 A
- JP 2008507962 A
- WO 2009072527 A
- JP 2004201612 A
- JP 2009527223 A
- JP 2005516616 A
- JP 2006238875 A
- JP 2008295420 A
- JP 2010268715 A
- JP 2011219432 A
- WO 2017099105 A


### Non-patent literature cited in the description


- **KATRITSIS DG.** *Catheter Cardiovasc Interv.,* 2005, vol. 65 (3), 321-9
- **GRONTHOS S.** *J Dent Res.,* 2002, vol. 81 (8), 531-5
- **TIRINO V.** *Stem Cell Rev.,* 2011, vol. 7 (3), 608-15
- **VISHWANATH VR.** *J Conserv Dent.,* 2013, vol. 16 (5), 423-8
- **PITTENGER MF. et al.** *Science,* 1999, vol. 284, 143-7
- **COLTER DC. et al.** *Proc Natl Acad Sci USA.,* 2001, vol. 98, 7841-5
- **KIANI C. et al.** *Cell Res.,* 2002, vol. 12, 19-32
- **AUNG A. et al.** *Arthritis Rheum.,* 2011, vol. 63, 148-58